(19) Europäisches Patentamt / European Patent Office / Office européen des brevets

(11) **EP 4 292 584 A1**

(12) **EUROPEAN PATENT APPLICATION**

(43) Date of publication:
**20.12.2023 Bulletin 2023/51**

(21) Application number: **23179038.7**

(22) Date of filing: **13.06.2023**

(51) International Patent Classification (IPC):
**A61K 9/127** (2006.01)   **C12N 15/88** (2006.01)
**A61K 31/7105** (2006.01)   **A61K 9/51** (2006.01)
**C12N 15/11** (2006.01)   **A61K 9/00** (2006.01)

(52) Cooperative Patent Classification (CPC):
**A61K 9/0021; A61K 9/1272; A61K 9/5123;**
**A61K 31/7105; C12N 15/88;** A61K 39/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.06.2022   CN 202210661776**
**28.07.2022   CN 202210897045**
**18.08.2022   CN 202210994917**
**24.05.2023   CN 202310592050**

(71) Applicant: Zcapsule Pharmaceuticals Co., Ltd.
**Shanghai 201203 (CN)**

(72) Inventors:
• **LIU, Weiwei**
**Shanghai, 201203 (CN)**
• **WANG, Lufan**
**Shanghai, 201203 (CN)**
• **GUO, Zhifeng**
**Shanghai, 201203 (CN)**
• **MAO, Junsong**
**Shanghai, 201203 (CN)**
• **GAO, Chenyang**
**Shanghai, 201203 (CN)**
• **TIAN, Yuanrui**
**Shanghai, 201203 (CN)**
• **GU, Zhen**
**Shanghai, 201203 (CN)**
• **LIU, Xiaoxi**
**Shanghai, 201203 (CN)**

(74) Representative: **Longland, Emma Louise et al**
**Venner Shipley LLP**
**Byron House**
**Cambridge Business Park**
**Cowley Road**
**Cambridge CB4 0WZ (GB)**

Remarks:
The complete document including Reference Table(s) and the Sequence Listing(s) can be downloaded from the EPO website

(54) **COMPOSITION FOR MICRONEEDLE ADMINISTRATION AND ITS APPLICATIONS**

(57)   The present invention provides a mRNA composition for microneedle administration and its application. The composition includes an aqueous phase solution and a lipid solution, wherein the lipid solution encapsulates the substance in the aqueous phase solution to form lipid nanoparticles (LNPs), and the aqueous phase solution includes mRNA encoding the corresponding protein and a buffer. It was found that an improved buffer pH could substantially increase the drug loading capacity of LNPs, and reduce the use level of composition and excipients and the dose for microneedle administration with less toxic side effects and better therapeutic effects, and the microneedle administration technology could achieve increased effective expression, decreased expression in the liver, and prolonged *in vivo* expression of mRNA composition, and increased antibody titer in response to low-dose mRNA *in vivo*. The present invention really realizes microneedle intradermal administration of mRNA composition. The composition for microneedle intradermal administration enables stable long-term storage, and has a potential application prospect of reducing toxicity and increasing efficacy.

EP 4 292 584 A1

## Description

### Cross-Reference to Related Applications

The present application claims the priority of the Chinese patent application with an application No. 2022106617762, filed on 2022-06-13; 2022109949172, filed on 2022-08-18; 2022108970458, filed on 2022-07-28; 2023105920502, filed on 2023-05-24. The abstract, description, claims, and drawings of the description of the present application are used in its entirety by the present application.

### Technical Field

The present invention relates to the field of biotechnology. Specifically, it relates to a composition for microneedle intradermal administration, and particularly to a high mRNA-loaded composition for microneedle administration and its application.

### Background

Naked mRNA is degraded when it enters the body directly. Efficient and safe mRNA delivery into cells for the translation and manufacturing of protein can effectively guarantee the efficacy of mRNA drugs. Lipid nanoparticles (LNPs) are a type of micro-sized vesicles encapsulating drugs therein, which can protect RNA from degradation by ribonucleases, so that it can be specifically absorbed by cells and released from endosomes in time after entering cytoplasm, and thus the delivered mRNA can be effectively translated into target proteins. LNPs are commonly used as a drug delivery carrier, and have developed into a mature novel formulation. Compared with traditional monolayer liposomes or lipid vesicles, LNPs have more complex microstructures. LNPs mainly include cholesterol, phospholipids, polyethylene glycol-modified lipids, lipids, etc., which are considered to be pharmacologically inactive and less toxic and act as a self-adjuvant to some extent. The LNP delivery system has comprehensive advantages, but existing LNP drug delivery systems also have obvious limitations, such as lack of target selectivity, short blood circulation time, and *in vivo* instability. According to some studies, LNPs are not completely immunologically inert in some cases, and LNP components are non-natural compounds (e.g., polyethylene glycol-modified lipids), which have an irritation or toxicity potential in humans under certain circumstances, such as causing serious adverse reactions and other problems and hidden dangers in individual cases.

Existing LNPs injections are usually injected intravenously, intramuscularly or subcutaneously. Intravenous injection faces some insurmountable problems, e.g., a certain injection volume required to produce effects, potential vascular damage, endangeitis or inflammation of other parts and tissues caused by the injected drug, injection pain, medical staff assistance needed for injection, inconvenience of use, and low expression of nucleic acids in the body. Moreover, existing LNPs are generally injected intramuscularly and subcutaneously, with low drug utilization, slow onset of action, and other limitations.

Microneedle administration is a novel local intradermal drug delivery technology, combining the convenience of patch and the effectiveness of intradermal injection, circumventing the shortcomings of other drug delivery methods, with various advantages such as no nerve touching, safety, painlessness, and efficient penetration. Microneedle devices usually include multiple microneedles typically measuring ≤1 mm in length, and said microneedle can create microchannels in the stratum corneum of the skin, break through the stratum corneum barrier and promote drug penetration, thus reducing the accumulation of drugs in the stratum corneum and increasing the dose of drugs reaching epidermis, dermis and subcutaneous tissues. Therefore, microneedle devices are widely applied to promote the transdermal absorption of micromolecule and macromolecule drugs, with a wide application prospect. In addition, microneedle devices are extremely convenient to use, allowing manipulation by the patients themselves with no need of professional training, and are easy to handle after use, with low risk of accidental needlestick injury and small possibility of accidental injury.

Existing mRNA compositions are generally injected intramuscularly or intravenously, with unsatisfactory immune effect, increased burden on the liver and toxicity.

Meanwhile, existing mRNA compositions have a low drug loading capacity and are usually injected intravenously at a high dose, causing potential harm of increased toxic side effects to patients. A large injection volume (> 50 μL per injection) is required to achieve therapeutic effect *in vivo* for large-volume mRNA compositions prepared by existing methods, making microneedle administration infeasible (the dose and volume for microneedle administration should not exceed 1 mg and 50 μL, respectively).

Therefore, in order to achieve microneedle administration of mRNA compositions, there is an urgent need to develop mRNA compositions for microneedle administration with higher drug loading capacity, lower dose and toxic side effects, and better therapeutic effects to meet the clinical needs.

**Summary of the Invention**

To solve the problems of traditional injections, the present invention provides a mRNA composition for microneedle administration and its application. The composition comprises an aqueous phase solution and a lipid solution, wherein the lipid solution encapsulates the substance in the aqueous phase solution to form LNPs, and the aqueous phase solution comprises a mRNA for encoding a substance for treating or preventing a disease and a buffer. It was found that an improved buffer pH could substantially increase the drug loading capacity of LNPs, and reduce the use level of composition and excipients and the dose for microneedle administration with less toxic side effects and better therapeutic effects, and the microneedle administration technology could achieve increased expression in muscles and decreased expression in the liver, and prolonged *in vivo* expression of mRNA composition, and increased antibody titer in response to low-dose mRNA *in vivo.* The present invention really realizes the microneedle intradermal administration of mRNA compositions. The composition for microneedle intradermal administration enables stable long-term storage, show very good immune effect as detected at the cellular level and significant tumor inhibitory effect via microneedle administration in immunodeficient tumor-bearing mouse model with a reconstituted immune system, and have a potential application prospect of reducing toxicity and increasing efficiency.

In one aspect, the present invention provides a high mRNA-loaded mRNA composition for microneedle administration, and said composition comprises an aqueous phase solution and a lipid solution, wherein the lipid solution encapsulates the substance in the aqueous phase solution to form LNPs, and the aqueous phase solution comprises a mRNA for encoding a substance for treating or preventing a disease and has a buffer with a pH of 4.5-6.8. Alternatively, the present invention provides a composition comprising LNPs encapsulating an aqueous phase solution therein or comprising an aqueous phase solution therein, wherein the aqueous phase solution comprises a mRNA for encoding a substance for treating or preventing a disease and has a pH of 4.5-6.8.

The "microneedle" described in the present invention differs from an ordinary injection device in that the needle tip is very short, or the therapeutic composition is administered at a low dose, for example, through microfluidic channels (e.g., 1-999 $\mu$ m in internal diameter), at a dose in $\mu$L or $\mu$g (e.g., 1-10 $\mu$g or 0.1-200 $\mu$L), or performing microneedle administration as follows: gradually administer the drug through microfluidic channels at a dose in $\mu$L into superficial tissues of human body (e.g., subcutaneous, intradermal or intramuscular tissues).

Therefore, the present invention provides a method, which employs microneedles for subcutaneous administration, particularly for administration of lipid-encapsulated mRNA capable of encoding a substance for treating a disease, such as a mRNA for encoding a substance for treating or preventing a disease. This method of administration enables significantly reduced expression in the liver, with a significant effect of reducing toxicity and increasing efficacy. Alternatively, the present invention provides a method for increasing the expression of a mRNA composition at the administration site and reducing the expression in the liver and other unrelated organs, wherein said method comprises microneedle administration, and said mRNA composition is encapsulated in LNPs; alternatively, a method for prolonging the duration of *in vivo* expression of a mRNA composition, wherein said method comprises microneedle administration, and said mRNA composition is encapsulated in LNPs; alternatively, a method for increasing the antibody titer in response to low-dose mRNA composition, wherein said method comprises microneedle administration, and said mRNA composition is encapsulated in LNPs. The administration described above means that said administration is subcutaneous injection. The mRNA composition exists in aqueous phase solution, wherein said aqueous phase solution is encapsulated in LNPs or the LNPs comprise a mRNA for encoding a substance for treating or preventing a disease. In some embodiments, said aqueous phase solution has a pH of 4.5-6.8

Preliminary studies by the research and development team found that compared to other injection modalities such as intramuscular injection, microneedle administration is capable to reach dermis where a great number of antigen-presenting cells are observed. Such immune cells are critical for initiating immune response and play a role in immune response. Therefore, microneedle delivery of immunologic compositions for anti-tumor treatment potentially improves the efficacy and mainly the immune effect in the dermis, without increasing the expression in the liver and spleen, and even reduces the expression in liver compared with intramuscular injection. Microneedle administration provides a stronger protection for the organism against viruses, but it is safer for internal organs, and has a significant effect of reducing toxicity and increasing efficacy, thus realizing local administration and reducing the potential toxicity of drugs to other organs and tissues.

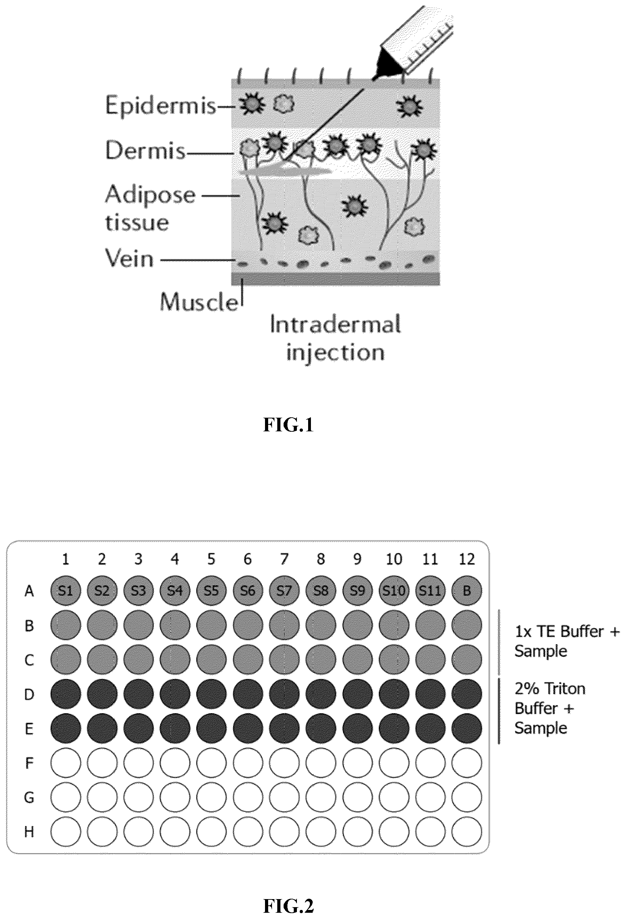

Microneedle can really achieve painless administration, but it is only suitable for small-volume intradermal injections. Due to specific limitations of the structure of intradermal tissue (Figure 1), a single administration can deliver only a very small amount of compositions, so there is an objective need for increasing the effective drug concentration. The volume of administration is very limited, e.g., the volume of administration in the mouse model should not exceed 200 $\mu$L, preferably controlled within 50 $\mu$L, because single administration of high-dose composition will cause reduced transdermal absorption efficiency, or swelling around the site of administration, making administration difficult.

Bispecific antibodies are artificial antibodies with two specific antigen binding sites, which can bridge the target cells and functional molecules (cells) and stimulate directed immune response. As a type of genetic engineering antibodies, bispecific antibodies have now become a hot spot in the field of antibody engineering and have broad application prospects in cancer immunotherapy. T cell-mediated bispecific antibodies, as a type of immunotherapy drug, act by recruiting killer T cells to tumor sites, inducing target-dependent polyclonal T cell activation, releasing cytokines as well as perforin and granzyme, and eventually leading to tumor cell lysis. Researchers are constantly working to develop BsAb with new structures, e.g., concatenating the single-chain fragment variable (scFv) of two different antibodies. Currently, the most successful BsAb is blinatumomab, a type of CD19XCD3 bi-(scFv)2, which can activate and recruit T cells to aggregate onto lymphoma cells. It has been approved for the treatment of acute lymphoblastic leukemia. However, most bispecific antibodies currently face challenges in expression and production, including low yields, poor long-term storage stability, tendency to aggregate over time, and the presence of various impurities. The development of manufacturing processes, production, and testing of new BsAb drugs and development of clinical-grade materials typically take several years. In addition, BsAbs with good efficacy such as blinatumomab have a half-life of less than 2 hours in the patient's serum, and should be continuously administered by infusion pump. Inconvenient administration results in poor patient compliance. In addition, such immunotherapy bispecific antibodies expose patients to the risk of immune factor storm and other toxic side effects. Therefore, it is hoped that the problems in production of BsAbs can be solved by encoding BsAbs with mRNA in the patient's body, in combination with microneedle intradermal administration capable of improving the immune effect to improve immune efficacy with a large number of antigen-presenting cells in the skin, so as to achieve the purpose of reducing the dose and enhancing the immune effect, thus reducing the toxic side effects of immunotherapy drugs.

Existing traditional mRNA compositions (such as mRNA vaccines, BsAb mRNA compositions, etc.) have low drug loading capacity, so a high dose is required to achieve therapeutic effect in organisms, making such compositions difficult for microneedle administration (the dose should be controlled at a milligram (mg) level for microneedle administration), and exposing patients to a potential risk of increased toxic side effects. In addition, more macromolecule excipients are carried into the body due to the low drug loading capacity, posing a higher risk of side effects. Therefore, it is necessary to develop a type of mRNA compositions for microneedle administration with higher drug loading capacity, lower dose and toxic side effects, and better immune effect for both prevention and treatment purposes, e.g., COVID-19 mRNA-based vaccine for microneedle administration or BsAb-coded mRNA composition for microneedle administration, which can produce antibodies for the prevention or treatment of virus infection in humans or mammals.

The LNPs described in the present invention are a drug delivery carrier comprising liposomes formed by one or more different lipid components.

For example, existing COVID-19 mRNA-based vaccine or mRNA-coded BsAb composition has a low drug loading capacity, resulting in a large volume of administration required at the same dose. A single dose of more than 50 μL in mouse model is likely to cause swelling and leakage during microneedle administration, making such composition difficult for microneedle administration, and posing a higher risk of side effects caused by increased macromolecule excipients carried into the body due to low drug loading capacity. Therefore, it is necessary to increase the drug loading capacity of mRNA compositions for purpose of microneedle administration.

In addition, the low drug loading capacity of compositions for microneedle administration will directly affect the therapeutic effect. If the drug content per unit volume (i.e., the drug loading capacity) can be increased, the efficacy via microneedle administration will also be greatly improved.

In the present invention, the mRNA-LNP loading capacity (or the quality of mRNA) of compositions for microneedle intradermal administration is significantly increased by adjusting the pH of the aqueous phase solution during the preparation of mRNA composition. With the same amount of excipient substance, increasing the mRNA loading capacity per unit volume can effectively reduce the volume of administration required at the same dose, enabling microneedle intradermal administration with significant therapeutic effect, and the amount of macromolecular excipients entering the body is reduced accordingly, thus reducing the possible side effects caused by the excipients to a certain extent, e.g., effectively reducing the immunostimulatory effect of components such as PEG in other organs or tissues.

In some embodiments, studies show that, compared with existing aqueous phase solutions containing trimethylamine and triethylamine hydrochloride, the aqueous phase solution containing trimethylamine and Tris hydrochloride provided in the present invention can significantly increase the drug loading capacity of the composition, thus enabling microneedle administration. In addition, the composition has very high long-term stability, and the contents of mRNA and other drug components remain stable, enabling clinical application.

According to the present invention, it is found that maintaining an appropriate pH of the aqueous phase solution in LNPs can significantly increase the drug loading capacity of mRNA composition for microneedle intradermal administration. If the pH of the aqueous phase solution is adjusted to a slightly acidic range such as 4.5-6.8, the amount of loaded mRNA in liposomes could be significantly increased, and it reaches an optimum value under a specific pH condition, i.e., pH 5.0-5.5. The reason may be that, during the preparation, the ionizable lipids are positively charged in an acidic environment, and more nucleic acid molecules (such as mRNA, negatively charged itself) can be adsorbed under the same conditions. This optimum situation appears when the pH of the aqueous phase solution is 5.0-5.5. Therefore, the nucleic acid molecules can be loaded more effectively at pH 4.5-6.8 than pH 7.4 or pH 4.0, resulting in a relatively stable and usable formulation with higher drug loading capacity. Preferably, the pH of said buffer is 4.5-6.0.

The "drug loading capacity" described herein essentially refers to the quantity or mass of active ingredient encapsulated in lipid particles, e.g., it refers to increased weight or quantity of mRNA encapsulated in single lipid particle at an appropriate pH in the present invention. It can also refer to the amount of mRNA or active substance per unit volume of LNPs, that is, the amount of active ingredient per unit volume of LNPs, which is equivalent to the effective drug concentration. The drug in this case can be mRNA. Certainly, it is not limited to mRNA itself, but also may be other auxiliary compositions contained in the aqueous phase solution, which facilitate the activity and stability of mRNA.It can also be the weight or quantity of the mRNA fully encapsulated in the lipid particles at a certain same volume, which will be increased when the pH of the aqueous phase solution changes.

The drug loading capacity of the mRNA composition for microneedle intradermal administration was further increased at pH 4.5-6.0 compared to pH 4.5-6.8, so the pH of the buffer is preferably 4.5-6.0.

In some embodiments, said buffer has a pH of 5.0-5.5; 5.5; 4.5; or 6.0.

In the present invention, extensive experiments proved that adjusting the pH of the buffer from the original 7.4 to 4.5-6.0 can maximize the mRNA loading capacity in LNPs. The reason may be that, during the preparation, the ionizable lipids are positively charged in an acidic environment, and more nucleic acid molecules (such as mRNA, negatively charged itself) are adsorbed under the same conditions. In the present system, during preparation, the ionizable lipids in the corresponding organic phase will have more positive charges when the pH of the aqueous phase solution is 5.5, more nucleic acid molecules can be adsorbed under the same conditions, and nucleic acid molecules can be more tightly encapsulated to obtain a relatively stable formulation. The drug loading capacity can be maximized at different optimal pH values for different buffers. However, it is found that the drug loading capacity can be significantly increased as long as the pH of different buffers is within such a range.

The "aqueous phase solution" described herein can be just water or a buffer containing water, and preferably, said buffer comprises one or more of the following: water, Tris buffer, acetate buffer, phosphate buffer, citrate buffer, carbonate buffer, barbital buffer, TE buffer, and PBS buffer.

In some embodiments, said acetate buffer comprises sodium acetate buffer, ammonium acetate buffer, and lithium acetate buffer.

Preferably, said Tris buffer comprises tromethamine, Tris HCL, glacial acetic acid, sodium acetate trihydrate, and water, and said sodium acetate buffer comprises sodium acetate, glacial acetic acid, and water.

It has been proved that the drug loading capacity of the mRNA composition for microneedle intradermal administration can be significantly increased at pH 4.5-6.8 compared to other pH ranges for any buffer system, even when water is used as the buffer system. That is, the changes in buffer pH directly affect the loading capacity or content of mRNA in LNPs, which is clearly present and is independent of the specific formulation of the buffer system.

According to the description in the invention and the context, it can be understood that, LNPs are prepared with an aqueous phase solution containing mRNA and a lipid solution (organic solution) through a microfluidic chip, and the pH of the aqueous phase solution has a significant effect on the drug loading capacity of the finally formed LNPs. In accordance with the essence of the invention, the drug loading capacity of LNPs can be significantly increased when the pH of the aqueous phase solution is 4.5-6.8 during preparation. It is commonly understood by the technicians in the field that LNPs encapsulate an aqueous phase solution, which contains mRNA capable of encoding active substances for treating diseases, so the pH of the aqueous phase solution encapsulated in lipid particles is actually between 4.5 and 6.8.

In accordance with the spirit or essence of the present invention, it is understood that the pH of the aqueous phase solution in the LNPs is 4.5-6.8. The particles are prepared with two solutions of different nature (mRNA-contained aqueous phase solution at a pH of 4.5-6.8, and lipid-contained solution) using a preparation apparatus, with an increased drug loading capacity. The pH of the aqueous phase solution in the lipid particles is also 4.5-6.8 in the final product formed. Of course, when the composition is applied or administered, a solution at a pH similar to that of human blood or human tissue (e.g., 7.0) can be prepared for administration, which contains the LNPs in the present invention. The pH of the aqueous phase solution of the mRNA contained in the LNPs remains 4.5-6.8. It can also be understood by the general technician in the field of the invention.

The said "active substance for treating or preventing a disease" can be any protein, peptide fragment, antigen or antigen fragment, antibody or antibody fragment. These substances are encoded by the mRNA as defined in the present invention. The therapy described herein mainly means that the above active substances can alleviate the symptoms of a disease or cure a disease, such as tumor or cancer. The purpose of disease prevention is to prevent a certain disease from occurring, for example, vaccine compositions, which can be administered to healthy people in advance to prevent the occurrence of a certain disease (e.g., infectious diseases and viral colds). The vaccine composition can be a mRNA encoding an antigen or antigen fragment.

The solvent of lipid is generally organic solvent, such as absolute ethanol. The aqueous phase and organic phase may dissolve each other. However, during microparticle preparation, a form similar to water-in-oil can be formed using a chip, so the pH of the aqueous phase solution in the particle is the pH of the aqueous phase solution itself and will not be substantially affected.

Therefore, it can be understood that there may be differences in the drug loading capacity of the final composition prepared using different buffer systems, and some buffer system preparations enable higher drug loading capacity of LNPs. However, the pH change of the same buffer system will also significantly affect the drug loading capacity. Therefore, regardless of the buffer system used, the pH should be adjusted to 4.5-6.8, preferably 4.5-6.0, so as to prepare mRNA compositions for microneedle intradermal administration with a relatively higher drug loading capacity.

Besides, the pH needed to achieve the maximum drug loading capacity may differ between different buffer solution systems. However, the optimal range of pH is generally 4.5 to 6.8. This range does not include the pH of 4.0 or 7.4, which is typically seen in the current LNPs.

In some embodiments, the said buffer solution system is Tris buffer solution.

In some embodiments, the said Tris buffer solution has a pH of 5.5.

In some embodiments, the mass ratio of tromethamine: Tris hydrochloride: glacial acetic acid: sodium acetate trihydrate: water in the said Tris buffer solution is 99.2:377.6:13.76:64:160000.

Preferably, the said lipid content of the lipid solution is 8-20 mg/mL.

Studies have shown that for lipid components in the LNPs, regardless of its kind, the drug loading capacity of mRNA compositions for microneedle intradermal administration can be significantly improved as long as the pH range of the buffer solution is adjusted to 4.5-6.8. That is, pH changes of the buffer solution have a direct impact on the drug loading capacity of mRNA in LNPs, which is clearly present and is independent of the lipid components in LNPs.

Preferably, the said lipids include ionizable lipids, cholesterol, phospholipids, and PEGylated lipids.

Moreover, a large number of studies have demonstrated that in the present invention, adjusting the lipid content in the composition can help improve the drug loading capacity of liposomes. The said lipids are a complex liposome containing cholesterol, ionizable lipids, phospholipids, and PEGylated lipids. Escalating the lipid content refers to raising up the contents of four lipid components mentioned above simultaneously, so that the lipid content in the lipid solution increases from 7.72 mg/mL to 8-20 mg/mL (preferably 15.44 mg/mL), thus increasing the drug loading capacity of the lipids formulation from about 29.39 μg/mL to at least about 157.75 μg/mL.

Preferably, the said ionizable lipids contain one or more of the following: C12-200, MC3, DLinDMA, DLin-MC3-DMA, DLinkC2DMA, cKK-E12, ICE, HGT5000, HGT5001, OF-02, DODAC, DDAB, DMRIE, DOSPA, DOGS, DODAP, DODMA, DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, SM-102, ALC-0315, HGT4003, and JK-102-CA.

Preferably, the said phospholipids contain one or more of the following: ceramides, cephalin, cerebroside, diacylglycerols, 1,2-dipalmitoyl-sn-glycero-3-phosphoglycerol, sodium salt (DPPG), 1,2-distearoyl-sn-glycero-3-phosphoethanolamine (DSPE), 1,2-distearoyl-sn-glycero-3-phosphocholine (DSPC), 1,2-dipalmitoyl-sn-glycero-3-phosphocholine (DPPC), 1,2-dioleoyl-sn-glycero-3-phosphoethanolamine (DOPE), 1,2-dioleoyl-sn-glycero-3-phosphocholine (DOPC), 1,2-dipalmitoyl-sn-glycero-3-phosphorylethanolamine (DPPE), 1,2-dimyristoyl-sn-glycero-3-phosphoethanolamine (DMPE), 1,2-dioleoyl-sn-glycero-3-phospho-(1'-rac-glycerol) sodium salt (DOPG), 1-palmitoyl-2-oleoyl-phosphatidylethanolamine (POPE), 1-palmitoyl-2-oleoyl-sn-glycero-3-phosphocholine (POPC), 1-stearoyl-2-oleoyl-phosphatidylethanolamine (SOPE), and sphingomyelin.

Preferably, the said PEGylated lipids contain one or more of the following: DMG-PEG1000, DMG-PEG1300, DMG-PEG1500, DMG-PEG1800, DMG-PEG2000, DMG-PEG2200, DMG-PEG2500, DMG-PEG2700, DMG-PEG3000, DMG-PEG3200, DMG-PEG3500, DMG-PEG3700, DMG-PEG4000, DMG-PEG4200, DMG-PEG4500, DMG-PEG4700, DMG-PEG5000, ALC-0159, M-DTDAM-2000, C8-PEG, DOGPEG, ceramide-PEG, and DSPE-PEG.

Preferably, the lipid content in the said lipid solution is 8-20 mg/mL.

Preferably, the said mass ratio of ionizable lipids: cholesterol: phospholipid: PEGylated lipid is (9-10):(3-4):(2-3):1.

In some embodiments, the said ionizable lipids are SM-102 heptadecan-9-yl-8-((2-hydroxyethyl)(6-oxo-6-(undecyloxy) hexyl) amino) octanoate)); the phospholipid is DSPC (1,2-distearoyl-sn-glycero-3-phosphocholine); the PEGylated lipid is DMG-PEG (1,2-dimyristoyl-rac-glycero-3-methoxypolyethylene glycol-2000), with a PEG molecular weight of 1000-5000 Da.

In some embodiments, the said PEGylated lipid is MG-PEG2000. The mass ratio of the said SM-102: cholesterol: DSPC: DMG-PEG2000 is 141.36:59.26:31.44:14.97.

Preferably, the said substances for treating diseases contain one or more from nucleic acid, protein, inorganic salt.

Studies have shown that the substances for treating diseases encapsulated in LNP can be any substances of therapeutic action. Furthermore, regardless of the substances, the fact remains that adjusting pH to an appropriate range can significantly increase the drug loading capacity of LNPs.

Preferably, the said substances for treating diseases can be a mRNA vaccine or a bispecific antibody (BsAb).

Studies have shown that for mRNA in the LNP, regardless of its kind, the drug loading capacity of mRNA compositions for microneedle intradermal administration can be significantly improved as long as the pH range of the buffer solution is adjusted to 4.5-6.8.That is, pH changes of the buffer solution have a direct impact on the drug loading capacity of mRNA in LNPs, which is clearly present and is independent of the type of mRNA in LNPs.

In some embodiments, the said mRNA is a mRNA encoding novel coronavirus.

In some embodiments, the said mRNA is the mRNA encoding the S protein of novel coronavirus.

In some embodiments, the mRNA encoding the S protein of novel coronavirus is S protein-mRNA (expressing the S protein sequence of novel coronavirus), purchased from Afana Biotechnology Company (Lot: 2011092101).

In some embodiments, the said mRNA encoding the bispecific antibody has a sequence as shown in SEQ ID NO: 1.

In another aspect, the present invention provides an application of a composition in preparation of high mRNA-loaded composition for microneedle administration. The said composition comprises an aqueous phase solution and a lipid solution. The lipid solution encapsulates the aqueous phase solution to form LNPs, wherein the aqueous phase solution comprises antigen-encoding mRNA and a buffer solution with a pH of 4.5-6.8.

Preferably, the pH of said buffer is 4.5-6.0.

In another aspect, the present invention provides an application of buffer solution pH in preparation of high mRNA-loaded composition for microneedle administration. The said composition comprises an aqueous phase solution and a lipid solution. The lipid solution encapsulates the aqueous phase solution to form LNPs, wherein the aqueous phase solution comprises antigen-encoding mRNA and a buffer solution with a pH of 4.5-6.8.

Studies have demonstrated that in the present invention, the buffer solution's pH in aqueous phase solution has a direct impact on the drug loading capacity of the mRNA-encoded bispecific antibody for microneedle intradermal administration. Adjusting the pH to an appropriate level can significantly increase the drug loading capacity in the LNPs.

Preferably, the pH of said buffer is 4.5-6.0.

A large number of experiments have shown that in the invention, by adjusting the buffer solution's pH from 4.0 or 7.4 to a range of 4.5-6.8 (preferably 4.5-6.0) can significantly increase the drug loading capacity of liposomes.

In another aspect, the present invention provides an application of lipid content in lipid solution in preparation of high mRNA-loaded composition for microneedle administration. The said composition comprises an aqueous phase solution and a lipid solution. The lipid solution encapsulates the aqueous phase solution to form LNPs, wherein the aqueous phase solution comprises antigen-encoding mRNA and a buffer solution with a pH of 4.5-6.8. The said lipid content in lipid solution is 10-20 mg/mL.

Preferably, the pH of said buffer is 4.5-6.0.

Preferably, the said lipids include ionizable lipids, cholesterol, phospholipids, and PEGylated lipids. The lipids content in the lipid solution is 8-20 mg/mL.

In another aspect, the present invention provides a method for delivering a drug comprising: using a microneedle to deliver lipid nanoparticles, wherein said lipid nanoparticles comprise an aqueous phase solution therein, and said aqueous solution comprises a mRNA for encoding an active substance for treating a disease.

Preferably, the said said administration is subcutaneous injection.

Preferably, the pH of the said aqueous phase solution is 4.5-6.8.

Preferably, the pH of the said aqueous phase solution is 4.5-6.0.

In another aspect, the present invention provides an application of microneedle in an administration device for preparing mRNA composition encoded drug for intradermal administration, wherein said composition refers to compositions for microneedle administration with high drug loading capacity mentioned above.

A large number of studies have demonstrated that in the invention, microneedle administration leads to a lower dose and a higher expression level of mRNA vaccines, mRNA-encoded BsAb or Fluc-mRNA, or a better efficacy of mRNA-encoded therapeutic substances.

The research and development team has found that compared with intramuscular administration and other administration methods, microneedle administration is capable to reach dermis where a great number of antigen-presenting cells are observed, which are critical for initiating immune responses and play important roles during immune responses. Therefore, microneedle administration of immune anti-tumor drugs has the potential to improve the therapeutic efficacy.

Preferably, the said microneedle administration regimen refers to once a week for at least three times.

In some embodiments, the injection dose for mice is 3.6 $\mu$g, with an injection volume less than 0.2 mL.

In some embodiments, the injection dose for humans can be further confirmed based on results from clinical trials.

In another aspect, the present invention provides an application of microneedles in preparation of an mRNA composition administration device for increasing the expression of mRNA compositions at the administration site and reducing the expression in liver.

Studies on the present invention have demonstrated that after administration of mRNA compositions by microneedles, mRNA is primarily expressed in intradermal tissues, skin, and muscles at the administration site, and is rarely expressed in liver and spleen; whereas after intramuscular administration of mRNA compositions, mRNA is primarily expressed in liver and spleen.

In some embodiments, administration of novel coronavirus-based mRNA vaccines by microneedles not only allow a lower dose, but also has a better immune effect.

The mRNA composition for microneedle administration provided in the present invention is manufactured using the microfluidic nanomedicine preparation system.

The mRNA composition for microneedle administration prepared using this method exhibits favorable features of a nanodrug. And the basic performance parameters of prepared nanocarrier such as particle size, particle size distribution, formulation stability are of excellent parameter control and it possesses outstanding microstructure as a drug delivery carrier; in the functional verification at a cell biology level, it displays outstanding biocompatibility, high nucleic acid encapsulation efficiency, and high gene transfection and expression efficiency, safely and effectively realizing encapsulation, delivery and expression of functional genes of mRNA and demonstrating potent biopharmaceutical potential and broad commercial development prospects. Microneedle administration to mice shows an outstanding expression level and a longer expression duration than commonly used tail vein intravenous injection or intramuscular injection and microneedle administration can also increase expression in muscles and decrease expression in liver, demonstrating a prospect to be widely adopted for its potential to reduce toxicity and increase efficacy.

In another aspect, the present invention provides an application of microneedle in preparation of administration device of mRNA composition for prolonging *in vivo* mRNA expression.

In another aspect, the present invention provides an application of microneedle in preparation of administration device of mRNA composition for increasing antibody titer of low-dose mRNA.

Studies on the present invention have shown that the mRNA composition via microneedle administration needs a lower dose but with a better immune effect than intramuscular or intravenous administration and can significantly prolong the mRNA expression duration in the skin and muscles.

Animal experiments have demonstrated that the mRNA through microneedle administration at a very low dose is still able to reach an antibody titer equal to that at a high dose. For example, only a dose of 1.2 $\mu$g can achieve an antibody titer the same as a dose of 10 $\mu$g.

The present invention provides the following benefits:

(1) It can be used to prepare high mRNA-loaded composition for microneedle administration, realizing microneedle intradermal administration of mRNA compositions, mRNA vaccines and mRNA-encoded bispecific antibodies;

(2) It can significantly increase the drug loading capacity of LNPs by adjusting the pH of buffer solution in the mRNA compositions for microneedle intradermal administration from 4.0 or 7.4 to 4.5-6.8, preferably 4.5-6.0;

(3) By increasing the contents of cholesterol, ionizable lipids, phospholipids, and PEGylated lipids in the mRNA9 composition for microneedle intradermal administration, the drug loading capacity of it can be significantly increased;

(4) It increases the drug loading capacity of the mRNA vaccine for microneedle intradermal administration from about 29.39 $\mu$g/mL to a maximum of 157.75 $\mu$g/mL and the drug loading capacity of the mRNA-encoded bispecific antibody drug composition for microneedle intradermal administration from about 45 $\mu$g/mL to a maximum of 102.9 $\mu$g/mL;

(5) The basic performance parameters of prepared nanocarrier such as particle size, particle size distribution, formulation stability are of excellent parameter control and it possesses an outstanding microstructure as a drug delivery carrier;

(6) It has an excellent stability, and can be stored for more than two years, with no degradation of mRNA;

(7) It has been found that mRNA vaccines administrated by microneedle can increase the mRNA expression at the administration site and decrease that in liver, prolonging the duration of the corresponding antibody expression;

(8) In the functional verification at a cell biology level, it displays outstanding biocompatibility, high nucleic acid encapsulation efficiency, and high gene transfection and expression efficiency, safely and effectively realizing encapsulation, delivery and expression of functional genes of mRNA and demonstrating potent biopharmaceutical potential and broad commercial development prospects;

(9) mRNA vaccines through microneedle administration shows an outstanding effect that a very low dose of it is still able to reach a high antibody titer and only a dose of 1.2 $\mu$g can achieve an antibody titer the same as a dose of 10 $\mu$g, thus making it possible to decrease vaccine dose to increase clinical safety.

(10) The detection at cellular level shows that it achieves an outstanding expression of bispecific antibodies and exerts a significant tumor-suppressing effect through microneedle administration to immunodeficient mice.

## Description of Attached Drawings

Figure 1 is a schematic diagram of microneedle intradermal administration;

Figure 2 is a schematic diagram of the sample loading procedure for the 96-well black plate during the measurement of the encapsulation efficiency of the Ribogreen kit in Embodiment 1;

Figure 3 shows the fluorescence imaging results of mRNA-LNP cells with different loading doses after transfection in Embodiment 2, wherein the upper left panel is control, the upper right panel is EGFP-mRNA-LNP at pH 7.0, the lower left panel is EGFP-mRNA-LNP at pH 6.5, and the lower right panel is EGFP-mRNA-LNP at pH 5.5;

Figure 4 shows the fluorescence expression analysis results of mRNA-LNP cells with different loading doses after transfection in Embodiment 2;

Figure 5 compares the cell-level expression of different LNPs in Embodiment 4;

Figure 6 shows the BLT imaging results of Fluc-mRNA expression in mice when using different methods of administration in Embodiment 5;

Figure 7 shows the analysis results of the optical signal intensity at the injection site of mice when using methods of administration in Embodiment 5;

Figure 8 shows the analysis results of the optical signal intensity in the liver of mice when using different methods of drug administration in Embodiment 5:

Figure 9 shows the optical signal intensity changes at the injection site and in the liver of mice injected with different doses of Fluc-mRNA in Embodiment 5, wherein the upper left panel shows the optical intensity changes of Fluc-mRNA at the injection site under different doses, the upper right panel shows the optical intensity changes of Fluc-mRNA in the liver under different doses, and the lower panel shows the total optical intensity changes of Fluc-mRNA under different doses;

Figure 10 shows the optical signal intensity changes at the injection site and in the liver of the mice when using different methods of administration in Embodiment 5, wherein the upper left panel compares the expression of Fluc-mRNA at the injection site across different methods of administration, and the upper right panel compares the expression of Fluc-mRNA in the liver across different methods of administration, and the lower panel compares the total expression of Fluc-mRNA across different methods of administration;

Figure 11 shows the analysis results of the optical signal intensity at the injection site and in the liver of mice 48 hours after drug administration with different methods in Embodiment 5, wherein the upper left panel compares the expression of Fluc-mRNA at the injection site after 48 hours, the upper right panel compares the expression of Fluc-mRNA in the liver after 48 hours, and the lower panel compares the total expression of Fluc-mRNA after 48 hours;

Figure 12 shows the BLT imaging results of different parts of the mouse body across different methods of drug administration in Embodiment 5;

Figure 13 presents the analysis of the fluorescence intensity of different parts of the mouse body when using different methods of administration in Embodiment 5;

Figure 14 is the flowchart of drug administration and blood sample collection in mice in Embodiment 6;

Figure 15 shows the assay results of the expression of SARS-CoV-2 S proteins in mouse serum in Embodiment 6;

Figure 16 and Figure 17 show the assay results of SARS-CoV-2 S protein antibody titer in mouse serum in Embodiment 6.

Figure 18 presents the expression results of ZSL303-mRNA-1 (CD3×EPCAM-mRNA) in Embodiment 7, respectively at 24 h and 36 h, when using transfection compositions Lipo2k and LipoMAX;

Figure 19 shows the plasma concentration-time curve of the BsAb CD3×EpCAM in mice in Embodiment 8;

Figure 20 is a schematic diagram of the animal study protocol for ZSL303-mRNA-1-LNP administration in Embodiment 9;

Figure 21 shows the tumor volume vs. time curves of HCT-15 xenograft tumors in PBMC humanized mice when using two different methods of drug administration in Embodiment 9;

Figure 22 shows the tumor-inhibiting effect of three microneedle administrations in Embodiment 9;

Figure 23 shows the imaging results of mouse solid tumors in Embodiment 9;

Figure 24 compares the tumor weights in mice 23 days after administration with two different methods in Embodiment 9;

Figure 25 presents the concentration assessment results of BsAbs in mouse serum after three drug administrations in Embodiment 9.

## Detailed Description of the invention

Definition

Unless defined otherwise, all technical and scientific terms used herein have the same meanings as commonly understood by the technicians in the field of the present invention. The references below provide the technicians in the field of the present invention with general definitions of most terms used in the present invention: Dictionary of Biochemistry and Molecular Biology (Second Edition), edited by J. Stenesh and published by Wiley-Interscience in 1989; Dictionary of Microbiology and Molecular Biology (Third Edition), edited by P. Singleton and D. Sainsbury and published by Wiley-Interscience in 2007; Chambers Dictionary of Science and Technology (Second Edition), edited by P. Walker and published by Chambers in 2007; Glossary of Genetics (Fifth Edition), edited by R. Rieger et al. and published by Springer-Verlag in 1991; The HarperCollins Dictionary of Biology, edited by W.G. Hale and J.P. Margham and published by HarperCollins in 1991.

Although any methods and compositions similar or equivalent to those described herein can also be used for the practice or testing of the present invention, the claim herein describes the preferred methods and compositions. For the purpose of the present invention, the terms below are defined for clarity and ease of reference: According to the long-standing patent law practice, when a term is used in the claims of this application without a quantity limitation, it means "one or more". The terms "about" and "approximately" are interchangeable as used herein and can be generally understood to refer to a range of numbers around a given number, as well as all numbers within the described range. Besides, all numerical ranges herein shall be understood to include all integers within that range.

**Nucleic Acid**

The term "nucleic acid" encompasses any compounds and/or substances that can be incorporated into the oligonucleotide chains. Exemplary nucleic acids used in accordance with this application include but are not limited to DNA, RNA (messenger RNA (mRNA) and its hybrids included), RNA interference (RNAi), siRNA, shRNA, miRNA, antisense RNA, ribozymes, catalytic DNA, RNA inducing formation of triple helix, aptamers, and carriers etc., as described in detail herein.

The term "desoxyribonucleic acid", "DNA", or "DNA molecule" refers to a molecule composed of two polynucleotide chains, each of which contains monomer nucleotides. Nucleotides are connected to each other in a chain through covalent bonds between the carbohydrate of one nucleotide and the phosphate group of another nucleotide, creating an alternating carbohydrate-phosphate backbone. Hydrogen bonds connect the nitrogenous bases of the two separate polynucleotide chains to make a double-stranded DNA.

The term "ribonucleic acid", "RNA", or "RNA molecule" refers to a strand of at least 2 base-glycosyl-phosphate combinations. In one embodiment, the term includes compounds consisting of nucleotides, with the carbohydrate part being a ribose. In another embodiment, the ends include RNA and RNA derivatives in which the main strand has been modified. In one embodiment, RNA may exist in the form of tRNA (transfer RNA), snRNA (small nuclear RNA), rRNA (ribosomal RNA), mRNA (messenger RNA), antisense RNA, small interfering RNA (siRNA), microRNA (miRNA), and ribozymes. The intended purposes of siRNA and miRNA have already been described (Caudy A A et al, Genes & Deve116: 2491-96 and references cited therein). Besides, all of the above forms of RNA may be single-stranded, double-stranded, triple-stranded, or quadruple-stranded. In another embodiment, the term comprises artificial nucleic acids with backbone of other types but the same bases. In another embodiment, the artificial nucleic acid is PNA (peptide nucleic acid). PNA consists of a peptide backbone and nucleotide bases, which can bind to DNA and RNA molecules in another embodiment. In another embodiment, the nucleotide is modified oxetane. In another embodiment, the thiophosphate bond substitutes one or more phosphodiester bonds to modify the nucleotide. In another embodiment, the modified nucleic acids include any other variants of the phosphate backbone of known natural nucleic acids in the present field. Ordinary technicians in the present field are familiar with the intended purposes of thiophosphate nucleic acids and PNA, as described in: Neilsen P E, CurrOpin Struct Biol 9: 353-57; [0280] and Raz N Ket al BiochemBiophys Res Commun. 297:1075-84. Technicians in the present field are familiar with the production and usage of nucleic acids, as described in Molecular Cloning, (2001), Sambrook and Russell, eds. And Methods in Enzymology: Methods for molecular cloningin eukaryotic cells (2003) Purchio and G. C. Fareed. Each nucleic acid derivative represents a separate embodiment of the present invention.

As used herein, the term "nucleic acid" refers to one or more types below: polydeoxyribonudeotide (containing 2-deoxy-D-ribose), polyribonucleotide (containing D-ribose), and any other types of polynucleotide, which is the N-glucoside with purine or pyrimidine bases or modified purine or pyrimidine bases (containing abasic sites). As used herein, the term "nucleic acid" also refer to polymers formed by covalent bonding between ribonucleotides or deoxyribonucleosides. The said covalent bonding is usually the phosphodiester bond between subunits or the phosphorothioate bond, methylphosphonate bond in some circumstances. The term "nucleic acid" encompasses single- and double-stranded DNA and single- and double-stranded RNA. Exemplary nucleic acids include but are not limited to gDNA, hnRNA, mRNA, rRNA, tRNA, microRNA (miRNA), small interfering RNA (siRNA), small nucleolar RNA (snoRNA), small nuclear RNA (snRNA), small temporal RNA (stRNA), and any combinations thereof.

**Modified Nucleotides**

In some embodiments, the said mRNA contains modified nucleotides, which include one or more of nucleotides below: 2-aminoadenosine, 2-thiothymidine, inosine, pyrropyrimidine, 3-methyladenosine, 5-methylcytidine, C-5propynyl-cytidine, C-5-propynyl-uridine, 2-aminoadenosine, C5-broxuridine, C5-floxuridine, C5-propynyl-uridine, C5-propynyl-cytidine, C5-methylcytidine, 2-aminoadenosine, 7-deazaadenosine, 7-deazaguanosine, 8-oxyadenosine, 8-oxyguanosine, O(6)-methylguanine, pseudouridine, N-1-methyl-pseudouridine, 2-thiouridine, and 2-thiocytidine; methylated bases; inserted bases; 2'-fluororibose, ribose, 2'-deoxyribose, arabinose, and hexose; thiophosphate, and 5'-N-phosphoramide bond. It can also be the modified nucleotides described in PCT/CN2020/074825 and PCT/CN2020/106696.

**mRNA**

The mRNA described in the present invention may consist of at least two nucleotides. The nucleotides can be either natural or modified. In some embodiments, RNA contains about 5 to about 5000 nucleotides. In some embodiments, RNA contains at least 5 nucleotides. In some embodiments, RNA contains at most 5000 nucleotides. In some embodiments, an RNA molecule contains about 5 to about 20, about 5 to about 40, about 5 to about 60, about 5 to about 80, about 5 to about 100, about 5 to about 200, about 5 to about 500, about 5 to about 1000, about 5 to about 2000, about 5 to about 5000, about 20 to about 40, about 20 to about 60, about 20 to about 80, about 20 to about 100, about 20 to about 200, about 20 to about 500, about 20 to about 1,000, about 20 to about 2,000, about 20 to about 5,000, about 40 to about 60, about 40 to about 80, about 40 to about 100, about 40 to about 200, about 40 to about 500, about 40 to about 1000, about 40 to about 2000, about 40 to about 5000, about 60 to about 80, about 60 to about 100, about 60 to about 200, about 60 to about 500, about 60 to about 1000, about 60 to about 2,000, about 60 to about 5,000, about 80 to about 100, about 80 to about 200, about 80 to about 500, about 80 to about 1,000, about 80 to about 2000, about 80 to about 5000, about 100 to about 200, about 100 to about 500, about 100 to about 1000, about 100 to about 2000, about 100 to about 5000, about 200 to about 500, about 200 to about 1000, about 200 to about 2000, about 200 to about 5000, about 500 to about 1000, about 500 to about 2000, about 500 to about 5000, about 1000 to about 2000, about 1000 to about 5000, or about 2000 to about 5000 nucleotides. In some embodiments, an RNA molecule contains about 5, about 20, about 40, about 60, about 80, about 100, about 200, about 500, about 1000, about 2000, or about 5000 nucleotides.

mRNA can include at least one said modified nucleotide in this application. In some embodiments, an RNA molecule contains about 1 to about 100 modified nucleotides. In some embodiments, an RNA molecule contains at least about 1 modified nucleotide. In some embodiments, an RNA molecule contains at most about 100 modified nucleotides. In some embodiments, an RNA molecule consists of about 1 to about 2, about 1 to about 3, about 1 to about 4, about 1 to about 5, about 1 to about 10, about 1 to about 20, about 1 to about 100, about 2 to about 3, about 2 to about 4, about 2 to about 5, about 2 to about 10, about 2 to about 20, about 2 to about 100, about 3 to about 4, about 3 to about 5, about 3 to about 10, about 3 to about 20, about 3 to about 100, about 4 to about 5, about 4 to about 10, about 4 to about 20, about 4 to about 100, about 5 to about 10, about 5 to about 20, about 5 to about 100, about 10 to about 20, about 10 to about 100, or about 20 to about 100 modified nucleotides. In some embodiments, an RNA molecule contains about 1, about 2, about 3, about 4, about 5, about 10, about 20, or about 100 modified nucleotides.

mRNA can include at least 0.1% modified nucleotides. The proportion of modified nucleotides is calculated as follows: number of modified nucleotides/total number of nucleotides * 100%. In some embodiments, an RNA molecule contains about 0.1% to about 100% modified nucleotides. In some embodiments, an RNA molecule contains at least about 0.1% modified nucleotides. In some embodiments, an RNA molecule contains at most about 100% modified nucleotides. In some embodiments, an RNA molecule contains about 0.1% to about 0.2%, about 0.1% to about 0.5%, about 0.1% to about 1%, about 0.1% to about 2%, about 0.1% to about 5%, about 0.1% to about 10%, about 0.1% to about 20%, about 0.1% to about 50%, about 0.1% to about 100%, about 0.2% to about 0.5%, about 0.2% to about 1%, about 0.2% to about 2%, about 0.2% to about 5%, about 0.2% to about 10%, about 0.2% to about 20%, about 0.2% to about 50%, about 0.2% to about 100%, about 0.5% to about 1%, about 0.5% to about 2%, about 0.5% to about 5%, about 0.5% to about 10%, about 0.5% to about 20%, about 0.5% to about 50%, about 0.5% to about 100%, about 1% to about 2%, about 1% to about 5%, about 1% to about 10%, about 1% to about 20%, about 1% to about 50%, about 1% to about 100%, about 2% to about 5%, about 2% to about 10%, about 2% to about 20%, about 2% to about 50%, about 2% to about 100%, about 5% to about 10%, about 5% to about 20%, about 5% to about 50%, about 5% to about 100%, about 10% to about 20%, about 10% to about 50%, about 10% to about 100%, about 20% to about 50%, about 20% to about 100%, or about 50% to about 100% modified nucleotides. In some embodiments, an RNA molecule contains about 0.1%, about 0.2%, about 0.5%, about 1%, about 2%, about 5%, about 10%, about 20%, about 50%, or about 100% modified nucleotides.

The total concentration of nucleotides used in reaction, such as ribonucleotides (e.g., varying combinations of ATP, GTP, CTP, and UTP), ranges from 0.5 mM to about 500 mM. In some embodiments, the total concentration of nucleotides ranges from 0.5 mM to about 500 mM. In some embodiments, the total concentration of nucleotides is at least about 0.5 mM. In some embodiments, the total concentration of nucleotides is at most about 500 mM. In some embodiments, the total nucleotide concentration is about 0.5 mM to about 1 mM, about 0.5 mM to about 5 mM, about 0.5 mM to about 10 mM, about 0.5 mM to about 50 mM, about 0.5 mM to about 100 mM, about 0.5 mM to about 200

mM, about 0.5 mM to about 300 mM, about 0.5 mM to about 500 mM, about 1 mM to about 5 mM, about 1 mM to about 10 mM, about 1 mM to about 50 mM, about 1 mM to about 100 mM, about 1 mM to about 200 mM, about 1 mM to about 300 mM, about 1 mM to about 500 mM, about 5 mM to about 10 mM, about 5 mM to about 50 mM, about 5 mM to about 100 mM, about 5 mM to about 200 mM, about 5 mM to about 300 mM, about 5 mM to about 500 mM, about 10 mM to about 50 mM, about 10 mM to about 100 mM, about 10 mM to about 200 mM, about 10 mM to about 300 mM, about 10 mM to about 500 mM, about 50 mM to about 100 mM, about 50 mM to about 200 mM, about 50 mM to about 300 mM, about 50 mM to about 500 mM, about 100 mM to about 200 mM, about 100 mM to about 300 mM, about 100 mM to about 500 mM, about 200 mM to about 300 mM, about 200 mM to about 500 mM, or about 300 mM to about 500 mM. In some embodiments, the total concentration of nucleotides is about 0.5 mM, about 1 mM, about 5 mM, about 10 mM, about 50 mM, about 100 mM, about 200 mM, about 300 mM, or about 500 mM.

## Post-synthetic Processing

The 5' cap and/or 3' tail can be added to mRNA after synthesis. The cap can provide nuclease resistance discovered in most eukaryotic cells. The "tail" can protect mRNA from exonuclease-mediated degradation and/or regulate the protein expression level.

The 5' cap can be added according to the following procedures: First, phosphatase at the end of RNA removes a terminal phosphate group from the 5' nucleotide to leave two terminal phosphate groups; second, the guanylyl transferase transfers the guanosine triphosphate (GTP) to the terminal phosphate group to produce 5,5,5 triphosphate bond; third, methyltransferase is used for methylation of N7 of guanine. Embodiments of cap structure include but are not limited to m7G(5')ppp(5'(A,G(5')ppp(5')A and G(5')ppp(5')G. More cap structures have been described in U.S. Patent No.US 2016/0032356. The cap structures described in the following are incorporated into the present invention through citations: Ashiqul Haque et al., Chemically modified hCFTR mRNAs recuperate lung function in a mouse model of cystic fibrosis, Scientific Reports (2018) 8:16776, and Kore et al., Recent Developments in 5'-Terminal Cap Analogs: Synthesis and Biological Ramifications, Mini-Reviews in Organic Chemistry, 2008, 5, 179-192.

The tail structures include poly(A) tail and/or poly(C) tail. The poly(A) tail at the 3' end of mRNA (e.g., 10, 20, 30, 40, 50, 60, 70, 80, 90, or 100 nucleotides at the 3' end) may contain at least 50%, 55%, 65%, 70%, 75%, 80%, 85%, 90%, 92%, 94%, 95%, 96%, 97%, 98%, or 99% adenosine nucleotides. The poly(A) tail at the 3' end of mRNA (e.g., 10, 20, 30, 40, 50, 60, 70, 80, 90, or 100 nucleotides at the 3' end) may contain at least 50%, 55%, 65%, 70%, 75%, 80%, 85%, 90%, 92%, 94%, 95%, 96%, 97%, 98%, or 99% cytosine nucleotides.

As described herein, adding the 5' cap and/or 3' tail can help with the detection of invalid transcripts generated during *in vitro* synthesis. Due to lack of a cap and/or a tail, the prematurely terminated mRNA transcripts may be too small in size to be detected. Therefore, in some embodiments, the 5' cap and/or 3' tail is added to the synthetic mRNA before testing the mRNA purity (e.g., the level of invalid transcripts in mRNA). In some embodiments, as described herein, the 5' cap and/or 3' tail is added to the synthetic mRNA before purifying mRNA. In other embodiments, as described herein, the 5' cap and/or 3' tail is added to the synthetic mRNA after purifying mRNA.

In addition to the above methods, the capping or tailing is completed during the *in vitro* transcription process in which DNA is transcribed into RNA. Ordinary technicians in the present field are familiar with these methods and can freely choose them.

mRNA synthesized according to the present invention does not need to be further purified before use. Specifically, mRNA synthesized according to the present invention is qualified for use with no need to remove short polymers. In some embodiments, mRNA synthesized according to the present invention needs to be further purified. The present invention allows the use of various methods to purify the synthetic mRNA. For example, mRNA can be purified by centrifugation, filtration, and/or chromatographic process. In some embodiments, the synthetic mRNA can be purified by ethanol precipitation, filtration, chromatographic process, gel purification, or any other suitable methods. In some embodiments, mRNA is purified by HPLC. In some embodiments, mRNA is extracted form standard phenol: chloroform: isoamyl alcohol solution, which is widely known among technicians in the present field. In some embodiments, mRNA is purified by tangential flow filtration. Appropriate purification methods include the methods described in US 2016/0040154, US 2015/0376220, PCT/US18/19954 (PCT application named "Method for purifying mRNA") submitted on February 27, 2018, and PCT/US18/19978 (PCT application named "Method for mRNA purification" ) submitted on February 27, 2018, which have been incorporated into the present application through citations and can be used to implement the present invention.

In some embodiments, mRNA is purified before capping and tailing. In some embodiments, mRNA is purified after capping and tailing. In some embodiments, mRNA is purified both before and after capping and tailing. In some embodiments, mRNA is purified by centrifugation, either before or after, or both before and after capping and tailing. In some embodiments, mRNA is purified by filtration, either before or after, or both before and after capping and tailing. In some embodiments, mRNA is purified by tangential flow filtration (TFF) or chromatographic process, either before or after, or both before and after capping and tailing.

In some embodiments, tailing is completed simultaneously with transcription. Therefore, the nucleic acids can also be purified after capping and tailing using the purification methods mentioned above. Therefore, in some embodiments, the purification procedure comes after tailing. Certainly, mRNA can be purified before capping, or just following the transcription. Any available methods in the present field can be used to detect and quantify the full length of mRNA or invalid transcripts. In some embodiments, synthetic mRNA molecules can be detected by Western Blot, capillary electrophoresis, chromatography, fluorescence, gel electrophoresis, HPLC, silver staining, spectroscopy, ultraviolet radiation (UV), or UPLC, or combinations of any of the above. The present invention covers other known detection methods in the present field. In some embodiments, mRNA is detected via capillary electrophoresis using UV absorption spectroscopy. In some embodiments, mRNA is denatured with glyoxal before gel electrophoresis. In some embodiments, the synthetic mRNA is characterized before capping and tailing. In some embodiments, the synthetic mRNA is characterized after capping and tailing.

In some embodiments, the mRNA prepared according to the present invention basically does not contain short polymers or invalid transcripts. Specifically, as shown by capillary electrophoresis or glyoxal gel electrophoresis, the mRNA prepared according to the present invention contains undetectable short polymers or invalid transcripts. As used herein, the terms "short polymers" and "invalid transcripts" refer to any transcript shorter than the full-length transcript. In some embodiments, "short polymers" or "invalid transcripts" are 100 or less, 90 or less, 80 or less, 70 or less, 60 or less, 50 or less, 40 or less, 30 or less, 20 or less, or 10 or less nucleotides in length. In some embodiments, the short polymers are detected or quantified after adding the 5'-cap and/or 3'-poly(A) tail.

**UTR Sequence**

3'-Untranslated Region (3'-UTR): Generally, the term "3'-UTR" refers to a part of the artificial nucleic acid molecule, which is located at 3' (i.e. "downstream") of an open reading frame and is not translated into protein. Typically, a 3'-UTR is a part of a mRNA, which is located between the protein coding region (open reading frame (ORF) or coding sequence (CDS) and the poly(A) sequence of the mRNA. In the context of the present invention, the term 3'-UTR may also comprise elements, which are not encoded in the template, from which an RNA is transcribed, but which are added after transcription during maturation, e.g. a poly(A) sequence. A 3'-UTR of the mRNA is not translated into an amino acid sequence. The 3'-UTR sequence is generally encoded by the gene, which is transcribed into the respective mRNA during the gene expression process. The genomic sequence is first transcribed into pre-mature mRNA, which comprises optional introns. The pre-mature mRNA is then further processed into mature mRNA in a maturation process. This maturation process comprises the steps of 5'capping, splicing the pre-mature mRNA to excise optional introns and modifications of the 3'-end, such as polyadenylation of the 3'-end of the pre-mature mRNA and optional endo-/or exonuclease cleavages, etc. In the context of the present invention, a 3'-UTR corresponds to the sequence of a mature mRNA which is located between the stop codon of the protein coding region, preferably immediately 3' to the stop codon of the protein coding region, and the poly(A) sequence of the mRNA. The term "corresponds to" means that the 3'-UTR sequence may be an RNA sequence, such as in the mRNA sequence used for defining the 3'-UTR sequence, or a DNA sequence, which corresponds to such RNA sequence. In the context of the present invention, the term "a 3'-UTR of a gene" is the sequence, which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence (both sense and antisense strand and both mature and immature) of the 3'-UTR. Preferably, 3'-UTR has a length of more than 20, 30, 40, or 50 nucleotides. 3'-untranslated region (3'-UTR): Typically, a 3'-UTR is a part of an mRNA, which is located between the protein coding region (open reading frame (ORF)) and the poly(A) sequence of the mRNA. The 3'-UTR of mRNA is not translated into amino acid sequences. In the context of the present invention, the 3'-UTR corresponds to the mature mRNA sequence located at the 3' end of the protein-coding region stop codon, preferably immediately adjacent to the 3' end of the protein-coding region stop codon, and extending towards the 5' side of the polyadenylate sequence, preferably towards the nucleotide immediately adjacent to the 5' side of the polyadenylate sequence. The term "corresponds to" means that the 3'-UTR sequence can be an RNA sequence in the mRNA sequence used to define the 3' UTR sequence, or a DNA sequence corresponding to this RNA sequence. In the context of the present invention, the term "a 3'-UTR of a gene", such as "a 3'-UTR of an albumin gene", is the sequence, which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence.

5'-untranslated region (5'-UTR): Generally, the term "5'-UTR" refers to a part of an artificial nucleic acid molecule located at 5' (i.e. "upstream") of the open reading frame and is not translated into protein. A 5'-UTR is typically understood to be a particular section of messenger RNA (mRNA). It is located at 5' of the open reading frame of the mRNA. Typically, the 5'-UTR starts with the transcriptional start site and ends one nucleotide before the start codon of the open reading frame. Preferably, the said 5'-UTR has a length of more than 20, 30, 40, or 50 nucleotides. The 5'-UTR may comprise elements for controlling gene expression, which are also called regulatory elements. Such regulatory elements may be, for example, ribosomal binding sites. The 5'-UTR may be post-transcriptionally modified, for example by addition of a 5'-cap. The 5'-UTR of mRNA is not translated into amino acid sequences. The 5'-UTR sequence is usually encoded by genes transcribed into various mRNAs during gene expression. The genome sequence is first transcribed into premature mRNAs containing optional introns. The premature mRNAs are then further processed into mature mRNAs during the maturation process. The maturation process includes the following steps: 5' capping, splicing of premature mRNAs to remove optional introns, and 3' end modification (such as polyadenylation of the 3' end of premature mRNAs and selective endonuclease/or exonuclease cleavage). In the context of the present invention, a 5'-UTR corresponds to the sequence of a mature mRNA, which is located between the 5'-cap and the start codon. Preferably, the 5'-UTR corresponds to the sequence, which extends from a nucleotide located 3' to the 5'-cap, preferably from the nucleotide located immediately 3' to the 5'-cap, to a nucleotide located 5' to the start codon of the protein coding region, preferably to the nucleotide located immediately 5' to the start codon of the protein coding region. The nucleotide located immediately 3' to the 5'-CAP of a mature mRNA typically corresponds to the transcriptional start site. The term "corresponds to" means that the 5'-UTR sequence may be an RNA sequence, such as in the mRNA sequence used for defining the 5'-UTR sequence, or a DNA sequence, which corresponds to such RNA sequence. In the context of the present invention, the term "a 5'-UTR of a gene" is the sequence, which corresponds to the 5'-UTR of the mature mRNA derived from this gene, i.e. the mRNA obtained by transcription of the gene and maturation of the pre-mature mRNA. The term "5'-UTR of a gene" includes the DNA sequence and RNA sequence of the 5'-UTR (both sense and antisense chains, as well as mature and immature ones).

As an alternative solution for mRNA stabilization, it has been found that naturally occurring eukaryotic mRNA molecules contain characteristic stabilizing elements. For example, they can contain so-called untranslated regions (UTRs) at their 5' end (5'-UTR) and/or at their 3' end (3'-UTR), as well as other structural features such as 5' cap structures or 3'-polyadenylate tails. Both 5'-UTR and 3'-UTR are typically transcribed from genomic DNA and therefore are premature mRNA elements. During mRNA processing, the unique structural features of the mature mRNA, such as the 5' cap and 3'-polyadenylate tail (also known as polyadenylate tail or polyadenylate sequence), are typically added to the transcribed (premature) mRNA.

The 3'-polyadenylate tail is typically a monotonic adenosine nucleotide sequence added to the 3' end of the transcribed mRNA. It can contain up to approximately 400 adenosine nucleotides. It was found that the length of the 3'-polyadenylate tail may play a key role in the stability of individual mRNAs. In addition, it has been observed that the 3'-UTR of $\alpha$-globulin mRNA may be an important factor for the mRNA stability of this well-known $\alpha$-globulin (Rodgers et al., Regulated alpha-globin mRNA decay is a cytoplasmic event proceeding through 3'-to-5' exosome-dependent decapping, RNA, 8, pp. 1526-1537, 2002). The 3'-UTR of $\alpha$-globulin mRNA is significantly involved in the formation of specific nuclear protein complexes (a-complexes), whose existence is related to the *in vitro* stability of mRNA (Wang et al., An mRNA stability complex functions with poly(A)-binding protein to stabilize mRNA in vitro, Molecular and Cellular biology, Vol. 19, No. 7, July 1999, pp. 4552-4560). It has been further demonstrated that the UTR in ribosomal protein mRNAs has an interesting regulatory function: While the typical growth-associated translational regulation is bestowed on an mRNA by the 5'-UTR in ribosomal protein mRNAs, the stringency of regulation depends on each 3'-UTR in ribosome protein mRNAs (Ledda et al., Effect of the 3'-UTR length on the translational regulation of 5'-terminal oligopyrimidine mRNAs, Gene, Vol. 344, 2005, pp. 213-220). This mechanism promotes the specific expression of ribosomal proteins, which are usually transcribed in a constant manner, so some ribosomal protein mRNAs, such as ribosomal protein S9 or ribosomal protein L32, are called housekeeping genes (Janovick Gutzky et al., Housekeeping gene expression in bovine liver is affected by physiological state, feed intake, and dietary treatment, J. Day Sci., Vol. 90, 2007, pp. 2246-2252). The growth related expression pattern of ribosomal proteins is therefore mainly due to the regulation at the translational level.

The term "3'-UTR element" refers to a nucleic acid sequence containing or composed of nucleic acid sequences derived from 3'-UTR or variants or fragments of 3'-UTR. The "3'-UTR element" preferably refers to an artificial nucleic acid sequence, such as the nucleic acid sequence contained in the 3'-UTR of an artificial mRNA. Therefore, in the meaning of the present invention, preferably, the 3'-UTR element can be contained in the 3'-UTR of mRNA, preferably an artificial mRNA, or the 3'-UTR element can be contained in the 3'-UTR of its respective transcription templates. Preferably, the 3'-UTR element corresponds to the 3'-UTR of mRNA, preferably an artificial mRNA, such as the nucleic acid sequence of the 3'-UTR of mRNA obtained through transcriptional gene modification of vector constructs. Preferably, the 3'-UTR element in the meaning of the present invention serves as a nucleotide sequence that performs the function of the 3'-UTR or encodes the execution of the 3'-UTR function.

Therefore, the term "5'-UTR element" refers to a nucleic acid sequence containing or composed of nucleic acid sequences derived from 5'-UTR or variants or fragments of 5'-UTR. The "5'-UTR element" preferably refers to an artificial nucleic acid sequence, such as the nucleic acid sequence contained in the 5'-UTR of an artificial mRNA. Therefore, in the meaning of the present invention, preferably, the 5'-UTR element can be contained in the 5'-UTR of mRNA, preferably an artificial mRNA, or the 5'-UTR element can be contained in the 5'-UTR of its respective transcription templates. Preferably, the 5'-UTR element corresponds to the 5'-UTR of mRNA, preferably an artificial mRNA, such as the nucleic acid sequence of the 5'-UTR of mRNA obtained through transcriptional gene modification of vector constructs. Preferably, the 5'-UTR element in the meaning of the present invention serves as a nucleotide sequence that performs the function of the 5'-UTR or encodes the execution of the 5'-UTR function.

According to the present invention, the 3'-UTR element and/or 5'-UTR element in the artificial nucleic acid molecule extend(s) and/or increase(s) the protein production of the said artificial nucleic acid molecule. Therefore, the artificial nucleic acid molecule according to the present invention can particularly include 3'-UTR elements and/or 5'-UTR elements with one or several functions as follows: 3'-UTR elements increasing the protein production of the said artificial nucleic acid molecule, 3'-UTR elements extending the protein production of the said artificial nucleic acid molecule, 3'-UTR elements increasing and extending the protein production of the said artificial nucleic acid molecule, 5'-UTR elements increasing the protein production of the said artificial nucleic acid molecule, 5'-UTR elements extending the protein production of the said artificial nucleic acid molecule, 5'-UTR elements increasing and extending the protein production of the said artificial nucleic acid molecule, 3'-UTR elements increasing the protein production of the said artificial nucleic acid molecule and 5'-UTR elements increasing the protein production of the said artificial nucleic acid molecule, 3'-UTR elements increasing the protein production of the said artificial nucleic acid molecule and 5'-UTR elements extending the protein production of the said artificial nucleic acid molecule, 3'-UTR elements increasing the protein production of the said artificial nucleic acid molecule and 5'-UTR elements increasing and extending the protein production of the said artificial nucleic acid molecule, 3'-UTR elements extending the protein production of the said artificial nucleic acid molecule and 5'-UTR elements increasing the protein production of the said artificial nucleic acid molecule, 3'-UTR elements extending the protein production of the said artificial nucleic acid molecule and 5'-UTR elements extending the protein production of the said artificial nucleic acid molecule, 3'-UTR elements extending the protein production of the said artificial nucleic acid molecule and 5'-UTR elements increasing and extending the protein production of the said artificial nucleic acid molecule, 3'-UTR elements increasing and extending the protein production of the said artificial nucleic acid molecule and 5'-UTR elements increasing the protein production of the said artificial nucleic acid molecule, 3'-UTR elements increasing and extending the protein production of the said artificial nucleic acid molecule and 5'-UTR elements extending the protein production of the said artificial nucleic acid molecule, or 3'-UTR elements increasing and extending the protein production of the said artificial nucleic acid molecule and 5'-UTR elements increasing and extending the protein production of the said artificial nucleic acid molecule. Preferably, the artificial nucleic acid molecule according to the present invention includes 3'-UTR elements extending the protein production of the said artificial nucleic acid molecule and/or 5'-UTR elements increasing the protein production of the said artificial nucleic acid molecule. Preferably, the artificial nucleic acid molecule according to the present invention comprises at least one 3'-UTR element and at least one 5'-UTR element, that is, at least one 3'-UTR element extending and/or increasing the protein production of the artificial nucleic acid molecule and derived from a stable mRNA, and at least one 5'-UTR element extending and/or increasing the protein production of the artificial nucleic acid molecule and derived from a stable mRNA. "Extending and/or increasing protein production of the said artificial nucleic acid molecule" typically refers to the amount of protein produced from the artificial nucleic acid molecules according to the present invention with each 3'-UTR element and/or 5'-UTR element, compared to the amount of protein produced from each reference nucleic acid molecule lacking 3'-UTR and/or 5'-UTR or containing the reference 3'-UTR and/or 5'-UTR (such as 3'-UTR and/or 5'-UTR naturally present in combination with ORF). In particular, compared to various nucleic acids lacking 3'-UTR and/or 5'-UTR or containing reference 3'-UTR and/or 5'-UTR, such as those naturally present in combination with ORF, at least one 3'-UTR element and/or 5'-UTR element of the artificial nucleic acid molecule according to the present invention extend the production of the protein of artificial nucleic acid molecules according to the present invention, such as that of mRNAs according to the present invention. In particular, compared to various nucleic acids lacking 3'- and/or 5'-UTR or containing reference 3'- and/or 5'-UTR, such as those naturally present in combination with ORF, at least one 3'-UTR element and/or 5'-UTR element of the artificial nucleic acid molecule according to the present invention increase protein production of the artificial nucleic acid molecule according to the present invention, such as that that of mRNAs according to the present invention, especially protein expression and/or total protein production. Preferably, compared to the translation efficiency of various nucleic acids lacking 3'- and/or 5'-UTR or containing the reference 3'- and/or 5'-UTR, such as those naturally combined with ORF, the said at least one 3'-UTR element and/or at least one 5'-UTR element of the artificial nucleic acid molecule according to the present invention do(es) not negatively affect the translation efficiency of the nucleic acid. Even more preferably, the translation efficiency is enhanced by the 3'-UTR and/or 5'-UTR compared to that for the protein encoded by each ORF in its natural state. The term "various nucleic acid molecules" or "reference nucleic acid molecule" used herein means that-except for the difference in 3'-UTR and/or 5'-UTR-reference nucleic acid molecules are equivalent to the artificial nucleic acid molecules of the present invention containing 3'-UTR elements and/or 5'-UTR elements, preferably the same.

**Pharmaceutical Composition**

This application also discloses pharmaceutical compositions including compounds, proteins (antigens, antibodies, antibody fragments, fusion proteins, peptide chains, amino acid sequences, etc.), modified nucleosides, modified nucleotides, or the modified nucleic acids provided in this application.

In some embodiments, the pharmaceutical composition of the present invention can be administered to subjects through any methods known to technicians in this field, such as parenteral, oral, mucosal, percutaneous, intramuscular, intravenous, intradermal, subcutaneous, intraperitoneal, ventricular, intracranial, vaginal, or intratumoral administration.

Pharmaceutical compositions can be administered through intravenous, intra-arterial, or intramuscular injection of liquid formulations. Suitable liquid formulations include solutions, suspensions, dispersions, emulsions, and oils. In some embodiments, the pharmaceutical composition is administered intravenously and is therefore formulated in a form suitable for intravenous administration. In some embodiments, the pharmaceutical composition is administered intra-arterially and is therefore formulated in a form suitable for intra-arterial administration. In some embodiments, the pharmaceutical composition is administered intramuscularly and is therefore formulated in a form suitable for intramuscular administration. Pharmaceutical compositions may be administered using vesicles, for example, liposomes (see Langer, Science 249:1527-1533(1990); Treatetal.,in Liposomes in the Therapy of Infectious Disease and Cancer, Lopez-Berestein and Fidler (eds.), Liss, NewYork, pp. 353-365 (1989); Lopez-Berestein, ibid., pp. 317-327; see generally ibid).

The pharmaceutical composition may be administered orally, so, it can be formulated in a form suitable for oral administration, i.e. solid or liquid formulations. Suitable solid oral formulations may include tablets, capsules, granules, pills, etc. Suitable liquid oral formulations may include solutions, suspensions, dispersions, lotion, and oils.

The pharmaceutical composition can be administered locally to the body surface, so it can be formulated in a form suitable for local administration. Suitable topical formulations may include gel, ointment, cream, lotion, drops, etc. For local administration, the composition or its physiologically tolerable derivatives can be prepared and used as a solution, suspension, or lotion in physiologically acceptable diluents with or without drug carriers. Pharmaceutical compositions can be administered as suppositories, such as rectal or urethral suppositories. In some embodiments, the pharmaceutical composition is administered through subcutaneous implantation of pellets. In some embodiments, the pellets provide controlled release of the medications over a period of time. The pharmaceutical composition may further include pharmaceutically acceptable excipients, as used in this application, including any and all solvents, dispersion media, diluents or other liquid carriers, dispersion or suspension additives, surfactants, isotopes, thickeners or emulsifiers, preservatives, solid adhesives, lubricants, etc., accommodating the specific dosage form required. "Remington: The Science and Practice of Pharmacy," 21st edition, A.R. Gennaro (Lippincott, Williams&Wilkins, Baltimore, Md., 2006; incorporated herein by reference) discloses various excipients used for preparing pharmaceutical compositions and known techniques for their preparation.

In some embodiments, the purity of pharmaceutically acceptable excipients is at least 95%, at least 96%, at least 97%, at least 98%, at least 99%, or 100%. In some embodiments, excipients are approved for human and veterinary use. In some embodiments, excipients are approved by the US Food and Drug Administration. In some embodiments, the excipient is of medicinal grade. In some embodiments, the excipient complies with the standards of the *United States Pharmacopoeia* (USP), *European Pharmacopoeia* (EP), *British Pharmacopoeia,* and/or *International Pharmacopoeia.*

Pharmaceutically acceptable carriers for liquid formulations can be aqueous or nonaqueous solutions, suspensions, lotion, or oils. Examples of nonaqueous solvents can be propylene glycol, polyethylene glycol, and injectable organic esters, such as ethyl oleate. Aqueous carriers may include water, alcohol/water solution, lotion, or suspension, including saline water and buffer medium. Examples of oil can be oil from petroleum, animal, plant, or synthetic sources, such as peanut oil, soybean oil, mineral oil, olive oil, sunflower oil, and cod liver oil.

Non-gastrointestinal drug delivery carriers (used for subcutaneous, intravenous, arterial, or intramuscular injection) may include sodium chloride solution, Ringer's glucose, dextrose and sodium chloride, lactated Ringer's solution, and fixed oil. Intravenous carriers include liquid and nutritional supplements, electrolyte supplements, such as Ringer's glucose-based electrolyte supplements. Examples can be sterile liquids, such as water and oil, with or without the addition of surfactants and other pharmaceutically acceptable adjuvants. Typically, water, saline water, glucose aqueous solutions, and related sugar solutions, as well as glycols such as propylene glycol or polyethylene glycol, are preferred liquid carriers, especially for injectable solutions. Examples of oil can be oil from petroleum, animal, plant, or synthetic sources, such as peanut oil, soybean oil, mineral oil, olive oil, sunflower oil, and cod liver oil.

Pharmaceutical compositions can further include adhesives (such as Acacia senegal, corn starch, gelatin, carbomer, ethyl cellulose, guar gum, hydroxypropyl cellulose, hydroxypropyl methyl cellulose, and povidone), disintegrants (such as corn starch, potato starch, alginate, silicon dioxide, cross-linked sodium carboxymethyl cellulose, cross povidone, guar gum, and sodium starch hydroxyacetate), buffers of various pH and ionic strength (such as Tris-HCl, acetate, and phosphate), albumin or gelatin and other additives to prevent absorption to the surface, detergents (such as Tween 20, Tween 80, Planck F68, and bile salts), protease inhibitors, surfactants (such as sodium dodecyl sulfate), osmotic enhancers, solubilizers (such as glycerol and polyethylene glycol glycerol), antioxidants (such as ascorbic acid, sodium metabisulfite, and butylated hydroxyanisole), stabilizers (such as hydroxypropyl cellulose and hydroxypropyl methyl cellulose), tackifiers (such as carbomer, colloidal silica, ethyl cellulose, and guar gum), sweeteners (such as aspartame and citric acid), preservatives (such as thiomersal, benzyl alcohol, and paraben), lubricants (such as stearic acid, magnesium stearate, polyethylene glycol, and sodium dodecyl sulfate), flow promoters (such as colloidal silica), plasticizers (such as diethyl phthalate and triethyl citrate), emulsifiers (such as carbomer, hydroxypropyl cellulose, and sodium dodecyl sulfate), polymer coatings (such as poloxamer or poloxamide), coatings and film-forming compositions (such as ethyl cellulose, acrylate, and polymethyl acrylate), and/or adjuvants.

The pharmaceutical composition provided in this application can be a controlled release composition, i.e. a composition in which the compound is released within a period of time after administration. Controlled or sustained release compositions may include formulations in lipophilic reservoirs (such as fatty acid, wax, and oil). In some embodiments, the pharmaceutical composition can be an immediate release composition, i.e. a composition in which the entire compound is released immediately after administration.

**Delivery Carrier**

The mRNA contained in the composition of the present invention may be prepared and delivered in any way to induce *in vivo* production of proteins such as antibodies, antigens, antibody fragments, or antigen fragments. In some embodiments, mRNA is encapsulated into transport carriers, such as nanoparticles. Other important purposes of such encapsulation usually include: 1) protecting the nucleic acid from the influence of the environment, which may contain enzymes or chemicals that can degrade the nucleic acid and/or activate systems or receptors that trigger rapid nucleic acid excretion; and 2) facilitating cellular uptake and expression of corresponding sequences. Thus, in some embodiments, a suitable delivery carrier can enhance the stability of its comprised mRNA and/or facilitate the delivery of mRNA to the target cell or tissue. In some embodiments, nanoparticles may be lipid-based, including, for example, liposomes or polymer-based nanoparticles. In some embodiments, the diameter of nanoparticles used as the delivery carrier ranges from 1 to 1000 nm. Each nanoparticle may comprise as low as 0.001 $\mu$g, 0.01 $\mu$g, 0.1 $\mu$g, 1 $\mu$g, 10 $\mu$g, 100 $\mu$g, 1 mg, 10 mg, 100 mg, 1 g or more mRNA.

Of course, nanoparticles can also be particles with a core-shell structure. For instance, the nucleic acid can first be mixed with a polymer to form a core, which is then encapsulated in liposomes. Such a structure can be generated by the mixer in the present invention. The mixer can first mix the nucleic acid with the polymer to form a particulate structure, and then mix this particulate with lipid components to create an even larger particulate structure. The mixer in the present invention can use, for example, all core materials and shell materials listed in patent application No. 201880001680.5 to generate this so-called core-shell structure. All core materials and shell materials in this patent are specific embodiments of the present invention.

In some embodiments, the transport carriers are liposomal vesicles or other substances that can facilitate the transfer of nucleic acid to target cells and tissues. Suitable transport carriers include, but are not limited to, liposomes, nanoliposomes, ceramide-containing nanoliposomes, proteoliposomes, nanoparticles, calcium phosphate-silicate nanoparticles, calcium phosphate nanoparticles, silica nanoparticles, nanocrystalline particles, semiconductive nanoparticles, poly(D-arginine), nanodendrimer, starch-based delivery systems, micelles, emulsions, vesicles, plasmids, viruses, calcium phosphate-based nucleotides, aptamers, peptides, and other carrier tags. Bioionic capsules and other viral capsid protein assemblies are also considered suitable transport carriers. (Hum. Gene Ther. 2008 September;19(9):887-95).

Lipid nanoparticles (LNPs) can comprise one or more ionizable lipids, one or more non-ionizable lipids, one or more sterol-based lipids, and/or one or more PEG-modified lipids. Liposomes can comprise three or more types of lipid components, one of which is a sterol-based lipid. In some embodiments, the sterol-based lipid is imidazole cholesteryl ester or "ICE" lipid (see WO2011/068810, which is incorporated in this application by reference). In some embodiments, the sterol-based lipids constitute no more than 70% (e.g., no more than 65% or 60%) of the total lipids in LNPs (e.g., liposomes). Examples of suitable lipids include, for example, phosphatidyl compounds (e.g., phosphatidylglycerol, phosphatidylcholine, phosphatidylserine, and phosphatidylethanolamine), sphingolipids, cerebrosides, and gangliosides.

Non-limiting examples of ionizable lipids include, but are not limited to, C12-200, MC3, DLinDMA, DLin-MC3-DMA, DLinkC2DMA, cKK-E12, ICE (imidazolyl), HGT5000, HGT5001, OF-02, DODAC, DDAB, DMRIE, DOSPA, DOGS, DODAP, DODMA and DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, SM-102, ALC-0315, HGT4003, JK-102-CA, etc., or the combination thereof.

Non-limiting examples of non-ionizable lipids include, but are not limited to, ceramides, cephalins, cerebrosides, diacylglycerols, 1,2-dipalmitoyl-sn-glycero-3-phosphoglycerol, sodium salt (DPPG), 1,2-distearoyl-sn-glycero-3-phosphoethanolamine (DSPE), 1,2-distearoyl-sn-glycero-3-phosphocholine (DSPC), 1,2-dipalmitoyl-sn-glycero-3-phosphocholine (DPPC), 1,2-dioleyl-sn-glycero-3-phosphoethanolamine (DOPE), 1,2-dioleyl-sn-glycero-3-phosphatidylcholine (DOPC), 1,2-dipalmitoyl-sn-glycero-3-phosphoethanolamine (DPPE), 1,2-dimyristoyl-sn-glycero-3-phosphoethanolamine (DMPE), and 1,2-dioleoyl-sn-glycero-3-phosphate-(1'-rac-glycerol) (DOPG), 1-palmitoyl-2-oleoyl-phosphatidylethanolamine (POPE), 1-palmitoyl-2-oleoyl-sn-glycero-3-phosphocholine (POPC), 1-stearoyl-2-oleoyl-phosphatidylethanolamine (SOPE), sphingomyelin, or the combination thereof.

In some embodiments, the PEG-modified lipid can be a poly(ethylene) glycol chain with a PEG length of 1000-5000 Da, which is covalently attached to a lipid with a C6-C20 long alkyl chain. Non-limiting examples of PEG-modified lipids may include, but are not limited to, DMG-PEG$_{1000}$, DMG-PEG$_{1300}$, DMG-PEG$_{1500}$, DMG-PEG$_{1800}$, DMG-PEG$_{2000}$, DMG-PEG$_{2200}$, DMG-PEG$_{2500}$, DMG-PEG$_{2700}$, DMG-PEG$_{3000}$, DMG-PEG$_{3200}$, DMG-PEG$_{3500}$, DMG-PEG$_{3700}$, DMG-PEG$_{4000}$, DMG-PEG$_{4200}$, DMG-PEG$_{4500}$, DMG-PEG$_{4700}$, DMG-PEG$_{5000}$, ALC-0159, M-DTDAM-2000, C8-PEG, DOGPEG, ceramide PEG, and DSPE-PEG, or a combination thereof.

Polymers may also be considered transport carriers, which can be used either alone or in combination with other transport carriers. Suitable polymers may include polyacrylates, polyalkyl cyanoacrylates, polylactides, polylactide-polyglycolide copolymers, polycaprolactone, dextran, albumin, gelatin, alginate, collagen, chitosan, cyclodextrin, and polyethyleneimine. Polymer-based nanoparticles may include polyethyleneimines (PEIs), such as branched PEIs.

The core-shell structure of the carrier is also another specific embodiment. In some embodiments, the said vaccine composition includes the aforementioned nucleic acid, which can be translated to express the antigen or antigen fragment of coronavirus. This nucleic acid is contained in multiple polyplexes or protein core particles, and said multiple polyplexes or protein core particles are encapsulated in a first-generation biocompatible lipid bilayer shell. In some embodiments, the said polyplex or protein core particle contains a first positively charged polymer or protein. The abovementioned first biocompatible lipid bilayer shell promotes macropinocytosis of multiple polyplexes or protein core particles in one or more types of mammalian antigen-presenting cells. In some embodiments, the vaccine composition also comprises an adjuvant selected from CpG, poly(I.C), alum, or any combination thereof, which is encapsulated within the said biocompatible lipid bilayer. In some embodiments, the vaccine composition also includes an immunomodulatory compound, such as IL-12p70 protein, FLT3 ligand, or an indoleamine 2,3 dioxygenase (IDO-1) inhibitor, encapsulated in the space between the said biocompatible lipid bilayers. In some embodiments, the said indoleamine 2,3-dioxygenase (IDO-1) inhibitor is GDC-0919, INCB24360, or a combination thereof. In some embodiments, the said positively charged polymer or protein includes protamine, polyethyleneimine, poly(P-amino ester), or any combination thereof. In some embodiments, the said biocompatible lipid bilayer comprises one or more of the following substances: 1,2-dioleoyl-sn-glycero-3-ethylphosphocholine (EDOPC); 1,2-dioleyl-sn-glycero-3-phosphoethanolamine (DOPE); 1,2-distearoyl-sn-glycero-3-phosphoethanolamine-N-[amino(polyethylene glycol)-2000] (DSPE-PEG); and the combinations thereof. In some embodiments, the said biocompatible lipid bilayer includes: (a) about 30% to about 70% of 1,2-dioleoyl-sn-glycero-3-ethylphosphocholine (EDOPC); (b) about 70% to about 30% of 1,2-dioleyl-sn-glycero-3-phosphoethanolamine (DOPE); and (c) about 0.5% to about 5% of 1,2-distearoyl-sn-glycero-3-phosphoethanolamine-N-[amino(polyethylene glycol)-2000] (DSPE-PEG). In some embodiments, the said biocompatible lipid bilayer contains: (a) about 45% to about 55% of 1,2-dioleoyl-sn-glycero-3-ethylphosphocholine (EDOPC); (b) about 55% to about 45% of 1,2-dioleyl-sn-glycero-3-phosphoethanolamine (DOPE); and (c) about 1% to about 2% of 1,2-distearoyl-sn-glycero-3-phosphoethanolamine-N-[amino(polyethylene glycol)-2000] (DSPE-PEG).

## Microneedle

The "microneedles" described herein are basically small needles with a length ranging from hundreds of micrometers to several millimeters, which are made of silicon, metal, macromolecular organic matter, or other materials through microelectronics manufacturing technology or microcasting.They can effectively pierce the stratum corneum of the skin and form tiny channels on the skin surface, which thereby allow targeted delivery of drugs to a predetermined depth beneath the skin and their absorption into the blood for effective functionality. Therefore, they act as a microneedle intradermal administration system, encompassing the advantages of both transdermal patching and subcutaneous injection. The main methods of administration of microneedles include "poke and patch", "dip and scrape", "coat and poke", encapsulated drug microneedle, and microneedle administration, etc., which have the advantages of high bioavailability, non-invasiveness, precise dosage control, high stability, and painlessness. They are mainly used for intradermal absorption of macromolecular substances such as proteins, nucleic acids, vaccines, etc. In addition, they are also widely used in the beauty industry. The tiny channels produced by microneedles allow the sweat produced by subcutaneous glands to be better excreted from the body, thereby relieving pore clogging, for example, in oily skin. In addition, they can increase the absorption efficacy of skin products such as emulsion, gel, and lotion, and improve the utilization rate of active ingredients.

Microneedle technology has garnered substantial recognition from international pharmaceutical companies and academic institutions due to its notable advantages of painlessness, minimal invasiveness, enhanced convenience, low risk of accidental acupuncture injury, and promising market potential.The advancement of micro-processing technology, coupled with China's augmented investment in medical research, has led to a steady growth in the number of research projects and applications related to microneedle drug delivery systems within China, which to a certain extent facilitated the progress of micro-needle drug delivery industry in the country.

Microneedles can be defined as needles with a length of 10-2000 μm and a width of 10-50 μm. The drug delivery functionality is achieved through a device known as a microneedle array, wherein an array of microneedles is

**EP 4 292 584 A1**

mounted on a drug delivery carrier.The lengths of microneedles range from a few hundred micrometers to several millimeters. They can pass through the stratum corneum of the skin without reaching the pain nerve, and form drug delivery channels on the skin surface. These channels allow targeted delivery of drugs to a predetermined depth beneath the skin and their absorption into the subcutaneous capillary network. In other words, microneedles can promote drug infiltration without causing pain or skin damage. Therefore, microneedles can help enhance drug delivery efficiency and improve the patient's compliance.

Theoretically, microneedles require a length of only 15-20 $\mu$m to penetrate the human skin's stratum corneum. However, considering the skin's inherent elasticity and stretchability, as well as the significant variation in stratum corneum thickness across different age groups and skin locations, it becomes necessary to utilize microneedles with lengths surpassing 20 $\mu$m to accommodate effective penetration across various skin types and ensure effective intradermal drug delivery, but the length generally falls below 1 mm.The material of microneedles also gradually transitioned from metal at the beginning to dissolvable materials.

Microneedles offer significant advantages, primarily in facilitating the penetration of macromolecules through the stratum corneum, and enabling drug administration that is minimally invasive and virtually painless as compared to injections, which is similar to subcutaneous administration, and therefore they can be easily accepted by patients.In addition, administration through microneedles also allows stable and controlled dose delivery.The application of a micro-needle patch elicits a sensation similar to that of applying a band-aid to the face, causing no discomfort to the patient at all.Therefore, microneedle administration is also called a revolutionary new dosage form and novel delivery method.Microneedle patches possess significant market potential as they have the capability to accommodate a wide range of substances including topical medications, cosmetics, biological compositions, and selected chemical drugs.

Microneedle intradermal administration is suitable for a wide range of applications including transdermal delivery of small molecules, biological compositions, vaccines, intracellular DNA/RNA, and more. Presently, research and development efforts surrounding microneedles primarily focus on their applications in vaccination, diabetes treatment, skin disease treatment, and aesthetic medicine.

In the 1990s, the advancement of micro-nano processing technology enables the application of microneedles in the field of pharmacy. Metal microneedles (main materials: stainless steel, titanium alloy, nickel, palladium, etc.), silicon and silicon oxide microneedles, glass microneedles, polymer microneedles, and other types of microneedles were developed sequentially.In practice, microneedles can be classified into solid microneedles and hollow microneedles based on the presence/absence of a microchannel in the center.Based on different administration methods, solid microneedles can further be classified into soluble drug-loaded microneedles, insoluble drug-coated microneedles, and tissue-pretreating microneedles.In addition, they can also be classified into biological microneedles and artificial microneedles, and out-of-plane microneedles and in-plane microneedles.

Hollow microneedles are hollow needles made by micro-processing technology with a length of less than 1 mm. They are used to inject liquid vaccine formulations through the needles into the skin to achieve transdermal drug delivery.The initial application of hollow microneedles can be attributed to McAllister *et al.* in the 1980s, who developed silicon-based microneedles with a length of approximately 150 $\mu$m.

Two main application methods are available for solid microneedles in transdermal vaccination: "poke and patch" and "coat and poke". Solid microneedles were initially made of materials like titanium, silicon, stainless steel, glass, etc. However, the use of these microneedles has the risk that the microneedles may break and remain in the skin, and none of these materials can be degraded into non-toxic substances in the skin.Therefore, subsequent studies started to develop solid microneedles with degradable but water-insoluble polymer materials such as PLGA, PGA, and PLA, which are more suitable for transdermal drug delivery.The shape of solid microneedles is mainly pyramidal or conical, and the length of the microneedle is between 150 and 1000 microns.

Poke and patch is the earliest application approach of solid microneedles for transdermal drug delivery. Briefly saying, the microneedles are used to pretreat the skin and then removed, followed by application of the gel patch or liquid formulation containing the drug to the pretreated site. In this way, the drug in the formulation can diffuse and penetrate into the skin through the pinholes created by the microneedle during pretreatment, so as to achieve transdermal drug delivery. However, this approach makes it difficult to precisely control the dosage.

In contrast, the coat and poke approach involves coating the tip surface of the microneedle array with the coating solution containing the vaccine to obtain coated microneedles. When the coated microneedles are applied to the skin, the drug carried on the needle tip surface will be quickly dissolved and released into the skin. Compared with the poke and patch method, the coat and poke approach allows relatively precise control of dosage during transdermal drug delivery, simplicity of use, and a shortened duration of administration to a few minutes.However, its disadvantage lies in that the drug loading capacity at the tip is easily affected by the shape of the microneedles and the number of microneedles in the microneedle array.

The poke and release (self-dissolving microneedles) approach involves the utilization of a microneedle patch made of water-soluble materials, which contains vaccines only in the microneedle body.This design not only increases the drug loading capacity but also offers the flexibility to select between rapid-release and sustained-release drug delivery by employing different materials for the microneedles. The water-soluble polymer materials commonly used nowadays include: CMC, PVP, PVA/PVP mixed materials, silk protein, chondroitin sulfate, sodium hyaluronate, polysaccharides, and other materials.

Dissolving microneedles refer to a type of microneedles that combines the base of solid microneedles with a soluble needle-like structure at the tip. They are composed of a soluble or biodegradable matrix that can be dissolved after being inserted into the skin, therefore manifesting good biocompatibility. Typically, this type of microneedle array is composed of sugars, carbohydrates, or synthetic polymers, and can be used to carry substances such as insulin, low molecular weight heparin, ovalbumin, adenoviral vectors, vaccine antigens, photosensitizers, and prodrugs. Dissolving microneedles have the following advantages: 1) They are designed for one-time use, thus avoiding the transmission of infectious diseases; 2) drugs carried by the soluble part of the microneedles can be automatically absorbed by the skin, bypassing the passive absorption process; 3) the base portion of the microneedles also serves as solid microneedles to trigger a second stage of drug delivery, therefore allowing for a superior $t_{max}$ and prolonged drug effectiveness duration compared to traditional subcutaneous injection method; 4) the self-dissolving microneedles also facilitate sustained release of drugs, with the option to customize their degradation rates to meet specific treatment needs.

Hydrogel-forming microneedles consist of a swelling material and a drug reservoir layer. The swelling material and the drug reservoir layer in the hydrogel-forming microneedle array can facilitate drug dissolution by absorbing intercellular fluid through swollen micro-projections, which is different from dissolving microneedles. Therefore, hydrogel-forming microneedles are classified into a separate category. Hydrogel-forming microneedles can load drugs via two methods. One is to use the base of the microneedles to load drugs. After the needle penetrates the skin, the hydrogel will absorb the intercellular fluid and expand to form a gel channel, through which the drug at the base can pass and penetrate into the human body. The penetration speed is determined by the crosslinking density of the hydrogel. The other method is to prepare both the base and the needle body of the hydrogel-forming microneedles with a mixture of drugs and polymers. After the needle penetrates the skin, the needle body will start to swell due to infiltration of body fluid, and the drug will be released. The production of hydrogel-forming microneedles does not entail the use of materials associated with residual degradation concerns, thus allowing large-scale production. The composition for microneedle administration provided by the present invention is compatible with all kinds of microneedles.

**Bispecific Antibody**

Bispecific antibodies (BsAbs) are capable of concurrently targeting two epitopes, thereby offering distinct advantages over monoclonal antibody drugs in terms of therapeutic efficacy and safety. As a result, they are regarded as a promising new generation of immunotherapy drugs.

BsAbs do not exist in nature. Instead, they are artificially constructed antibodies. They can be classified into IgG-like and non-IgG-like forms, depending on whether they contain an Fc region or not. IgG-like BsAbs have a larger molecular weight and possess an Fc region, enabling them to engage in Fc-mediated effector functions. Moreover, they demonstrate an extended half-life compared to non-IgG-like forms, along with enhanced attributes of purity, solubility, and stability. The primary advantage of non-IgG-like BsAbs lies in their higher antigen-binding affinity. Despite a lower circulation kinetics compared to IgG-like BsAbs due to the absence of the Fc region, they have better tissue penetration ability and lower immunogenicity, and elicit reduced levels of non-specific activation within the innate immune system.

In the first application of BsAbs in tumor therapy, the BsAb was used to redirect T cells to target tumor cells and mediate T cell killing by facilitating their recognition of tumor-associated antigens (TAAs), thus playing the role of immune cell engagement. A representative BiTE (Bispecific T-cell engager) is the CD3×CD19 BsAb from Amgen, Blinatumomab, which was approved for marketing in 2014 for the indication of acute lymphoblastic leukemia (ALL). However, due to the lack of the Fc region, it has a short serum half-life, and therefore requires continuous intravenous infusion to achieve a therapeutic serum level. The launch of Blinatumomab has accelerated the development of a series of CD3-targeting BsAbs, wherein the other end of the BsAbs recognizes various TAAs, including CD19, CD20, BCMA, CD33, CD123 and CLEC12A associated with hematological malignancies, as well as CLDN18.2, CEA, EpCAM, HER2, PSMA, pCadherin, GPC3, GPA33 and more related to solid tumors.

The mRNA-encoded BsAbs provided by the present invention is CD3×EpCAM-mRNA (namely, ZSL303-mRNA-1), which is used to prepare the composition for microneedle administration ZSL303-mRNA-1-LNP. As verified by cell level assays, the composition has a remarkable expression effect. After being administered via microneedles into immunodeficient tumor-bearing mice with a reconstituted immune system, it manifested significant tumor inhibitory effects.

Although methods and materials similar or equivalent to those described herein can be used in the practice or testing of the present invention, suitable methods and materials are described below. All publications, patent applications, patents, and other references mentioned herein are incorporated by reference in their entirety. References cited herein are not admitted as prior art to the claimed invention. In addition, the materials, methods, and embodiments are illustrative only and not restrictive.

**Embodiments**

The present invention is described in further detail below in combination with the Attached Figures and embodiments. It is noted that the embodiments described below are intended to illustrate how the present invention is realized by way of example, or to illustrate the experimental process and effects of the present invention in a limited manner only, and thus to facilitate the understanding of the present invention, but without any limitation on the present invention, the scope of which is defined by the claims.

The experimental reagents, consumables and instruments used in the following embodiments are as follows: Experimental reagents and consumables: Anhydrous ethanol (Sigma-Aldrich, E7023-500 mL), cholesterol (Sigma-Aldrich, C8667-5G), DSPC (Avanti, SKU: 850365P-1g), ionizable lipid SM-102 (Shochem (Shanghai) Co., Ltd, Batch No: 20210401), DMG-PEG$_{2000}$ (Shochem (Shanghai) Co., Ltd, Batch No: 20200601), water (Invitrogen, REF: 750023), sodium acetate trihydrate (Sigma-Aldrich, 32318-500G-R), Tris hydrochloride (Roche, REF: 10812846001), tromethamine (Sigma-Aldrich, 76687-100G), glacial acetic acid (MACKLIN, A801295-500mL), S protein-mRNA (Hefei Afana Biotechnology Co., Ltd; Cat: S_mRNA_2P_01; Lot: 2011092101), ultrafiltration centrifugal tube 15 mL 10K (MilLipore, REF: UFC901096), Ribogreen kit (Invitrogen, REF: R11490), Fluc-mRNA (Hongene Biotech, FLUCmRNA (N1-Me-pseudo U); Lot: FLUCMPH1B; abbreviated as Fluc-mRNA; which has been marketed for sale. The purchased sequences contain UTR sequences, promoters, etc., which can be expressed *in vitro* at the cellular level and *in vivo* after delivery, HEK 293T cells (COBIOER), and ZSL303-mRNA-1 (Novoprotein, Lot: 20211129)

Experimental instruments: Microfluidic nanomedicine preparation system (Micro & Nano, model: INano L), high-speed frozen centrifuge (Thermo Scientific, model: Sorvall ST 16R), ultra-micro spectrophotometer (Thermo Scientific, model: Nanodrop One), laser particle size analyzer (Dandong Bettersize Instruments Ltd., model: BT-90+), precision balance (Sartorius, model: SQP Quintix124-1CN), pH meter (METTLER TOLEDO, model: S210), microneedle syringe head (Minank, Multi-needle CC-XW-04), 1-mL syringe (Kindly, 0.45 * 16 mm RWLB), flow cytometer (Agilent), and cell counting plate (WATSON)

**Embodiment 1 Preparation and testing of mRNA composition for microneedle administration**

**1. Preparation of a novel coronavirus mRNA vaccine for microneedle administration**

The novel coronavirus mRNA used in this embodiment is S protein-mRNA (sequence expressing S protein of the novel coronavirus) was purchased from Hefei Afana Biotechnology Co., Ltd (Cat: S_mRNA_2P_01; Lot: 2011092101), which has been marketed for sale. The purchased sequence contains UTR sequences, promoters, etc., and can be expressed *in vitro* at cellular level and *in vivo* after delivery.

The preparation method for a novel coronavirus mRNA vaccine for microneedle administration provided in this embodiment includes the steps of:

**1. Preparation of aqueous phase solution and lipid solution**

Prepare the aqueous phase solution and lipid mixture solution, wherein the 4X aqueous phase solution and the stock solution for lipid solution are prepared according to the method described in Table 1:

Table 1. Formulation for 4X aqueous phase solution and stock solution for lipid solution

| 4X aqueous phase solution | | Stock solution for lipid solution | | |
|---|---|---|---|---|
| Sample name | Sample weight | Sample name | Sample weight | Amount of anhydrous ethanol added |
| Tromethamine | 99.20 mg | SM-102 | 141.36 mg | 4 mL |
| Tris hydrochloride, Tris-HCl | 377.60 mg | Cholesterol | 59.26 mg | 4 mL |
| Glacial acetic acid | 13.76 mg | DSPC | 31.44 mg | 4 mL |
| Sodium acetate trihydrate | 64.00 mg | DMG-PEG$_{2000}$ | 14.97 mg | 4 mL |
| Water | 40 mL | NA | NA | NA |

The preparation method is as follows:

(1) Preparation of aqueous phase solution: Weigh accurately specified amounts of tromethamine, Tris hydrochloride, sodium acetate trihydrate and acetic acid, respectively, and dissolve in water to obtain the stock solution. Then dilute with water to obtain a 1X solution for use. Test the diluted aqueous phase solution with a pH meter, the pH of which was 7.4, and then adjust the diluted aqueous phase solution with glacial acetic acid to a pH of 5.5. Pipet an appropriate amount of S protein-mRNA (S protein-mRNA is a solution, the pH of its stock solution is around 6. Since a very small volume of S protein-mRNA stock solution is taken, the change to overall pH is very small and almost negligible), and dilute with 1X aqueous phase solution to obtain a 200 μg/mL aqueous mRNA solution. Here it is understood that the pH of the final aqueous phase solution is 5.5 or substantially at 5.5, and the slight change in pH due to dilution of nucleic acid was negligible.

(2) Preparation of lipid solution: Weigh accurately specified amounts of the four kinds of lipids, separately add 4 mL of anhydrous ethanol to dissolve, as shown in Table 1, and store for later use. Pipet the above solutions as per a ratio of 1:1:1:1, and mix well to obtain a lipid mixture solution with a final concentration of 15.44 mg/mL.

## 2. Preparation of S protein-mRNA-LNP for novel coronavirus mRNA vaccine by microfluidic nanomedicine preparation system

(1) Prepare the S protein-mRNA-LNP by microfluidic nanomedicine preparation system as per the following parameters: ratio of aqueous mRNA solution to organic phase lipid solution = 3:1, flow rate of 12 mL/min, preparation volume of 4 mL, initial waste solution of 0.3 mL and terminal waste solution of 0.05 mL.

(2) Immediately dilute the prepared LNP with 1X aqueous phase solution at pH 7.4 by 20-fold, concentrate by centrifugation with an ultrafiltration centrifugal tube (centrifugation conditions: 2500 g, 4°C, 10 min), and discard the solution in the lower part of the ultrafiltration centrifugal tube until the volume of the solution in the upper part of the ultrafiltration centrifugal tube is close to the volume of the initially prepared LNP. Then, add 1X aqueous phase solution at a volume of 10 times the volume of the initially prepared LNP, and continue to perform ultrafiltration centrifugation, so as to further reduce the concentration of ethanol. Take the sample obtained finally by ultrafiltration centrifugation as the final sample of S protein-mRNA-LNP, the volume of which should be close to the volume of the initially prepared LNP, and store at 4°C for later use.

## 2. Preparation of mRNA-encoded bispecific antibody drug composition for microneedle administration

The preparation method for a mRNA-encoded bispecific antibody drug composition ZSL303-mRNA-1-LNP for microneedle administration provided in this embodiment includes the steps of:

## 1. Synthesis of ZSL303-mRNA-1

The synthesis of bispecific antibody ZSL303-mRNA-1-LNP (wherein the nucleic acid CD3 × EpCAM-mRNA expressing the bispecific antibody has the sequence as shown in SEQ ID NO: 1) used in this embodiment is commissioned to novoprotein, and the specific method for synthesis is as follows: Linearize the plasmid template by using BsaI restriction endonuclease. Recover and purify the plasmid linearization product after reacting for 3 hours at 37°C. Prepare the mRNA IVT reaction system, mix the purified plasmid linearization product with mRNA IVT reaction system, and react for 3 hours at 37°C. After the reaction, add DNaseI, and react at 37°C for 20 minutes. After the reaction, purify the transcription product to remove the excess enzyme and raw material of IVT reaction. Cap the purified transcription product enzymatically, and react at 37°C for 1 hour. After the reaction, purify the capped product by LiCl method, and perform routine quantitative testing and purity testing for the purified product.

## 2. Preparation of aqueous phase solution and lipid solution

Prepare the aqueous phase solution and lipid mixture solution, wherein the 4X aqueous phase solution and stock solution for lipid solution are prepared in the manner described in Table 1. The preparation method is as follows:

(1) Preparation of aqueous phase solution: Weigh accurately specified amounts of tromethamine, Tris hydrochloride, sodium acetate trihydrate and acetic acid, respectively, and dissolve in water to obtain the stock solution. Then dilute with water to obtain a 1X solution for use. Pipet an appropriate amount of ZSL303-mRNA-1, and dilute with 1X aqueous phase solution to obtain 3.8 mL of 0.15 mg/mL aqueous mRNA solution. Adjust the pH of the aqueous phase solution to 5.5 with glacial acetic acid.

(2) Preparation of lipid solution: Weigh accurately specified amounts of the four kinds of lipids, separately add 4 mL of anhydrous ethanol to dissolve, as shown in Table 1, and store for later use. Pipet the above solutions as per a ratio of 1:1:1:1, and mix well to obtain a final lipid mixture solution.

**3. Preparation of ZSL303-mRNA-1-LNP by microfluidic nanomedicine preparation system**

(1) Prepare the ZSL303- mRN A -1- LNP by microfluidic nanomedicine preparation system as per the following parameters: ratio of aqueous mRNA solution and organic phase lipid solution = 3:1, flow rate of 12 mL/min, preparation volume of 5 mL, initial waste solution of 0.3 mL and terminal waste solution of 0.05 mL.

(2) Immediately dilute the prepared LNP with 1X aqueous phase solution by 20-fold, concentrate by centrifugation with an ultrafiltration centrifugal tube (centrifugation conditions: 2500 g, 4°C, 10 min), and discard the solution in the lower part of the ultrafiltration centrifugal tube until the volume of the solution in the upper part of the ultrafiltration centrifugal tube is close to the volume of the initially prepared LNP. Then, add 1X aqueous phase solution at a volume of 10 times the volume of the initially prepared LNP, and continue to perform ultrafiltration centrifugation, so as to further reduce the concentration of ethanol. Take the sample obtained finally by ultrafiltration centrifugation as the final sample of ZSL303-mRNA-1-LNP, the volume of which should be close to the volume of the initially prepared LNP, and store at 4°C for later use.

**3. Testing and analysis**

The S protein-mRNA-LNP and ZSL303-mRNA-1-LNP samples were prepared according to step 1 and step 2, and three preparation experiments were performed at weekly intervals to test the particle size, PDI (polydispersity index), encapsulation efficiency and drug loading capacity, respectively.

(1) Testing of particle size and PDI.

The obtained samples of S protein-mRNA-LNP and ZSL303-mRNA-1-LNP were tested for particle size and PDI by a particle size analyzer, wherein the particle size was tested as per the following method: Place 1 mL of sample into the cuvette, and disperse the corresponding sample in the cuvette. Radiate the sample by a laser at a wavelength of 671 nm. Detect the scattered light intensity fluctuations over time caused by the Brownian motion of the particles at an angle of 90° by an APD photodetector, and then obtain the autocorrelation curve of the sample by the autocorrelation operation through the correlator. The diffusion coefficient of the particles can be obtained by combining the mathematical methods, and further the results of the particle size distribution of the sample, i.e., the hydrodynamic diameter $D_H$ and its distribution, are obtained by using the Stockes-Einstein equation. The particle distribution information is systematically calculated to get PDI; the average particle size of S protein-mRNA-LNP was 213.5 nm, and PDI was 0.15; the average particle size of ZSL303-mRNA-1-LNP was 125.5 nm, and PDI was 0.265.

(2) The encapsulation efficiency is measured by Ribogreenkit and the drug loading capacity is calculated.

The encapsulation efficiency is tested as follows:

a. Prepare an appropriate amount of 1X TE buffer by diluting 20X TE buffer with sterile enzyme-free water. For example, to 10 mL of 20X TE buffer, add 190 mL of sterile enzyme-free water, and mixethoroughly;

b. Prepare Triton buffer. For example, to 100 mL of TE buffer, add 2 mL of Triton X-100, and stir for 15 min to mix well;

c. Take a 96-well black plate, add 15 $\mu$L of sample to the first row, and add one well with PBS, respectively, and add 1X TE buffer to a total volume of 250 $\mu$L;

d. Perform sample addition to the 96-well black plate as shown in Figure 2, wherein add 50 $\mu$L of 1X TE buffer plus 50 $\mu$L of Row A sample diluent to rows B and C; and add 50 $\mu$L of Triton buffer plus 50 $\mu$L of Row A sample diluent to rows D and E:

e. Prepare the standard curve solutions according to Table 2 and add to the 96-well black plate respectively, wherein RNA standard is diluted to 20 $\mu$g/mL using S protein-mRNA stock solution, as the RNA stock solution for standard curve.

Table 2. Preparation of standard curve solution

| Final RNA $\mu$g/mL | RNA Stock Required $\mu$L | TE Buffer Required $\mu$L | Triton Buffer Required $\mu$L | Total Volume per Well $\mu$L |
|---|---|---|---|---|
| 2.5 | 25 | 25 | 50 | 100 |
| 1 | 10 | 40 | 50 | 100 |

(continued)

| Final RNA μg/mL | RNA Stock Required μL | TE Buffer Required μL | Triton Buffer Required μL | Total Volume per Well μL |
|---|---|---|---|---|
| 0.5 | 5 | 45 | 50 | 100 |
| 0.25 | 2.5 | 47.5 | 50 | 100 |
| 0.1 | 1 | 49 | 50 | 100 |

f. Perform fluorescence detection on the microplate reader after chromogenic solution is added to all sample wells. The results of encapsulation efficiency are calculated according to the standard curve.

The encapsulation efficiency data are then calculated according to the calculation formula for encapsulation efficiency (ratio of encapsulated drug to total drug input).

The drug loading capacity, which is the amount of mRNA drug per unit volume of the lipid nanoparticles, is calculated from the amount of unencapsulated drug (S protein-mRNA) and the total amount of drug (S protein-mRNA) as follows:

$$\text{Drug loading capacity} = (\text{total amount of drug - amount of free drug (not effectively}$$
$$\text{encapsulated}))/\text{volume}$$

Where, the volume is the initial volume of lipid nanoparticles prepared by microfluidic nanomedicine preparation system. Although the initially prepared lipid nanoparticles need to be diluted by 20-fold with 1X aqueous phase solution and concentrated by centrifugation in an ultrafiltration centrifugal tube to reduce the concentration of ethanol, the final sample obtained after centrifugation and concentration still maintains the initial volume of lipid nanoparticles, and thus the initial volume of lipid nanoparticles is used for the calculation of drug loading capacity. If the drug loading capacity is measured by using diluted sample, appropriate dilution factor should be multiplied.

The average encapsulation efficiency of the S protein-mRNA-LNP sample prepared in this embodiment was 83.5% with a drug loading capacity of 157.75 μg/mL, as tested and calculated.

The average encapsulation efficiency of the ZSL303-mRNA-1-LNP sample prepared in this embodiment was 95.5% with a drug loading capacity of 102.9 μg/mL.

The results of three tests on S protein-mRNA-LNP and ZSL303-mRNA-1-LNP were relatively stable, with consistent particle size, and stable encapsulation efficiency and drug loading capacity.

**Embodiment 2 Effect of buffer pH on drug loading capacity for preparation of mRNA-LNP**

**1. Effect of buffer pH on drug loading capacity for preparation of mRNA-LNP in multiple buffer formulations**

**1. Effect of buffer pH on drug loading capacity for preparation of Fluc-mRNA-LNP when using Tris buffer system**

The aqueous phase solution and the lipid mixture solution were prepared according to the method provided in Embodiment 1, wherein the aqueous phase solution was prepared using the Tris buffer system: Weigh accurately specified amounts of tromethamine, Tris hydrochloride, sodium acetate trihydrate and acetic acid, respectively, and dissolve in water for later use. Then dilute with water to obtain a 1X solution for use. Test the diluted aqueous phase solution by pH meter, and the pH was 7.4. Adjust the pH with glacial acetic acid to obtain aqueous phase solutions at pH of 4.0, 4.5, 5.0, 5.5, 6.0, 6.5 and 7.0, respectively.

To facilitate observation and detection, the S protein-mRNA was replaced by Fluc-mRNA (Hongene Biotech, FLUCmRNA (N1-Me-pseudo U), lot: FLUCMPH1B, abbreviated as Fluc-mRNA, which has been marketed for sale. The purchased sequences contain UTR sequences, promoters, etc., and can be expressed *in vitro* at cellular level and *in vivo* after delivery, so that the difference in cell expressions of Fluc-mRNA-LNP with different drug loading capacity can be more intuitively characterized by fluorescence.

The Fluc-mRNA stock solution was diluted with aqueous phase solutions at different pH to obtain solutions with a concentration of 200 μg/mL, and the particle size, PDI, encapsulation efficiency and drug loading capacity of the obtained Fluc-mRNA-LNP were analyzed. The particle size, PDI, encapsulation efficiency and drug loading capacity were tested according to the methods provided in Embodiment 1. The results are summarized in Table 3.

Table 3. Effect of Tris buffer solutions at different pH on the preparation of Fluc-mRNA-LNP

| pH | Particle size (nm) | PDI | Zeta (mV) | Encapsulation efficiency (%) | Drug loading capacity ($\mu$g/mL) |
|---|---|---|---|---|---|
| 4.0 | 89.79 | 0.1030 | -5.640 | 51.25 | 85.06 |
| 4.5 | 95.51 | 0.1416 | -2.864 | 80.82 | 118.59 |
| 5.0 | 102.7 | 0.1618 | -2.464 | 78.41 | 99.17 |
| 5.5 | 77.24 | 0.1093 | -3.596 | 80.01 | 134.72 |
| 6.0 | 86.85 | 0.0724 | -2.625 | 66.73 | 84.50 |
| 6.5 | 79.95 | 0.1073 | -3.693 | 68.54 | 98.52 |
| 6.8 | 84.42 | 0.1352 | -3.978 | 36.25 | 47.62 |
| 7.0 | 105.70 | 0.1664 | -4.886 | 14.51 | 15.83 |
| 7.4 | 104 | 0.2559 | -4.134 | 7.98 | 12.15 |

After repeated experiments for several times, the results of Fluc-mRNA prepared in Tris buffer solutions at different pH were basically consistent, with only slight variations in particle size, which might be related to the testing apparatus, and the encapsulation efficiency and drug loading capacity were basically consistent.

As can be seen from Table 3, for Fluc-mRNA-LNP prepared by using Tris buffers at different pH, the particle size results were quite different, and the drug loading capacities were completely different; the drug loading capacity was particularly low at pH 7.0 or 7.4 due to the use of Fluc-mRNA in this embodiment; the encapsulation efficiency did not differ much from pH 4.0 to pH 5.5, but the drug loading capacity had an obvious increasing trend. The highest total mRNA loaded (134.72 $\mu$g/mL) was measured at pH 5.5, meaning that the unit volume of LNP contained more mRNA, and the stability was good. It can be seen that maintaining an appropriate pH of the aqueous phase solution can also significantly increase the loading capacity of Fluc-mRNA. If the pH is adjusted to be in the acidic range such as 4.5-6.8, the content of Fluc-mRNA loaded in liposomes increases significantly, and reaches the optimum at a specific pH condition, i.e. pH 5.5. The possible reason for this may be as follows: During the preparation, the ionizable lipids can be positively charged in an acidic environment, and more nucleic acid molecules (such as mRNA, etc., negatively charged itself) can be adsorbed under the same conditions. This optimum situation appears when the pH of the aqueous phase solution is 5.5. Compared to buffer solutions at pH 7.4 or pH 4, etc., nucleic acid molecules can be loaded more effectively at pH 4.5 to 6.5, preferably pH 4.5 to 5.5, more preferably pH 5.0 to 5.5, most preferably pH 5.5, to obtain a relatively stable and usable formulation, and thus the drug loading capacity is higher.

In addition, it was also found from this embodiment that the drug loading capacity at pH 7.0 or 7.4 was particularly low when Fluc-mRNA was used compared to other mRNAs.

**2. Effect of buffer pH on drug loading capacity for preparation of mRNA-LNP when using sodium acetate buffer system**

The aqueous phase solution and the lipid mixture solution were prepared according to the method provided in Embodiment 1, wherein the aqueous phase solution was prepared using a sodium acetate buffer system, and the specific preparation method was as follows: Weigh accurately a suitable amount of sodium acetate trihydrate, and dissolve in water. Adjust the pH with glacial acetic acid to obtain aqueous phase solutions at pH of 4.0, 4.5, 5.0, 5.5, 6.0, 6.5 and 7.0, respectively. The Fluc-mRNA stock solution was diluted with aqueous phase solutions at different pH to obtain solutions with a concentration of 200 $\mu$g/mL, and the particle size, PDI, encapsulation efficiency and drug loading capacity of the obtained Fluc-mRNA-LNP were analyzed. The particle size, PDI, encapsulation efficiency and drug loading capacity were tested according to the methods provided in Embodiment 1. The results are summarized in Table 4.

Table 4. Effect of sodium acetate buffer solutions at different pH on the preparation of Fluc-mRNA-LNP

| pH | Particle size (nm) | PDI | Zeta (mV) | Encapsulation efficiency (%) | Drug loading capacity ($\mu$g/mL) |
|---|---|---|---|---|---|
| 4.0 | 74.25 | 0.0855 | -8.183 | 92.56 | 98.70 |
| 4.5 | 84.62 | 0.1416 | -5.106 | 84.86 | 109.03 |
| 5.0 | 87.86 | 0.0906 | -4.754 | 85.79 | 114.10 |

(continued)

| pH | Particle size (nm) | PDI | Zeta (mV) | Encapsulation efficiency (%) | Drug loading capacity ($\mu$g/mL) |
|---|---|---|---|---|---|
| 5.5 | 100.3 | 0.1537 | -7.833 | 90.21 | 117.66 |
| 6.0 | 101.1 | 0.0963 | -2.748 | 41.78 | 54.11 |
| 6.5 | 98.53 | 0.1907 | -3.563 | 1.83 | 2.32 |
| 7.0 | 93.29 | 0.1904 | -4.548 | 1.32 | 1.70 |

After repeated experiments for several times, the results of Fluc-mRNA prepared in sodium acetate buffer solutions at different pH were basically consistent, with only slight variations in particle size, which might be related to the testing apparatus, and the encapsulation efficiency and drug loading capacity were basically consistent.

As can be seen from Table 4, although the buffer systems were different, the pH of buffer solutions had a consistent effect on the change trend of drug loading capacity of the prepared Fluc-mRNA-LNP. For Fluc-mRNA-LNP prepared by using sodium acetate buffer solutions at different pH, the particle size results were of certain differences, and the drug loading capacities were completely different; the encapsulation efficiency did not differ much from pH 4.5 to 5.5, but the drug loading capacity had an obvious increasing trend, preferably from pH 5.0 to 5.5, and most preferably pH 5.5, at which the highest total mRNA loaded (117.66 $\mu$g/mL) was measured, meaning that the unit volume of LNP contained more mRNA, and the stability was good. It can be seen that maintaining an appropriate pH of the aqueous phase solution can significantly increase the loading capacity of Fluc-mRNA indeed, and this trend does not change with changes in the specific buffer composition.

## 3. Effect of buffer pH on drug loading capacity for preparation of mRNA-LNP when using PBS system

The aqueous phase solution and the lipid mixture solution were prepared according to the method provided in Embodiment 1, wherein the aqueous phase solution was prepared using PBS system. The specific preparation method is as follows: Adjust the pH of the purchased PBS (Biosharp, Cat: BL302A) with glacial acetic acid to obtain aqueous phase solutions at pH of 4.0, 5.5, 6.5 and 7.0, respectively. The Fluc-mRNA stock solution was diluted with PBS aqueous phase solutions at different pH to obtain solutions with a concentration of 200 $\mu$g/mL, and the particle size, PDI, encapsulation efficiency and drug loading capacity of the obtained Fluc-mRNA-LNP were analyzed. The particle size, PDI, encapsulation efficiency and drug loading capacity were tested according to the methods provided in Embodiment 1. The results are summarized in Table 5.

Table 5. Effect of PBS at different pH on the preparation of Fluc-mRNA-LNP

| pH | Particle size (nm) | PDI | Zeta (mV) | Encapsulation efficiency (%) | Drug loading capacity ($\mu$g/mL) |
|---|---|---|---|---|---|
| 4.0 | 100.2 | 0.0977 | -5.103 | 83.81 | 118.18 |
| 5.5 | 93.81 | 0.124 | -4.935 | 83.80 | 130.41 |
| 6.5 | 85.1 | 0.1998 | -5.011 | 28.25 | 38.27 |
| 7.0 | 76.75 | 0.1726 | -4.748 | 8.68 | 8.69 |

As can be seen from Table 5, although the buffer systems were different, the pH of buffer solutions had a consistent effect on the change trend of drug loading capacity of the prepared Fluc-mRNA-LNP. For Fluc-mRNA-LNP prepared by using PBS at different pH, the particle size results were of certain differences, and the drug loading capacities were completely different; the encapsulation efficiency did not differ much from pH 4.0 to 5.5, but the drug loading capacity had an obvious increasing trend, most preferably pH 5.5, at which the highest total mRNA loaded (130.41 $\mu$g/mL) was measured, meaning that the unit volume of LNP contained more mRNA, and the stability was good. It can be seen that maintaining an appropriate pH of the aqueous phase solution can significantly increase the loading capacity of Fluc-mRNA indeed, and this trend does not change with changes in the specific buffer composition.

**2. Effect of buffer pH on drug loading capacity for preparation of mRNA-LNP in multiple lipid formulations**

**1. Effect of buffer pH on drug loading capacity for preparation of mRNA-LNP when using lipids of JK-102-CA, DMG-mPEG2000, Chol and DSPC**

The aqueous phase solution and the lipid mixture solution were prepared according to the method provided in Embodiment 1, wherein the aqueous phase solution was prepared using the Tris buffer system: Weigh accurately specified amounts of tromethamine, Tris hydrochloride, sodium acetate trihydrate and acetic acid, respectively, and dissolve in water for later use. Then dilute with water to obtain a 1X solution for use. Test the diluted aqueous phase solution by pH meter, and the pH was 7.4. Adjust the pH with glacial acetic acid to obtain aqueous phase solutions at pH of 4.0, 5.5 and 6.0, respectively. As shown in Table 6, the ionizable lipid JK-102-CA, the PEG lipid DMG-mPEG2000 and other two lipid components Chol and DSPC were selected as the organic phases for the preparation of Fluc-mRNA-LNP. The Fluc-mRNA stock solution was diluted with aqueous phase solutions at different pH to obtain solutions with a concentration of 200 μg/mL, and the particle size, PDI, encapsulation efficiency and drug loading capacity of the obtained Fluc-mRNA-LNP were analyzed. The particle size, PDI, encapsulation efficiency and drug loading capacity were tested according to the methods provided in Embodiment 1. The results are summarized in Table 6.

Table 6. Effect of buffer solutions at different pH on the preparation of Fluc-mRNA-LNP by specific lipid formulation

| Lipid composition | pH | Particle size (nm) | PDI | Zeta (mV) | Encapsulation efficiency (%) | Drug loading capacity (μg/mL) |
|---|---|---|---|---|---|---|
| Flue mRNA LNP(JK-102-CA, DMG-mPEG2000, Chol, DSPC) | 4.0 | 101.8 | 0.4219 | -0.6913 | 90.35 | 74.90 |
| | 5.5 | 99.34 | 0.1622 | -1.579 | 91.38 | 106.77 |
| | 6.0 | 95.02 | 0.2343 | -0.6216 | 85.47 | 87.65 |

As can be seen from Table 6, when the lipid formulation was changed, the effect of pH of buffer solutions on the change trend of drug loading capacity of the prepared Fluc-mRNA-LNP still existed, and under the condition of buffer solutions at pH of 5.5 to 6.0, the drug loading capacity of Fluc-mRNA-LNP increased significantly. The most preferred pH was 5.5, at which the highest total mRNA loaded was measured (106.77 μg/mL), meaning that the unit volume of LNP contained more mRNA, and the stability was also very good. It can be seen that maintaining an appropriate pH of the aqueous phase solution can significantly increase the loading capacity of Fluc-mRNA indeed, and this trend does not change with changes in the specific lipid formulation composition.

**2. Effect of buffer pH on the drug loading capacity for preparation of mRNA-LNP when using lipids of SM102, M-DTDAM-2000, Chol and DSPC**

The aqueous phase solution and the lipid mixture solution were prepared according to the method provided in Embodiment 1, wherein the aqueous phase solution was prepared using the Tris buffer system: Weigh accurately specified amounts of tromethamine, Tris hydrochloride, sodium acetate trihydrate and acetic acid, respectively, and dissolve in water for later use. Then dilute with water to obtain a 1X solution for use. Test the diluted aqueous phase solution by pH meter, and the pH was 7.4. Adjust the pH with glacial acetic acid to obtain aqueous phase solutions at pH of 4.0, 5.5 and 6.0, respectively. As shown in Table 7, the ionizable lipid SM102, the PEG lipid DTDAM-2000 and other two lipid components Chol and DSPC were selected as the organic phases for the preparation of Fluc-mRNA-LNP. The Fluc-mRNA stock solution was diluted with aqueous phase solutions at different pH to obtain solutions with a concentration of 200 μg/mL, and the particle size, PDI, encapsulation efficiency and drug loading capacity of the obtained Fluc-mRNA-LNP were analyzed. The particle size, PDI, encapsulation efficiency and drug loading capacity were tested according to the methods provided in Embodiment 1. The results are summarized in Table 7.

Table 7. Effect of buffer solutions at different pH on the preparation of Fluc-mRNA-LNP by specific lipid formulation

| Lipid composition | pH | Particle size (nm) | PDI | Zeta (mV) | Encapsulation efficiency (%) | Drug loading capacity (μg/mL) |
|---|---|---|---|---|---|---|
| Flue mRNA LNP(SM102, M-DTDAM-2000, Chol, DSPC) | 4.5 | 73.02 | 0.0572 | -5.924 | 90.75 | 99.20 |
| | 5.5 | 74.03 | 0.1011 | -3.549 | 89.07 | 108.13 |
| | 6.0 | 75.71 | 0.0726 | -4.045 | 75.80 | 78.17 |

As can be seen from Table 7, when the lipid formulation was changed, the effect of pH of buffer solutions on the change trend of drug loading capacity of the prepared Fluc-mRNA-LNP still existed, and under the condition of buffer solutions at pH of 4.5 and 5.5, the drug loading capacities of Fluc-mRNA-LNP were higher, meaning that the unit volume of LNP contained more mRNA, and the stability was good. It can be seen that maintaining an appropriate pH of the aqueous phase solution can significantly increase the loading capacity of Fluc-mRNA indeed, and this trend does not change with changes in the specific lipid formulation composition.

**3. Effect of buffer pH on the drug loading capacity for preparation of mRNA-LNP when using lipids of JK-102-CA, M-DTDAM-2000, Chol and DSPC**

The aqueous phase solution and the lipid mixture solution were prepared according to the method provided in Embodiment 1, wherein the aqueous phase solution was prepared using the Tris buffer system: Weigh accurately specified amounts of tromethamine, Tris hydrochloride, sodium acetate trihydrate and acetic acid, respectively, and dissolve in water for later use. Then dilute with water to obtain a 1X solution for use. Test the diluted aqueous phase solution by pH meter, and the pH was 7.4. Adjust the pH with glacial acetic acid to obtain aqueous phase solutions at pH of 4.0, 5.5 and 6.0, respectively. As shown in Table 7, the ionizable lipid JK-102-CA, the PEG lipid M-DTDAM-2000 and other two lipid components Chol and DSPC were selected as the organic phases for the preparation of Fluc-mRNA-LNP. The Fluc-mRNA stock solution was diluted with aqueous phase solutions at different pH to obtain solutions with a concentration of 200 μg/mL, and the particle size, PDI, encapsulation efficiency and drug loading capacity of the obtained Fluc-mRNA-LNP were analyzed. The particle size, PDI, encapsulation efficiency and drug loading capacity were tested according to the methods provided in Embodiment 1. The results are summarized in Table 7.

Table 7. Effect of buffer solutions at different pH on the preparation of Fluc-mRNA-LNP by specific lipid formulation

| Lipid composition | pH | Particle size (nm) | PDI | Zeta (mV) | Encapsulation efficiency (%) | Drug loading capacity (μg/mL) |
|---|---|---|---|---|---|---|
| Flue mRNA LNP(JK-102-CA, M-DTDAM-2000, Chol, DSPC) | 4.0 | 101.3 | 0.3915 | -0.3359 | 90.41 | 78.56 |
| | 5.5 | 92.94 | 0.2445 | -0.259 | 91.02 | 97.00 |
| | 6.0 | 91.43 | 0.2472 | -0.6001 | 91.19 | 100.46 |

As can be seen from Table 7, when the lipid formulation was changed, the effect of pH of buffer solutions on the change trend of drug loading capacity of the prepared Fluc-mRNA-LNP still existed, and under the condition of buffer solutions at pH of 5.5 to 6.0, the drug loading capacity of Fluc-mRNA-LNP increased significantly. The drug loading capacities at pH 5.5 and pH 6.0 were very close to each other, at which the measured total mRNA loaded were higher, meaning that the unit volume of LNP contained more mRNA, and the stability was good. It can be seen that maintaining an appropriate pH of the aqueous phase solution can significantly increase the loading capacity of Fluc-mRNA indeed, and this trend does not change with changes in the specific lipid formulation composition.

**3. Effect of buffer pH on the drug loading capacity for preparation of mRNA-LNP using multiple mRNAs**

**1. Effect of buffer pH on the drug loading capacity for preparation of mRNA-LNP when using S protein-mRNA**

The aqueous phase solution and the lipid mixture solution were prepared according to the method provided in Embodiment 1, wherein the aqueous phase solution was prepared as follows: Weigh accurately specified amounts of tromethamine, Tris hydrochloride, sodium acetate trihydrate and acetic acid, respectively, and dissolve in water for later use. Then dilute with water to obtain a 1X solution for use. Test the diluted aqueous phase solution by pH meter, and the pH was 7.4. Adjust the pH with glacial acetic acid to obtain aqueous phase solutions at pH of 4.0 and 5.5, respectively. The S protein-mRNA stock solution was diluted with aqueous phase solutions at different pH to obtain solutions with a concentration of 200 $\mu$g/mL. The detailed preparation method is shown in Table 8. The blank LNP (without S protein-mRNA) was used as the control, and the particle size, PDI, encapsulation efficiency and drug loading capacity of the prepared S protein-mRNA-LNP were analyzed. The particle size, PDI, encapsulation efficiency and drug loading capacity were tested according to the methods provided in Embodiment 1. The results are summarized in Table 8.

Table 8. Effect of buffer solutions at different pH on the preparation of S protein-mRNA-LNP

| Aqueous phase solution | pH 4.0 Aqua phase | | pH 5.5 Aqua phase | | pH 7.4 Aqua phase | |
|---|---|---|---|---|---|---|
| | Blank@LNP | 200 $\mu$g/mL S protein mRNA-LNP | Blank@LNP | 200 $\mu$g/mL S protein mRNA-LNP | Blank@LNP | 200 $\mu$g/mL S protein mRNA-LNP |
| Lipid content | 15.44 mg/mL | | | | | |
| Results | | | | | | |
| Particle size (nm) | 114.9 | 268.4 | 151.7 | 213.5 | NA | 195.4 |
| PDI | 0.058 | 0.195 | 0.069 | 0.15 | NA | 0.166 |
| Encapsulatio n efficiency (%) | NA | 87.80 % | NA | 88.50 % | NA | 30.3 % |
| Drug loading capacity ($\mu$g/mL) | NA | 133.83 | NA | 157.75 | NA | 29.39 |

After repeated experiments for several times, the results of S protein-mRNA-LNP prepared in buffer solutions at different pH were basically consistent, with only slight variations in particle size, which might be related to the testing apparatus, and the encapsulation efficiency and drug loading capacity were basically consistent.

As can be seen from Table 8, for S protein-mRNA-LNP prepared by using buffer solutions at different pH, the particle size results were quite different, and the drug loading capacities were completely different; the encapsulation efficiency at pH 4.0 and pH 5.5 did not differ much, but the measured total mRNA loaded at pH 5.5 was particularly high (157.75 $\mu$g/mL), meaning that the unit volume of LNP contained more mRNA, and the stability was good. It can be seen that maintaining an appropriate pH of the aqueous phase solution can also significantly increase the drug loading capacity of the novel coronavirus mRNA vaccine. If the pH is adjusted to the acidic range such as 4-6, the content of S protein-mRNA loaded in liposomes can be significantly increased, and reaches the optimum at a specific pH of 5.5. The possible reason for this may be as follows: During the preparation, the ionizable lipids can be positively charged in an acidic environment, and more nucleic acid molecules (such as mRNA, etc., negatively charged itself) can be adsorbed under the same conditions. This optimum situation appears when the pH of the aqueous phase solution is 5.5, at which the nucleic acid molecules can be loaded more effectively compared to buffer solutions at pH 7.4 or pH 4, etc., to obtain a relatively stable and usable formulation, and thus the drug loading capacity is higher. Thus, the novel coronavirus mRNA vaccine can be used for microneedle administration, meeting the dose of no more than 50 $\mu$L for microneedle administration, with less dose, less toxic side effects and better immunization effect, and is especially suitable for microneedle administration.

## 2. Effect of buffer pH on the drug loading capacity for preparation of mRNA-LNP when using ZSL303-mRNA

The aqueous phase solution and the lipid mixture solution were prepared according to the method provided in Embodiment 1, wherein the aqueous phase solution was prepared as follows: Weigh accurately specified amounts of tromethamine, Tris hydrochloride, sodium acetate trihydrate and acetic acid, respectively, and dissolve in water for later use. Then dilute with water to obtain a 1X solution for use. Test the diluted aqueous phase solution by pH meter, and the pH was 7.4. Adjust the pH with glacial acetic acid to obtain aqueous phase solutions at pH of 4.0 and 5.5, respectively.

A volume of 5 $\mu$L, of ZSL303-mRNA-1 stock solution was tested in an ultra-micro spectrophotometer Nanodrop to determine the concentration, and the result was 2186 $\mu$g/mL, which was basically the same as the labeled value at the time of purchase. Subsequent dilutions of mRNA for preparation were performed as per this concentration. Solutions with a concentration of 200 $\mu$g/mL were obtained by diluting with aqueous phase solutions at different pH, respectively. The detailed preparation method is shown in Table 9.

Table 9. Preparation method for buffer solutions at different pH

| pH 4 | pH 5.5 | pH 7.4 |
|---|---|---|
| 0.07 mL of mRNA stock solution + 0.7 mL of aqueous buffer at pH 4 | 0.07 mL of mRNA stock solution + 0.7 mL of aqueous buffer at pH 5.5 | 0.07 mL of mRNA stock solution + 0.7 mL of aqueous buffer at pH 7.4 |

Experimental results and analysis: The results of the particle size, PDI, encapsulation efficiency and drug loading capacity of the prepared ZSL303-mRNA-1-LNP are summarized in Table 7. The particle size, PDI, encapsulation efficiency, and drug loading capacity were tested according to the methods provided in Embodiment 1. The test results are shown in Table 10.

Table 10. Effect of buffer solutions at different pH on the preparation of ZSL303-mRNA-1-LNP

| Buffer pH | pH 4.0 buffer for mRNA-LNP | pH 5.5 buffer for mRNA-LNP | pH 7.4 buffer for mRNA-LNP |
|---|---|---|---|
| Particle size (nm) | 97.27 | 125.5 | 133.4 |
| PDI | 0.214 | 0.265 | 0.349 |
| Encapsulation efficiency (%) | 95.8 | 95.5 | 32.1 |
| Drug loading capacity ($\mu$g/mL) | 85.5 | 102.9 | 36.1 |

As can be seen from Table 10, for ZSL303-mRNA-1-LNP prepared by using mRNA aqueous phase solutions at different pH, the particle size results were quite different, and the encapsulation efficiency was completely different. The encapsulation efficiency at pH 4.0 and pH 5.5 did not differ much, but the measured total mRNA loaded at pH 5.5 was particularly high (102.9 $\mu$g/mL), at which the stability was better, the dosage was lower, the toxic side effects were less, the therapeutic effect was better, and it was more suitable for microneedle administration.

It can be seen that maintaining an appropriate pH of the aqueous phase solution can also significantly increase the loading capacity of mRNA encoding bispecific antibody for microneedle intradermal administration. If the pH is adjusted to an acidic range such as 4-6, the content of bispecific antibody mRNA loaded in liposomes can be significantly increased, and reaches the optimum at a specific pH of 5.5. The possible reason for this may be as follows: During the preparation, the ionizable lipids can be positively charged in an acidic environment, and more nucleic acid molecules (such as mRNA, etc., negatively charged itself) can be adsorbed under the same conditions. This optimum situation appears when the pH of the aqueous solution is 5.5, at which nucleic acid molecules can be encapsulated more tightly compared to buffer solutions at pH 7.4 or pH 4, etc., to obtain a relatively stable and usable formulation, and thus the drug loading capacity is higher.

### 3. Effect of buffer pH on the drug loading capacity for preparation of mRNA-LNP when using EGFP-mRNA

The aqueous phase solution and the lipid mixture solution were prepared according to the method provided in Embodiment 1, wherein the aqueous phase solution was prepared using the Tris buffer system: Weigh accurately specified amounts of tromethamine, Tris hydrochloride, sodium acetate trihydrate and acetic acid, respectively, and dissolve in water for later use. Then dilute with water to obtain a 1X solution for use. Test the diluted aqueous phase solution by pH meter, and the pH was 7.4. Adjust the pH with glacial acetic acid to obtain aqueous phase solutions at pH of 4.0, 4.5, 5.5, 6.5 and 7.0, respectively. The mRNA was replaced by EGFP-mRNA (purchased from Afana Biotechnology Co., Ltd, Type: mRNA-22-220605, which has been marketed for sale. The purchased sequences contain UTR sequences, promoters, etc., and can be expressed *in vitro* at cellular level and *in vivo* after delivery), so that the difference in cell expressions of EGFP-mRNA-LNP with different loading capacity can be more intuitively characterized by fluorescence. The EGFP-mRNA stock solution was diluted with aqueous phase solutions at different pH to obtain solutions with a concentration of 200 $\mu$g/mL, and the particle size, PDI, encapsulation efficiency and drug loading capacity of the obtained EGFP-mRNA-LNP were analyzed. The particle size, PDI, encapsulation efficiency and drug loading capacity were tested according to the methods provided in Embodiment 1. The results are summarized in Table 11.

Table 11. Effect of buffer solutions at different pH on the preparation of EGFP-mRNA-LNP

| mRNA | pH | Particle size (nm) | PDI | Zeta (mV) | Encapsulation efficiency (%) | Drug loading capacity ($\mu g/mL$) |
|---|---|---|---|---|---|---|
| EGFP-mRNA | 4.0 | 81.53 | 0.06797 | -5.915 | 90.65 | 139.78 |
| | 4.5 | 84.41 | 0.09315 | -3.219 | 83.96 | 130.28 |
| | 5.5 | 80.94 | 0.05904 | -3.798 | 91.27 | 153.61 |
| | 6.5 | 107.1 | 0.08361 | -4.263 | 58.17 | 95.32 |
| | 7.0 | 95.81 | 0.1181 | -3.932 | 35.86 | 55.56 |

As can be seen from Table 11, when EGFP-mRNA was used, the effect of pH of buffer solutions on the change trend of drug loading capacity of the prepared EGFP-mRNA-LNP still existed, and under the condition of buffer solutions at pH of 4.5 to 5.5, the drug loading capacity of EGFP-mRNA-LNP increased significantly. pH 5.5 is preferred, at which the measured total mRNA loaded was the highest (153.61 $\mu g/mL$), meaning that the unit volume of LNP contained more mRNA, and the stability was good. It can be seen that maintaining an appropriate pH of the aqueous phase solution can significantly increase the loading capacity of EGFP-mRNA indeed, and this trend does not change with changes in the encapsulated mRNA drug.

**4. Difference in cell expressions of EGFP-mRNA-LNP with different drug loading capacities**

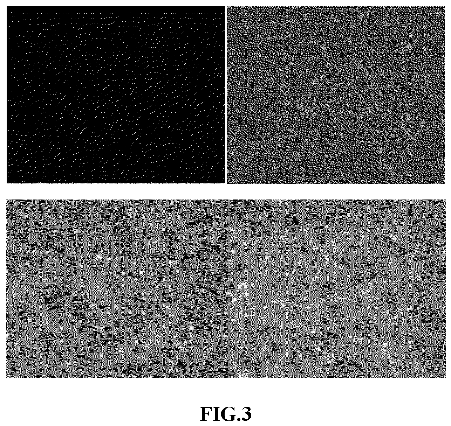

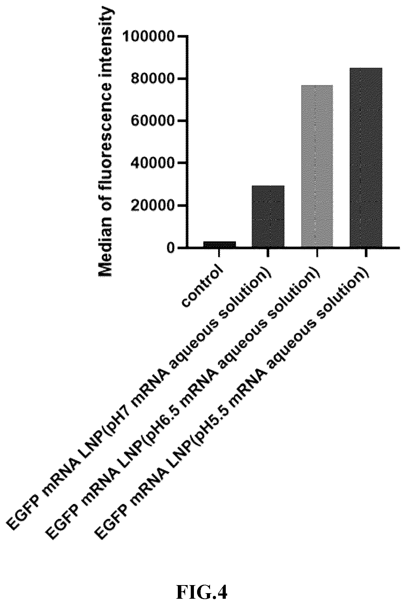

Cell transfection of HEK-293T was performed with EGFP-mRNA-LNP prepared from Tris buffer solutions at pH 5.5, 6.5 and 7.0, respectively, using blank LNP without mRNA as the control. The differences in cellular expression of mRNA-LNP with different drug loading capacities prepared from Tris mRNA aqueous phase solutions at different pH were compared through the intensity of EGFP fluorescence expression (fluorescence microscopy and flow cytometry). The results are shown in Figure 3, where control is shown at the upper left, EGFP-mRNA-LNP at pH 7.0 is shown at the upper right, EGFP-mRNA-LNP at pH 6.5 is shown at the lower left, and EGFP-mRNA-LNP at pH 5.5 is shown at the lower right. Analysis of fluorescence expression is shown in Figure 4.

According to the results of fluorescence imaging and flow cytometry after transfection of cells in Figure 3 and Figure 4, the fluorescence expression of EGFP-mRNA-LNP prepared by mRNA Tris aqueous phase solution system at pH 5.5 was correspondingly higher due to relatively higher loading capacity. However, the fluorescence expression of the other two groups of EGFP-mRNA-LNP was slightly lower due to the difference in EGFP-mRNA loaded. There was a significant difference in their fluorescence expression intensity, adequately demonstrating the enhanced biological effect due to the increase of actual drug loading capacity.

**Embodiment 3 Effect of lipid content in lipid solution on the preparation of mRNA-LNP**

**1. Effect of lipid content in lipid solution on the preparation of S protein-mRNA-LNP**

In this embodiment, the aqueous phase solution and the lipid mixture solution were prepared according to the method provided in Embodiment 1, and then used to prepare S protein-mRNA-LNP, wherein the pH of aqueous phase solution was 5.5, and the stock solutions for lipid solution were prepared according to the methods in Table 12 and Table 13, respectively:

Table 12. Formulation of composition for microneedle administration with low lipid content

| 4X aqueous phase solution | | Stock solution for lipid solution | | |
|---|---|---|---|---|
| Sample name | Sample weight | Sample name | Sample weight | Amount of anhydrous ethanol added |
| Tromethamine | 99.20 mg | SM-102 | 106.024 mg | 6 mL |
| Tris hydrochloride, Tris-HCl | 377.60 mg | Cholesterol | 44.448 mg | 6 mL |
| Glacial acetic acid | 13.76 mg | DSPC | 23.577 mg | 6 mL |
| Sodium acetate trihydrate | 64.00 mg | DMG-PEG$_{2000}$ | 11.231 mg | 6 mL |

(continued)

| 4X aqueous phase solution | | Stock solution for lipid solution | | |
|---|---|---|---|---|
| Sample name | Sample weight | Sample name | Sample weight | Amount of anhydrous ethanol added |
| Water | 40 mL | NA | NA | NA |

Table 13. Formulation of composition for microneedle administration with high lipid content

| 4X aqueous phase solution | | Stock solution for lipid solution | | |
|---|---|---|---|---|
| Sample name | Sample weight | Sample name | Sample weight | Amount of anhydrous ethanol added |
| Tromethamine | 99.20 mg | SM-102 | 141.36 mg | 4 mL |
| Tris hydrochloride, Tris-HCl | 377.60 mg | Cholesterol | 59.26 mg | 4 mL |
| Glacial acetic acid | 13.76 mg | DSPC | 31.44 mg | 4 mL |
| Sodium acetate trihydrate | 64.00 mg | DMG-PEG$_{2000}$ | 14.97 mg | 4 mL |
| Water | 40 mL | NA | NA | NA |

The particle size, PDI, encapsulation efficiency and drug loading capacity of the obtained S protein-mRNA-LNP with different lipid contents were tested respectively to investigate the effects of different lipid contents on the preparation of S protein-mRNA-LNP. The particle size, PDI, encapsulation efficiency, and drug loading capacity were tested according to the methods provided in Embodiment 1. The test results are shown in Table 14.

Table 14. Effect of different lipid contents on the preparation of S protein-mRNA-LNP

| Lipid content | Particles with low lipid content | Particles with high lipid content |
|---|---|---|
| Particle size (nm) | 175.1 | 213.5 |
| PDI | 0.09 | 0.15 |
| Encapsulation efficiency (%) | 93.99% | 88.50% |
| Drug loading capacity ($\mu$g/mL) | 75.48 | 157.75 |

As shown in Table 14, when the lipid content in the lipid solution was increased, the drug loading capacity of S protein-mRNA-LNP was significantly increased, resulting in less amounts of excipients in and less toxic side effects of the novel coronavirus mRNA vaccine; meanwhile, less dose is required to achieve better immunity, the stability is very good, and therefore, it is more suitable for microneedle administration.

**2. Effect of lipid content in lipid solution on the preparation of ZSL303-mRNA-1-LNP**

In this embodiment, the aqueous phase solution and the lipid mixture were prepared according to the method provided in Embodiment 1, and then used to prepare ZSL303-mRNA-1-LNP, wherein the stock solutions for lipid solution were prepared according to the methods in Table 15 and Table 16, respectively:

Table 15. Formulation of composition for microneedle administration with low lipid content

| 4X aqueous phase solution | | Stock solution for lipid solution | | |
|---|---|---|---|---|
| Sample name | Sample weight | Sample name | Sample weight | Amount of anhydrous ethanol added |
| Tromethamine | 99.20 mg | SM-102 | 106.024 mg | 6 mL |

(continued)

| 4X aqueous phase solution | | Stock solution for lipid solution | | |
|---|---|---|---|---|
| Sample name | Sample weight | Sample name | Sample weight | Amount of anhydrous ethanol added |
| Tris hydrochloride, Tris-HCl | 377.60 mg | Cholesterol | 44.448 mg | 6 mL |
| Glacial acetic acid | 13.76 mg | DSPC | 23.577 mg | 6 mL |
| Sodium acetate trihydrate | 64.00 mg | DMG-PEG$_{2000}$ | 11.231 mg | 6 mL |
| Water | 40 mL | NA | NA | NA |

Table 16. Formulation of composition for microneedle administration with high lipid content

| 4X aqueous phase solution | | Stock solution for lipid solution | | |
|---|---|---|---|---|
| Sample name | Sample weight | Sample name | Sample weight | Amount of anhydrous ethanol added |
| Tromethamine | 99.20 mg | SM-102 | 141.36 mg | 4 mL |
| Tris hydrochloride, Tris-HCl | 377.60 mg | Cholesterol | 59.26 mg | 4 mL |
| Glacial acetic acid | 13.76 mg | DSPC | 31.44 mg | 4 mL |
| Sodium acetate trihydrate | 64.00 mg | DMG-PEG$_{2000}$ | 14.97 mg | 4 mL |
| Water | 40 mL | NA | NA | NA |

The particle size, PDI, encapsulation efficiency and drug loading capacity of the obtained ZSL303-mRNA-1-LNP with different lipid contents were tested respectively to investigate the effects of different lipid contents on the preparation of ZSL303-mRNA-1-LNP. The particle size, PDI, encapsulation efficiency, and drug loading capacity were tested according to the methods provided in Embodiment 1. The test results are shown in Table 17.

Table 17. Effect of different lipid contents on the preparation of ZSL303-mRNA-1-LNP

| Lipid content | Particles with low lipid content | Particles with high lipid content |
|---|---|---|
| Particle size (nm) | 162.4 | 139.7 |
| PDI | 0.216 | 0.303 |
| Encapsulation efficiency (%) | 91.8 | 88.0 |
| Drug loading capacity ($\mu$g/mL) | 45.9 | 96.1 |

As shown in Table 17, when the lipid content in the lipid solution was increased, the drug loading capacity of ZSL303-mRNA-1-LNP was significantly increased, resulting in less dose and less toxic side effects, better therapeutic effects; meanwhile, the stability is very good, and it is more suitable for microneedle administration.

**Embodiment 4 Expression effect of Fluc-mRNA-LNP at cellular level**

Transfection experiment was performed on HEK 293T cells (COBIOER) in 96-well plates with the culture medium of DMEM + 10% FBS. The LNP was prepared as per the procedure in Embodiment 1, in order to facilitate observation and detection, and the S protein-mRNA is replaced by Fluc-mRNA (Hongene Biotech, FLUCmRNA (N1-Me-pseudo U), lot: FLUCMPH1B, abbreviated as Fluc-mRNA, which has been marketed for sale. The purchased sequences contain UTR sequences, promoters, etc., and can be expressed *in vitro* at cellular level and *in vivo* after delivery, so that the expression effect of mRNA at cellular level can be more intuitively characterized by fluorescence.

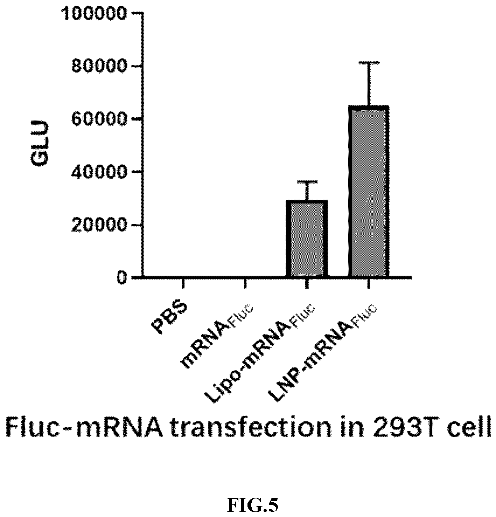

Cell transfection experiment was performed with the prepared Fluc-mRNA-LNP system; PBS was used as the blank control; the unencapsulated Fluc-mRNA was used as the negative control; Lipofectamine® 2000 Reagent (invitrogen #11668-027, abbreviated as Lipo) was used to transfect Fluc-mRNA, as the positive control. HEK 293T cells were cultured in 96-well plates (96 Well Opaque Plate, Jingan Biology, J09601) at a cell plating density of $2 \times 10^4$ cells/well, and transfected at a cell density of 60%-70% after plating for 24 hours, and the amount of transfected mRNA in the cells was 0.1 $\mu$g/well. After transfection for 48 h, fluorescent substrate working solution (D-Luciferin, Sodium Salt, Yeasen Biotechnology (Shanghai) Co., Ltd., 40901ES01) was added, and the plate was read and analyzed after incubation at 37°C for 5 min-10 min. The results are shown in Figure 5.

As can be seen from Figure 5, Fluc-mRNA was difficult to be expressed when Fluc-mRNA not encapsulated in liposomes was used for cell transfection, while the expression at cellular level could be significantly improved after Fluc-mRNA was encapsulated in liposomes; wherein the fluorescence reading of Lipofectamine 2000 was about 2800 GLU, that of the prepared Fluc-mRNA-LNP was about 6500 GLU, which increased by more than 2 times compared to Lipofectamine 2000. The main reason was that the pH of aqueous solution of Lipofectamine 2000 was not suitable, and the lipid content was low, resulting in a low drug loading capacity. Even if the amounts of transfected drugs are consistent (which means consistent contents of mRNA other than consistent volumes of drug used, i.e. with equal amounts of Fluc-mRNA), it is difficult to achieve good expression effect due to more excipients added. However, the Fluc-mRNA-LNP prepared in this embodiment is of higher drug loading capacity, less excipients and is more stable, which can stimulate more expression at cellular level with the same amount of transfected drug, achieve better delivery and expression effect, and realize the biological function efficiently.

**Embodiment 5 Expression effect of Fluc-mRNA-LNP at animal level**

In this Embodiment, the LNP was prepared as per the procedure in Embodiment 1, in order to facilitate observation and detection, and the S protein-mRNA was replaced by Fluc-mRNA (Hongene Biotech, FLUCmRNA (N1-Me-pseudo U), lot: FLUCMPH1B, abbreviated as Fluc-mRNA, which has been marketed for sale. The purchased sequences contain UTR sequences, promoters, etc., and can be expressed *in vitro* at cellular level and *in vivo* after delivery, so that the expression effect of mRNA at animal level can be more intuitively characterized by fluorescence.

The expression level and tissue distribution of Fluc-mRNA-LNP under different routes of administration are observed at animal level in this Embodiment. The specific experimental design is shown in Table 18. The small-animal imaging method (multi-mode *in vivo* animal imaging system, Guangzhou Biolight Biotechnology Co., Ltd., BLT) was used to compare the differences in Flue expression and duration after five different routes of administration, including intravenous tail vein administration, intramuscular administration, subcutaneous administration, conventional intradermal administration, and three-needle microneedle administration.

Table 18. Different routes of administration

| Groups | N | Treatment group | Administered dose | Route of administration | Frequency of administration | Imaging system | Imaging time |
|---|---|---|---|---|---|---|---|
| G1 | 3 | NT | N/A | N/A | Single dose | Ani View 100 (BL T) imaging | 6 h, 12 h, 24 h, 48 h, and 96 h |
| G2 | 3 | Empty LNP | N/A | IV (regular syringe, intravenous tail vein injection) | | | |
| G3 | 3 | Fluc-mRNA-LNP | 10 $\mu$g/ mouse | SC (regular syringe, subcutaneous injection) | | | |
| G4 | 3 | Fluc-mRNA-LNP | 10 $\mu$g/ mouse | IV (regular syringe, intravenous tail vein injection) | | | |
| G5 | 3 | Fluc-mRNA-LNP | 10 $\mu$g/ mouse | IM (regular syringe, intramuscular injection) | | | |
| G6 | 3 | Fluc-mRNA-LNP | 10 $\mu$g/ mouse | ID (regular syringe, intradermal injection) | | | |
| G7 | 3 | Fluc-mRNA-LNP | 10 $\mu$g/ mouse | MN (G) (microneedle administration) | | | |

(continued)

| Groups | N | Treatment group | Administered dose | Route of administration | Frequency of administration | Imaging system | Imaging time |
|---|---|---|---|---|---|---|---|
| G8 | 3 | Fluc-mRNA-LNP | 3.6 μg/ mouse | MN (microneedle administration) | | | |
| G9 | 3 | mRNA | 10 μg/ mouse | ID (regular syringe, intradermal injection) | | | |

A total of 30 female Balb/c mice, SPF grade, 6-8 weeks old were used for the study, they were randomly assigned to one of the groups with 3 mice per group based on body weight, and then administered once. The administration time point was defined as 0 h; after the last imaging time point, the follow-up observation duration was tentatively 1 week, and the details were to be determined; the actual concentration of Fluc-mRNA was 71.92 μg/mL. (The actual injection volume for 10 μg/mouse was 140 μL, and that for 3.6 μg/mouse was 50 μL). G9 was naked Fluc-mRNA not encapsulated in LNP. Small-animal imaging was started from 6 h after administration with the following imaging method. Before imaging, hair removal was performed on mice.

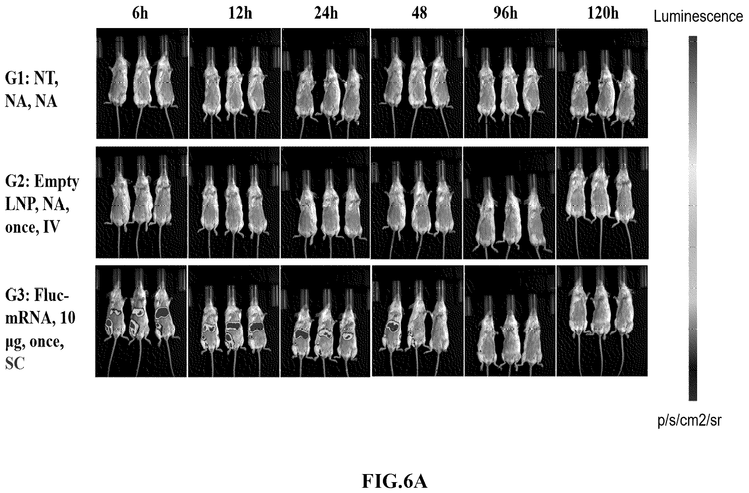

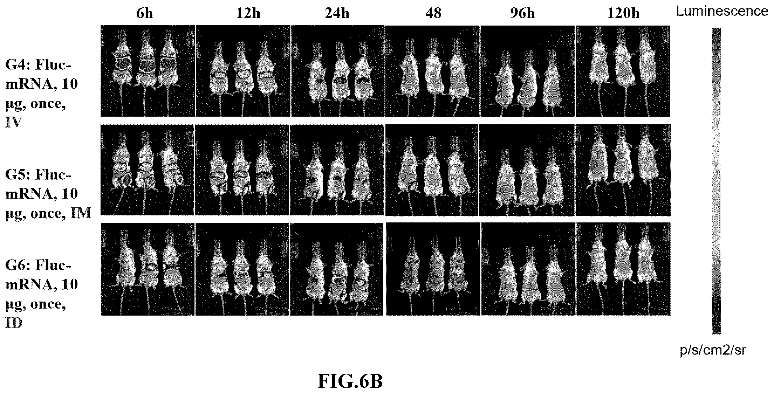

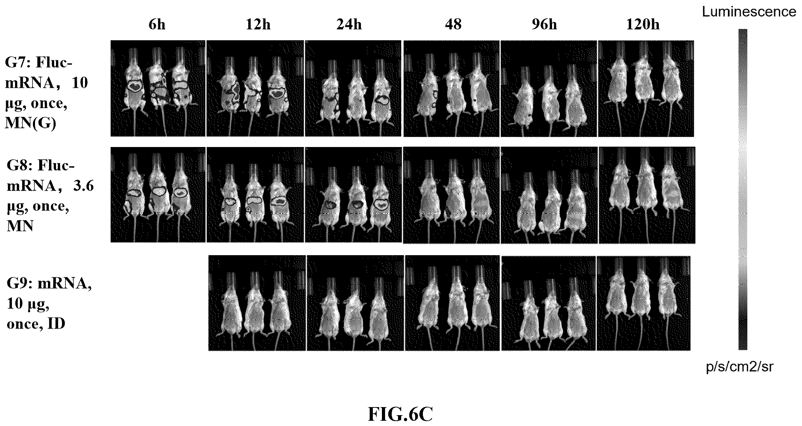

The imaging results are shown in Figure 6. According to the imaging results, after injection of Fluc-mRNA-LNP, signals were shown for all routes of administration, which proved that the nucleic acid delivery system of Fluc-mRNA-LNP has good performance. Meanwhile, Fluc-mRNA-LNP was mainly expressed in the injection site and liver of mice. There were also a few expressions in other viscera that shall be analyzed for details from the results of tissue distribution. In this Embodiment, the optical signal intensity of the injection site and liver of mice was analyzed respectively, additionally, the relationship between the injection site and liver and the dose of microneedle administration, as well as that between different routes of administration with the total expression were investigated.

**1. Analysis of optical signal intensity at the injection site under different routes of administration**

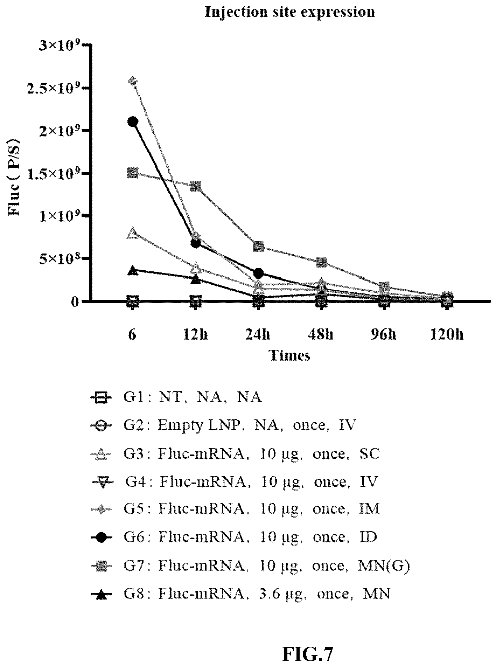

The analysis results of optical signal intensity at the injection site under different routes of administration are shown in Figure 7. The detailed data of intensity is shown in Table 19. Figure 7 and Table 19 indicate that Flue expression at the injection site decreased over time for all routes of administration. Compared with other groups, group MN (micro-needle) showed the longest duration of expression, and a high expression was detected even at 96 h. The signals of groups G5 and G6 were the strongest at 6 h, but then decreased rapidly; after 6 h, the signal of group MN at each time point was stronger than that of other groups, and reached 5.42E+07 at 120 h. Even the injection dose of MN in G9 group decreased to 3.6 μg, and the high expression lasted for a long time. Thus, microneedle administration can improve mRNA expression at the injection site and prolong the expression time.

Table 19. Results of signal intensity at the injection site of mice under different routes of administration

| Groups | 6h | 12h | 24 h | 48 h | 96 h | 120 h |
|---|---|---|---|---|---|---|
| G1: NT | 0 | 0 | 0 | 0 | 0 | 0 |
| G2: Empty LNP | 0 | 0 | 0 | 0 | 0 | 0 |
| G3: Fluc-mRNA-LNP, 10 μg, once, SC | 8.07 E+08 | 3.92 E+08 | 1.53 E+08 | 1.35 E+08 | 3.65 E+07 | 9.19 E+06 |
| G4: Fluc-mRNA-LNP, 10 μg, once, IV | N/A | N/A | N/A | N/A | N/A | N/A |
| G5: Fluc-mRNA-LNP, 10 μg, once, IM | 2.58 E+09 | 7.70 E+08 | 1.97 E+08 | 2.19 E+08 | 9.83 E+07 | 3.09 E+07 |
| G6: Fluc-mRNA-LNP, 10 μg, once, ID | 2.11 E+09 | 6.87 E+08 | 3.27 E+08 | 1.46 E+08 | 4.97 E+07 | 3.46 E+07 |
| G7: Fluc-mRNA-LNP, 10μg, once, MN (G) | 1.51 E+09 | 1.35 E+09 | 6.42 E+08 | 4.56 E+08 | 1.72 E+08 | 5.42 E+07 |
| G8: Fluc-mRNA-LNP, 3.6μg, once, MN | 3.67 E+08 | 2.68 E+08 | 4.62 E+07 | 8.56 E+07 | 2.39 E+07 | 1.52 E+07 |

**2. Analysis of optical signal intensity in the liver under different routes of administration**

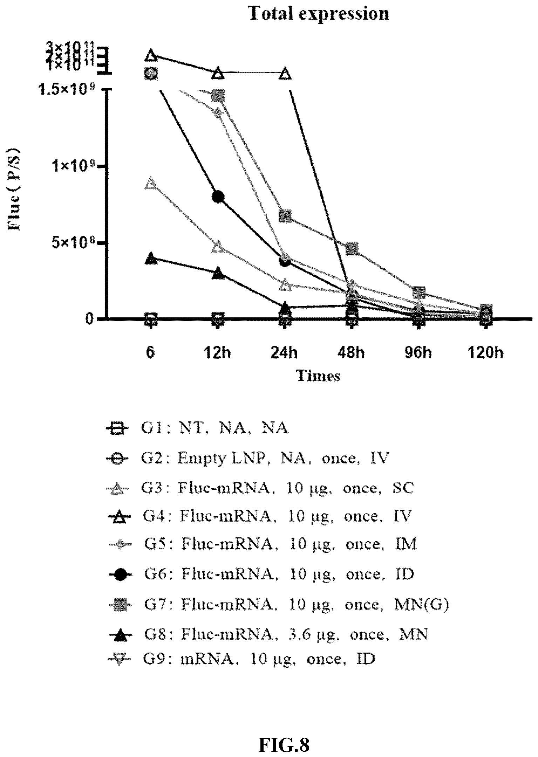

Analysis results of optical signal intensity in the liver under different routes of administration are shown in Figure 8, and the detailed data of intensity are shown in Table 20.

Table 20. Results of optical signal intensity in the liver under different routes of administration

| Groups | 6h | 12h | 24h | 48h | 96h | 120h |
|---|---|---|---|---|---|---|
| G1: NT | 0 | 0 | 0 | 0 | 0 | 0 |
| G2: Empty LNP | 0 | 0 | 0 | 0 | 0 | 0 |
| G3: Fluc-mRNA-LNP, 10 $\mu$g, once, SC | 8.50E+07 | 8.84E+07 | 7.22E+07 | 3.38E+07 | 1.90E+06 | 8.00E+05 |
| G4: Fluc-mRNA-LNP, 10 $\mu$g, once, IV | 2.19E+11 | 1.13E+10 | 3.88E+09 | 1.38E+08 | 3.88E+06 | 3.02E+06 |
| G5: Fluc-mRNA-LNP, 10 $\mu$g, once, IM | 1.15E+09 | 5.80E+08 | 2.08E+08 | 6.54E+06 | 1.03E+06 | 1.29E+06 |
| G6: Fluc-mRNA-LNP, 10 $\mu$g, once, ID | 1.41E+08 | 1.14E+08 | 5.55E+07 | 9.60E+06 | 1.91E+06 | 6.91E+05 |
| G7: Fluc-mRNA-LNP, 10 $\mu$g, once, MN (G) | 1.12E+08 | 1.11E+08 | 3.26E+07 | 6.26E+06 | 2.12E+06 | 8.56E+05 |
| G8 : Fluc-mRNA-LNP, 3.6 $\mu$g, once, MN | 3.39E+07 | 3.33E+07 | 2.96E+07 | 3.27E+06 | 1.82E+06 | 2.87E+05 |
| G9:mR NA, 10 $\mu$g, once, MN | N/A | N/A | N/A | N/A | N/A | N/A |

As can be seen from Figure 8 and Table 20, compared with other groups, group IV (intravenous tail vein injection) and group IM (intramuscular injection) showed a faster and higher expression in the liver. The signal of group IV began to decrease rapidly after 6 h, and that of group IM began to decrease rapidly after 12 h. The expression in the liver of group MN was significantly lower than that of group IV and group IM. Compared with group SC (subcutaneous injection) and group ID (intradermal injection), group MN showed a lower expression in the liver at the first 24 h or 48 h. Later, all the expression decreased gradually over time, and there was little difference or slight increase between group MN and group SC (subcutaneous injection) or group ID (intradermal injection), which may be due to the fact that microneedles can prolong mRNA expression time. However, the overall expression intensity was still lower than that of group SC (subcutaneous injection) and group ID (intradermal injection). Thus, microneedle administration of mRNA can reduce the expression in the liver.

**3. Relationship between injection site, liver and the dose of microneedle administration**

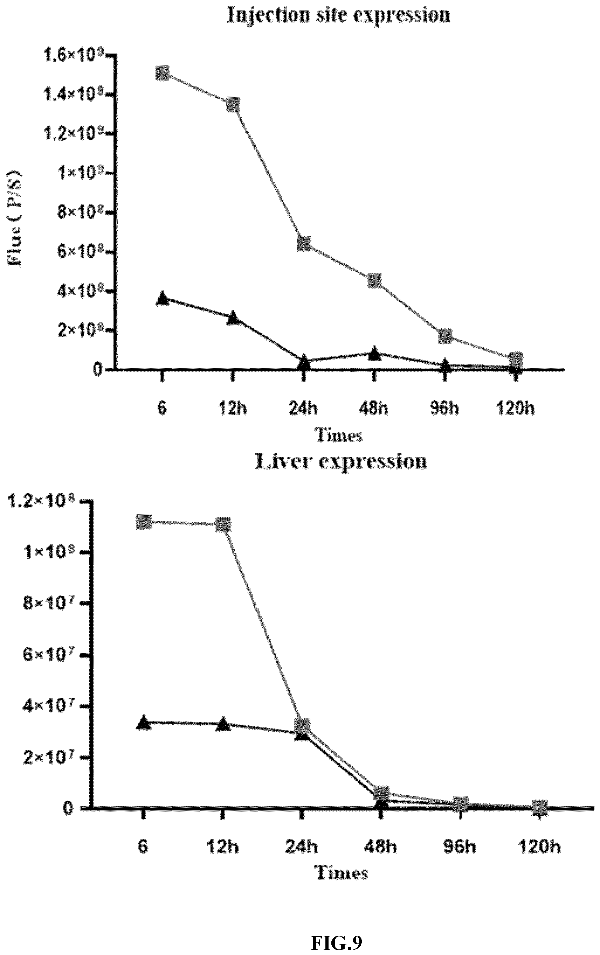

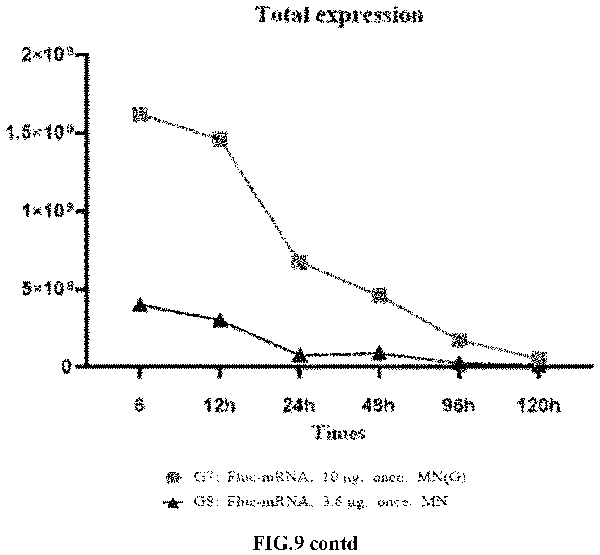

Microneedle was used to administer 10 $\mu$g and 3.6 $\mu$g of Fluc-mRNA, respectively, so as to investigate the optical intensity changes at the injection site and in the liver with different doses. The results are shown in Figure 9, wherein the upper left panel shows the optical intensity changes of Fluc-mRNA at the injection site at different doses, the upper right panel shows the optical intensity changes of Fluc-mRNA in the liver at different doses, and the lower panel shows the total optical intensity changes of Fluc-mRNA at different doses.

As can be seen from Figure 9, in terms of the total expression, the two groups showed an obvious dose-dependent trend. The optical signal intensity at the injection site showed an obvious dose-dependent trend. The liver also showed a dose-dependent trend before 24 h, and then all the signals showed a decreasing trend subsequently. It can be seen that, during microneedle administration, the injection site was more responsive to the dose, and the high level of expression lasted longer. While the liver showed less expression, and the expression in the liver decreased rapidly even at a higher dose. Therefore, during microneedle administration, mRNA expression is mainly concentrated at the injection site, which can increase the expression in skin and muscle, but decrease the expression in the liver.

**4. Analysis of the relationship between different routes of administration and injection site, liver, and total expression**

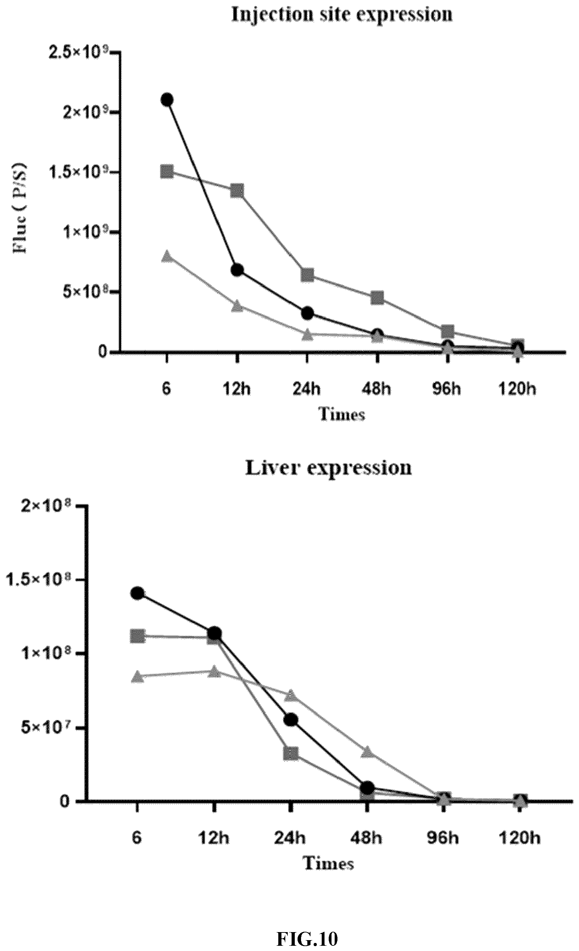

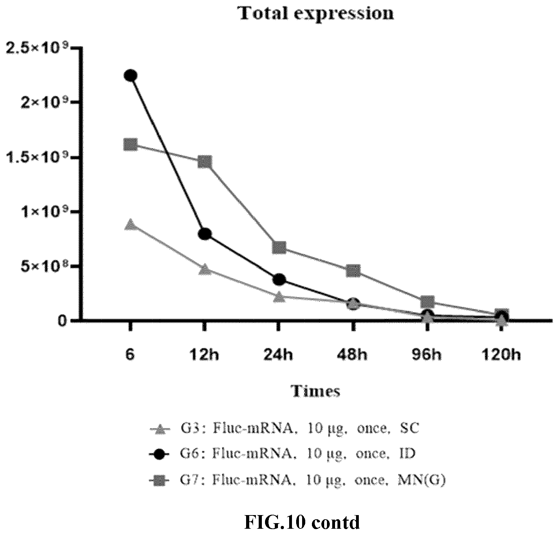

10 $\mu$g of Fluc-mRNA-LNP was injected once by SC (subcutaneous injection), ID (intradermal injection), and MN (microneedle administration), respectively. The analysis results of the optical signal intensity and total expression at the injection site and in the liver under different routes of administration are shown in Figure 10, wherein the upper left panel compares the expression of Fluc-mRNA at the injection site across different routes of administration, and the upper right panel compares the expression of Fluc-mRNA in the liver across different routes of administration, and the lower panel compares the total expression of Fluc-mRNA across different routes of administration;

As can be seen from Figure 10, the total expression and injection site expression of the group MN (microneedle administration) were significantly higher than those of the group SC and group ID after 12 h, and the downward trend of the group MN was more gentle and could last longer than the other two groups. However, in the liver, the expression of the group MN decreased rapidly after 12 h, which was significantly lower than that of the group SC and group ID, and the downward trend was steeper, indicating that microneedle administration can significantly reduce the expression in the liver and increase the expression at the injection site.

### 5. Analysis of the relationship between different routes of administration and injection site, liver, and total expression after 48 h

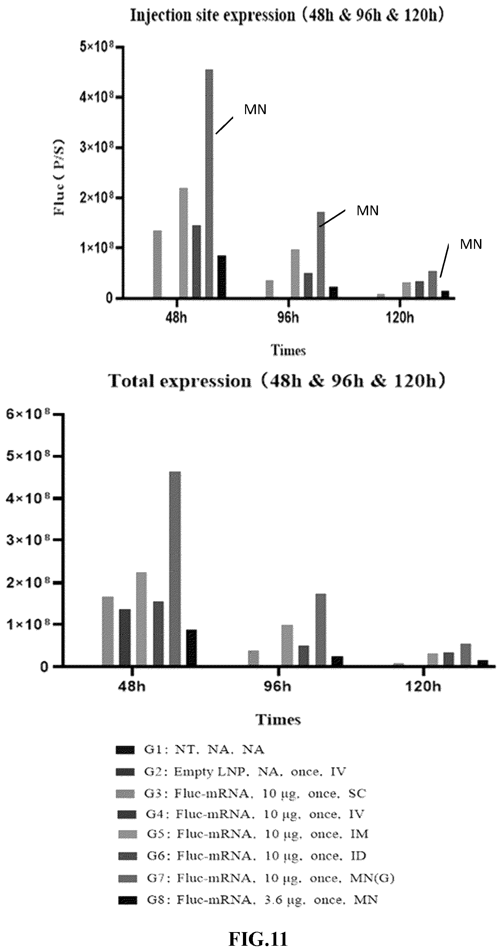

The analysis results of the optical signal intensity and total expression at the injection site and in the liver of mice 48 h after drug administration with different routes are shown in Figure 11, wherein the upper left panel compares the expression of Fluc-mRNA at the injection site after 48 h, the upper right panel compares the expression of Fluc-mRNA in the liver after 48 h, and the lower panel compares the total expression of Fluc-mRNA after 48 h.

As can be seen from Figure 11, in terms of the expression at the injection site and the total expression, the expression of group MN was significantly higher than that of other groups 48 h after drug administration, and even with a 3.6 $\mu$g injection dose (group G8), the expression could reach that with other routes of administration, and the expression time can be significantly prolonged. However, the expression in the liver of group MN was low after 48 h and decreased the fastest.

### 6. Analysis of Fluc-mRNA distribution in different parts of mice under different routes of administration

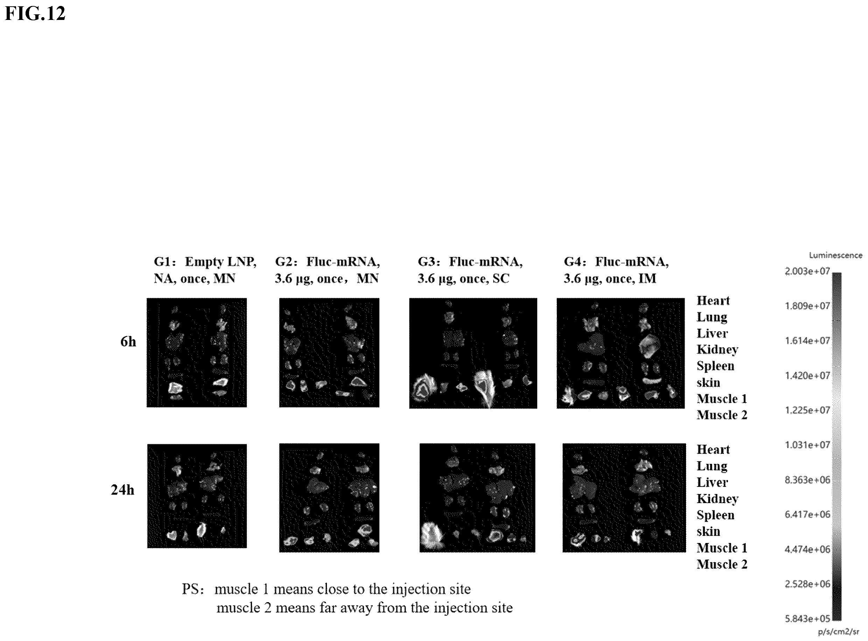

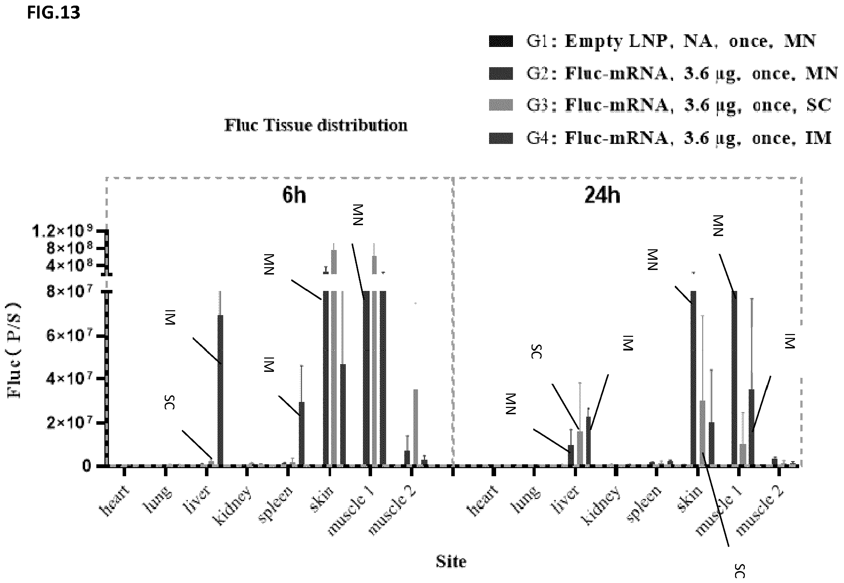

Four different routes of administration as shown in Table 21 were performed at animal level in this Embodiment, and the expression of Flue in viscera was observed by necropsize mice 6 h and 24 h after administration. The BLT imaging is shown in Figure 12, and the specific intensity analysis of each part of the mice is shown in Figure 13.

Table 21. Study protocol

| Groups | N | Treatment group | Administered dose | Route of administration | Frequency of administration | Imaging system | Imaging time | Site necropsized |
|--------|---|-----------------|-------------------|-------------------------|-----------------------------|----------------|--------------|------------------|
| 1 | 4 | Empty LNP | N.A. | MN | Single dose | AniView 100 (BLT) imaging | The necropsy was performed 5 min after substrate injection, two mice were necropsized each time, and the images were taken | Heart, lung, liver, kidney, spleen, skin, and muscles |
| 2 | 4 | Fluc-mRNA-LNP | 3.6 μg/mouse | MN | | | | | |
| 3 | 4 | Fluc-mRNA-LNP | 3.6 μg/mouse | SC | | | | | |
| 4 | 4 | Fluc-mRNA-LNP | 3.6 μg/mouse | IM | | | | | |
| | | | | | | | at two time points (6 h and 12 h). | |

As can be seen from Figure 13, at 6 h, the expression of Flue mRNA was primarily distributed at the injection site for group MN and group SC, especially for group MN, the expression was also distributed in the skin and muscles. For group IM, the expression was primarily distributed in the liver and spleen. Group MN showed expression in the liver at 24 h, but the signal was weaker than that of group IM and group SC. However, the signal in the skin and muscles remained high, and the MN had a higher signal intensity than SC, followed by IM. Thus, after microneedle administration, the expression is mainly promoted in the skin and muscles and decreased in the liver, which can decrease toxicity and increase efficacy.

**Embodiment 6 Expression effect of S protein-mRNA-LNP at animal level**

In this Embodiment, a total of 60 female Balb/c mice, SPF grade, 6-8 weeks old were used, they were randomly assigned to one of the groups with 3 mice per group based on body weight, and then administered from Day 0 as per the study protocol in Table 22.

Table 22. Study protocol

| Groups | Number of mice | Treatment group | Administered dose | Route of administration | Drug administration time | Blood sampling time |
|---|---|---|---|---|---|---|
| G1 | 6 | PBS | N/A | MN (microneedle administration) | Day0, Day14 | Day-7, Day1 (24h after injection) Day 7, Day 14, Day 21, Day 28 |
| G2 | 6 | Empty LNP | N/A | | | |
| G3 | 6 | S protein-mRNA | 10 μg/mouse | | | |
| G4 | 6 | S protein-mRNA-LNP | 10 μg/mouse | IM (intramuscular injection) | | |
| G5 | 6 | | | ID (intradermal injection) | | |
| G6 | 6 | | 10 μg/mouse | MN (microneedle administration) | | |
| G7 | 6 | | 3.6 μg/mouse | | | |
| G8 | 6 | | 1.2 μg/mouse | | | |

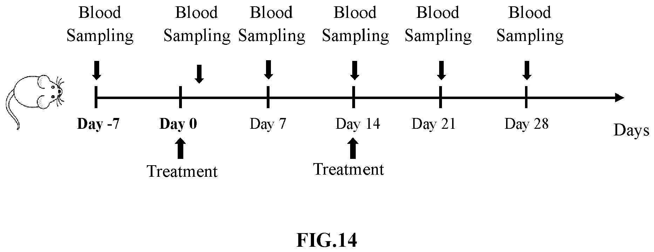

Orbital blood samples were collected before and after administration on Day 7, Day 14, Day 21, and Day 28. The process of serum collection is as follows: According to the experimental design, mice in all groups were selected in accordance with the order of ear tags, serum was collected from 6 mice in each group at 6 time points, and blood samples of each mouse were collected. The 6 time points are: 7 days before the first dose, 1 day after the first dose, 7 days after the first dose, at the second dose, 7 days after the second dose, and 14 days after the second dose (the process is shown in Figure 14). Blood samples were collected from the orbit, and after standing at room temperature for half an hour, serum samples were collected by centrifugation at 3000 rpm for 20 min. The collected serum samples were stored at -80°C for subsequent assays. About 100 μL of blood was collected, and the anti-S protein antibody level in the serum of mice was assayed to evaluate the humoral immunity effect of the mRNA vaccine.

The SARS-CoV-2 S protein antibody titer in the serum was assayed after each blood collection, and the expression of novel coronavirus S protein was assayed on Day 1. Meanwhile, the reagents required for this assay were purchased from ACRO (Beijing AcrobioSystems Biotechnology Co., Ltd.).

The assay procedure for SARS-CoV-2 S protein antibody titer is as follows: 1. Prepare 1 × Washing Buffer: Dilute 50 mL of 10 × Washing Buffer to 500 mL with ultrapure water/deionized water. Prepare the Positive Control working solution and Negative Control working solution. Dilution of samples: For the assay of antibody titer. 2. Add 100 μL of the diluted test sample, Positive Control working solution, and Negative Control or reference standard working solution to the corresponding plate well. For blank control, add 100 μL of Dilution Buffer into the well. 3. Incubation: Seal the plate with plate sealers, and incubate at 37°C in a constant temperature incubator for 1.0 h. 4. Wash the microplate. 5. Add the HRP marker. 6. Color development: Add 100 μL of Substrate Solution into each well, seal the plate with plate sealers, and incubate at 37°C in a constant temperature incubator for 20 min, protected from light. 7. Add 50 μL of Stop Solution into each well, and gently shake the ELISA plate to mix well. 8. Determine the absorbance of each well at wavelengths of 450 nm and 630 nm by a microplate reader.

The assay procedure for S protein is as follows: 1. Dilute the SARS-CoV-2 Spike Protein with Dilution Buffer at 2-fold serial dilution, with a dilution interval of 0.195 ng/mL-12.5 ng/mL. 2. Add 100 μL of diluted samples and 100 μL of prepared standard curve samples into the corresponding wells. Add 100 μL of Dilution Buffer into the blank control well. Seal the plate with plate sealers, gently shake the ELISA plate to mix well, and incubate at 37°C for 1.0 h. 3. Dilute 50 mL of 10 × Washing Buffer to 500 mL with ultrapure water/deionized water. Discard the liquid in the wells, pat the ELISA plate dry, soak with 1 × Washing Buffer by 300 μL/well for 30 s to wash, pat the ELISA plate dry, and wash 3 times in total. 4. Dilute Biotin-Anti-SARS-CoV-2 Spike Protein Antibody to 0.5 μg/mL with Dilution Buffer, add 100 μL into each well, seal the plate with plate sealers, and incubate at 37°C for 1.0 h. 5. Discard the liquid in the wells, pat the ELISA plate dry, soak with 1 × Washing Buffer by 300 μL/well for 30 s to wash, pat the ELISA plate dry, and wash 3 times in total. 6. Dilute Streptavidin-HRP to 0.1 μg/mL with Dilution Buffer, add 100 μL into each well, seal the plate with plate sealers, and incubate at 37°C for 1.0 h. Prepare freshly before use. 7. Discard the liquid in the well, pat the ELISA plate dry, soak with 1 × Washing Buffer by 300 μL/well for 30 s to wash, pat dry, and wash 3 times in total. 8. Add 100 μL of Substrate Solution into each well, seal the plate with plate sealers, and incubate at 37°C for 20 min, protected from light. 9. Add 50 μL of Stop Solution into each well and gently shake the ELISA plate to mix well. 10. Determine the absorbance of each well at wavelengths of 450 nm and 630 nm by a microplate reader. Take a reading within 3 min before stopping.

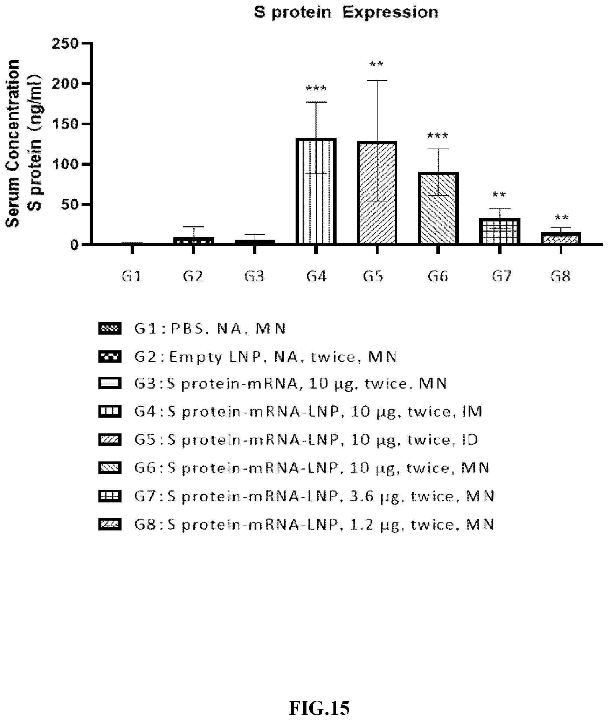

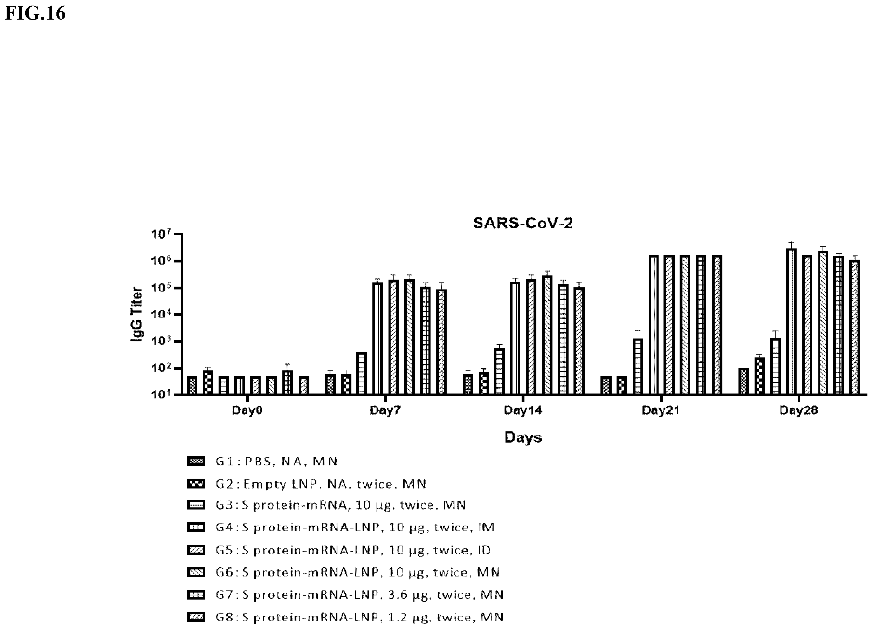

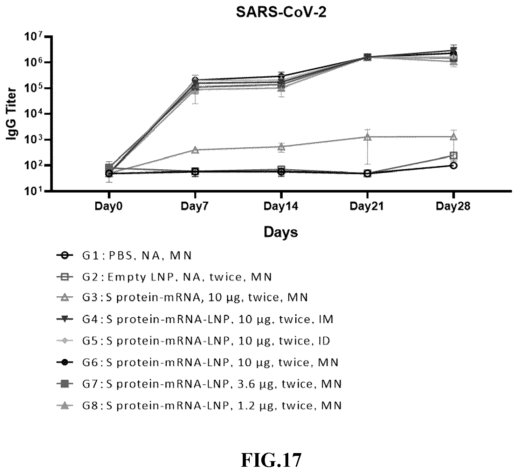

The assay results of the expression of the novel coronavirus S protein in serum are shown in Figure 15 (G1-G8 from left to right), and the assay results of the SARS-CoV-2 S protein antibody titer are shown in Figure 16 and Figure 17.

As can be seen from Figure 15, S protein could be detected in the serum at 24 h after injection of S protein-mRNA-LNP. G4 & G5 & G6 were significantly different from the blank group. The expression by ID & IM was about 125 ng/ml, and the expression by MN was about 100 ng/ml. G6 & G7 & G8 had a dose-effect relationship, the expression of which was dose-dependent. This assay was for the expression of protein in serum, while expression by MN was more distributed at the injection site and had less ability to enter the blood, so the expression of group MN was lower than that of other groups, but its antibody titer was not low (see Figure 16).

As can be seen from Figures 16 and 17, the titer of S protein-mRNA-LNP was about 105 on Day 7 by IM (intramuscular injection), ID (intradermal injection), and MN (microneedle administration). On Day 14, there was no obvious decrease, while on Day 21 and Day 28, there was a certain increase after the second booster injection and the titer remained stable. In addition, three doses were set for microneedle administration, but the antibody titer showed no statistical difference, and only a dose of 1.2 μg by microneedle administration can achieve an antibody titer the same as a dose of 10 μg by the other three routes of administration, thus making it possible to decrease vaccine dose in order to increase clinical safety, further indicating that microneedle administration can achieve the same antibody titer with a lower dose that provides support for the use of a low dose of vaccine. Thus microneedle administration has the potential to improve the safety of vaccines.

**Embodiment 7 Expression of bispecific antibody mRNA protein at cellular level**

In this embodiment, the expression level of 293T cells with high mRNA-loaded ZSL303-mRNA-1-LNP prepared in Embodiment 1 was detected. EpCAM (Aero Biosystems #EPM-H5254) was used as antigen, Anti-His-HRP (Genscript #A00612) was used as secondary antibody, and the QELISA method was adopted to detect whether there are expression products in the 293T expression supernatant, and to detect the expression of ZSL303-mRNA-1-LNP using two transfection reagents, Lipofectamine®2000 Reagent (invitrogen #11668-027; Lipo2k) and Lipofectamine®MessengerMAXTM Reagent (invitrogen#LMRNA008; Hereinafter referred to as LipoMAX). The experiment process is as follows:

1. HEK 293T (human embryonic kidney cells), Culture Medium: DMEM+10%FBS;
2. Culture in a 24-well plate with a cell density of $2 \times 10^5$. Cells should be plated 24 h prior to transfection.
3. Perform transfection with the transfection conditions as shown in Table 3, and the antibody expression results after transfection are shown in Table 23.

Table 23. Transfection conditions

| No. | Transfection reagent | Content ofZSL303-mRNA-1 | mRNA: Transfect ion reagent | Volume of transfection reagent | Concentration of antibody (μg/mL) | | Max OD450 (1:40) | |
|---|---|---|---|---|---|---|---|---|
| | | | | | 24 h | 36 h | 24 h | 36 h |
| 1 | Lipo2k | 2 μg | 1:2 | 4 μL | 1.802 | 2.556 | 2.151 | 2.250 |
| 2 | | | 1:3 | 6 μL | 1.657 | 2.752 | 2.057 | 2.304 |
| 3 | | | 1:4 | 8 μL | 1.549 | 1.915 | 1.980 | 2.018 |
| 4 | | 3μg | 1:2 | 6 μL | 1.583 | 2.087 | 2.005 | 2.090 |
| 5 | | | 1:3 | 9 μL | 1.628 | 2.078 | 2.037 | 2.086 |
| 6 | | | 1:4 | 12 μL | 1.467 | 1.773 | 1.918 | 1.952 |
| 7 | LipoMAX | 2 μg | 1:2 | 4 μL | 1.748 | 2.243 | 2.117 | 2.148 |
| 8 | | | 1:3 | 6 μL | 1.612 | 2.027 | 2.026 | 2.066 |
| 9 | | | 1:4 | 8 μL | 1.477 | 1.897 | 1.925 | 2.010 |
| 10 | | 3 μg | 1:2 | 6 μL | 1.678 | 2.261 | 2.071 | 2.155 |
| 11 | | | 1:3 | 9 μL | 1.577 | 1.968 | 2.000 | 2.041 |
| 12 | | | 1:4 | 12 μL | 1.297 | 1.507 | 1.775 | 1.810 |
| 13 | Blank | | | | 0.000102 | 0.000171 | 0.0659 | 0.0558 |

As can be seen from Table 23, ZSL303-mRNA-1 was expressed under all transfection conditions. The expression effect of ZSL303-mRNA-1 was proved to be good at the cellular level, and the bispecific antibody can be secreted to the cell supernatant.

For the group in which the content of mRNA in ZSL303-mRNA-1 was 3 μg, and the ratio of mRNA to the transfection reagent volume was 1:4, the expression effect was the worst during all the groups. The reason should be that the transfection reagent ratio was larger and the toxicity to the same amount of cells was greater.

Regardless of the transfection reagent, when the group in which the content of ZSL303-mRNA-1 was 2 μg, and the ratio of mRNA to the transfection reagent volume was 1:2, the expression effect was the best during all the groups. It was speculated that 4 μL of transfection reagent can saturate about 2 μg of mRNA, so the transfection effect was good and the efficiency was high. Therefore, it is preferred to select the group in which the content of ZSL303-MRN-1 is 2 μg, the ratio of mRNA to the transfection reagent volume is 1:2, and the transfection reagent volume is 4 μL, so that a high expression can be achieved after transfection.

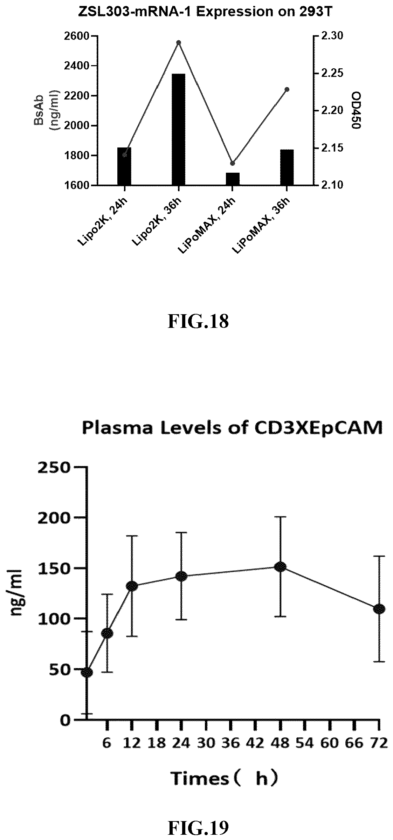

The expression results of ZSL303-mRNA-1 respectively at 24 h and 36 h when using transfection reagents Lipo2k and LipoMAX are shown in Figure 18 and Table 24.

Table 24. Expression results of ZSL303-mRNA-1 in cell supernatant by ELISA

| Conditions | Bispecific antibody (ng/mL) | OD450 |
|---|---|---|
| Lipo2k 24 h | 1805 | 2.151 |
| Lipo2k 36 h | 2556 | 2.25 |
| LipoMAX 24 h | 1748 | 2.117 |
| LipoMAX 36 h | 2243 | 2.148 |

As can be seen from Figure 18 and Table 24, when different transfection reagents were used, the ELISA assay results of the supernatants collected at different time points indicated that the concentration of antibody was the highest at 36 h, and the samples transfected with Lipo2K transfection reagent had the highest expression.

**Embodiment 8 Expression of ZSL303-mRNA-1-LNP in mouse plasma**

In this Embodiment, the expression level of ZSL303-mRNA-1-LNP provided in Embodiment 1 was detected in mouse plasma. EpCAM was used as antigen and Anti-His-HRP was used as secondary antibody to detect the accumulation of drug expression in the mouse plasma extracted at different time points, and predict a good combined condition according to the accumulation of expression. The specific experimental process is as follows:

1. Collection of samples: At 1 h, 6 h, 12 h, 24 h, 48 h, and 72 h after administration (ZSL303-mRNA-1-LNP, 5μg/mouse) on Day 15, collect blood from animals by tail clipping with a collection volume of about 40 μL/mouse. Use EDTA-$K_2$ as the anticoagulant and place the blood on the ice. Centrifuge the anticoagulated whole blood at 8000 rpm for 10 min at 2-8°C. Collect 20 μL of upper plasma and store it at -80°C. 2. Coat the antigen EpCAM (Aero Biosystems #EPM-H5254) at a concentration of 0.5 μg/mL using antigen coating reagent (Sangon Biotech #BBI E661004-0100) and 96-well ThermoFisher NUNC ELISA plate, and store in a refrigerator at 4°C for 12 h. 3. Take out the coated ELISA plate and wash it once with 1 × PBST (containing 0.5% Tween 20) solution. 4. Block the ELISA plate with the blocking solution balanced to room temperature (PBS solution containing 2%BSA (Vetec V900933-100G)) and add 200 μL to each well. After completion, cover all the wells with the plate sealers (Sangon Biotech #BBI F600418-0001), and incubate at room temperature for 1 h. 5. Dilute the mouse plasma obtained at various time points with the above blocking solution and place into 96-well dilution plate (Beyotime #FPT021). 6. Dilute the CD3xEpCAM bisspecific antibody with the blocking solution and place it into the 96-well dilution plate. 7. Take out the blocked ELISA plate, and wash it with 1 × BST solution 3 times. 8. Add 100 μL of the diluted mouse plasma and positive control in Steps 5 and 6 above to each well, respectively, cover all the wells with the above blocking solutions, and incubate at room temperature for 2 h. 9. Dilute the secondary antibody (THE™ His Tag Antibody [HRP], mAb, Mouse, Genscript-A00612) in this Embodiment with the blocking solution in a ratio of 1:2500. 10. Take out the ELISA plate incubated for 2 h, and wash it 3 times with the above 1 × PBST solution. 11. Add 100 μL of the diluted secondary antibody solution in Step 8 to each well, cover all the wells with the sealers, and incubate at room temperature for 1 h; 12. Take out the ELISA plate incubated for 1 h and wash it 6 times with 1 × PBST solution. 13. Add 100 μL of TMB single-component substrate solution (Solarbio #PR1200) into each well. After completion, incubate in a dark environment at room temperature for 8 min. 14. Add 100 μL of the ELISA reaction stop solution (Sangon Biotech #BBI E661006-0200) to each well to stop the above reaction. 15. Read immediately the OD value under the excitation light at 450 nm in the software (Molecular Devices SoftMax Pro 7.1) using the microplate reader (Molecplar Devices SpextraMax® iD3). 16. Record and analyze OD values obtained by GraphPad Software Prism 8 and make a plot (see Figure 19).

As can be seen from Figure 19, the concentration of the CD3×EpCAM bisspecific antibody in mouse plasma gradually reached or exceeded the peak at 12 h, 24 h, and 48 h, among which the highest concentration was about 150 ng/mL at 48 h, and then gradually decreased over time.

**Embodiment 9 Tumor inhibitory effect in animal model with a reconstituted immune system**

In this Embodiment, the antitumor activity of the test article (ZSL303-mRNA-1-LNP prepared in Embodiment 1) by single administration was evaluated using PBMC humanized mice xenograft tumor model of human colon cancer HCT-15 cells. A total of 16 animals were inoculated in this Embodiment, 12 of which were divided into 2 groups and administered immediately after grouping by microneedle administration once a week for 3 consecutive times. Three microneedle administrations of PBS (blank control) were performed for the first group, and three microneedle administrations (MN, QW × 3) were performed for the second group. In the treatment group, PK sampling was performed after the last administration (Day 15), and all animals were killed on Day 23. The experiment ended after the tumors were weighed and photographed.

Experimental animals: Species and strain: NOG mice; sex and age: Female, 8-10 weeks old; body weight: 18-20 g, the deviation is about ± 20% of the mean body weight; number of animals inoculated: 16; number of animals included: 12; animal source: Beijing Vital River Laboratory Animal Technology Co., Ltd.; production license number: SCXK (Beijing) 2021-0006; animal certificate number: 110011220100517757.

Cell line: Human colon cancer cell line HCT-15, purchased from the National Collection of Authenticated Cell Cultures.

Culture medium: RPMI-1640 medium, DMEM medium, and fetal bovine serum (FBS), purchased from GIBCO (Grand Island, NY, USA); Substrate gel (Matrigel), purchased from CORNING (Corning, NY, USA).

hPBMC, purchased from Zhejiang Maishun Biotechnology Co., Ltd.; Source ID: DZ20976, Lot #: A10Z976076.

Model establishment: HCT-15 cells were cultured in RPMI-1640 medium containing 10% FBS and maintained in a 37°C incubator with 5% $CO_2$ and saturating humidity. HCT-15 cells in logarithmic growth phase were collected and re-suspended in RPMI-1640 basal medium containing 50% Matrigel, and cell concentration was adjusted to $2 × 10^7$/mL. Under sterile conditions, 0.1 mL of cell suspension was inoculated subcutaneously into right dorsal flanks of the mice. The inoculated concentration was $2 × 10^6$/0.1 mL/mouse.

Two days after inoculation of tumor cells, hPBMCs frozen in liquid nitrogen were resuscitated and cultured in DMEM medium containing 10% HIFBS (FBS, 56°C $\times$ 30 min), and incubated in a 37°C incubator with 5% $CO_2$ for 6 h. After incubation, hPBMCs were collected and re-suspended in PBS buffer, and cell concentration was adjusted to 2.5 $\times$ $10^7$/mL. Under sterile conditions, 0.2 mL of cell suspension was intraperitoneally injected into the mice at the injection concentration of 5 $\times$ $10^6$/0.2 mL/mouse.

Grouping and administration: When the average tumor volume reached 100 mm$^3$-120 mm$^3$, the animals were randomly grouped according to tumor volume, so that the difference in tumor volume between the groups was less than 10% of the mean.

The day of grouping was Day 0, and the animals were administered from Day 0 based on body weight. During administration, when the body weight loss of single animal was more than 15% (BWL $\geq$ 15%) compared to Day 0, the animal would be discontinued until body weight loss resumed to within 15% (BWL < 15%).

Weighing and observation: During the experiment, the animal body weights and tumor volumes were measured twice a week. The length and width of the tumors were measured using digital calipers, and tumor volumes were estimated by the measured length and width.

During the experiment, the clinical symptoms were observed and recorded once a day, and the time of death of the animals was recorded. Clinical observations included the animals' general health status, weight abnormalities, behavioral abnormalities, and other adverse reactions associated with administration.

Collection of samples: At 1 h, 6 h, 12 h, 24 h, 48 h, and 72 h after administration on Day 15, the blood was collected from animals by tail clipping with a collection volume of about 40 $\mu$L/mouse. EDTA-$K_2$ was used as the anticoagulant and the blood was placed on the ice. The anticoagulated whole blood was centrifuged at 8000 rpm for 10 min at 2°C-8°C, and 20 $\mu$L of upper plasma was collected and stored at -80°C.

Description of the study endpoint: According to the relevant provisions of animal welfare, an individual experimental animal will be excluded from the experimental group and euthanized if it meets any of the following conditions during the experiment: 1. The body weight loss of the animal is more than 20% (BWL $\geq$ 20%) compared to Day 0. 2. Animals have serious adverse reactions, such as blindness, paralysis, etc. 3. Tumor volume was greater than 2000 mm$^3$. 4. Open ulcers are formed on the surface of the tumor.

If an individual animal reached the animal welfare endpoint, it was euthanized with $CO_2$ after the final weighing. The remaining animals in the same group continued to be administered and observed until the animal welfare endpoint or experimental endpoint was reached.

The experiment period was set as 23 days. At the study endpoint, the remaining animals were euthanized with $CO_2$ after the final weighing. The tumors were weighed and photographed.

Evaluation indicators: Tumor volume (TV) was calculated by the following formula: $1/2 \times a \times b^2$, where, a and b are the measured length and width of a tumor, respectively. The tumor growth inhibition rate (%$TGI_{TV}$) was calculated by the formula: $(1 - TV_T/TV_C) \times 100\%$, where, TVc is the average tumor volume in the negative control group, and $TV_T$ is the average tumor volume in the treatment group. The relative tumor volume (RTV) was calculated by the formula: $V_t/V0$, where, $V_0$ is the tumor volume at the time of grouping, and Vt is the tumor volume at each measurement. The relative tumor proliferation rate (%$T/C_{RTV}$) was calculated by the following formula: $T_{RTV}/C_{RTV} \times 100\%$, where, $T_{RTV}$ is RTV of the treatment group, and $C_{RTV}$ is RTV of the negative control group. The tumor inhibition rate (%$TGI_{TW}$) was calculated as follows: %$TGI_{TW} = (1 - TW_T/TW_C) \times 100\%$, where, TWc is the average tumor weight of the negative control group, and $TW_T$ is the average tumor weight of the treatment group. The animal body weight change rate (%BWC) was calculated as follows: $1(BW_t - BW_0)/BW_0 \times 100\%$, where, $BW_t$ is the animal weight at each measurement, and $BW_0$ is the animal weight at grouping.

Statistical analysis: In this study, experimental data were expressed as Mean $\pm$ SEM. The tumor growth curve was plotted with the time point as X-axis and tumor volume (mm$^3$) as Y-axis. The animal body weight change curve was plotted with the time point as X-axis and animal body weight (g) as Y-axis. A two-tailed t-test was used for comparison between groups, with P < 0.05 indicating a significant difference, and P < 0.01 indicating a high significant difference (Microsoft Excel 2007, Redmond, WA, USA).

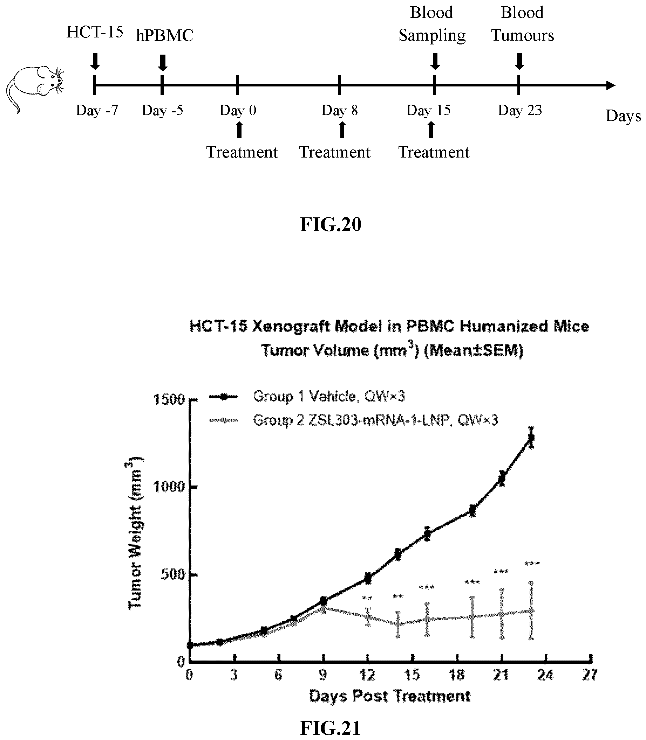

Study protocol: The study protocol of ZSL303-mRNA-1-LNP administration in animals is shown in Figure 20. HCT-15 cells in logarithmic growth phase were collected first, and then tumor cells were inoculated subcutaneously into right dorsal flanks of the mice, and the actual inoculated concentration was 2 $\times$ $10^6$/0.1 mL/mouse. Two days after inoculation of tumor cells, 0.2 mL of hPBMC cell suspension was intraperitoneally injected into the mice at the concentration of 5 $\times$ $10^6$/0.2 mL/mouse. A total of 16 animals were inoculated in this Embodiment, 12 of which were divided into groups and administered immediately after grouping by microneedle administration. Test Article 1 (Namely, CD3$\times$EpCAM-mRNA bispecific antibody group) was administered once a week for 3 consecutive times (MN, QW $\times$ 3). After the last administration of the treatment group of Test Article 1 on Day 15, the blood samples were collected to detect the concentration of bispecific antibody in plasma. All animals were killed on Day 23, tumors were weighed and photographed, and the experiment was ended.

Evaluation of antitumor activity:

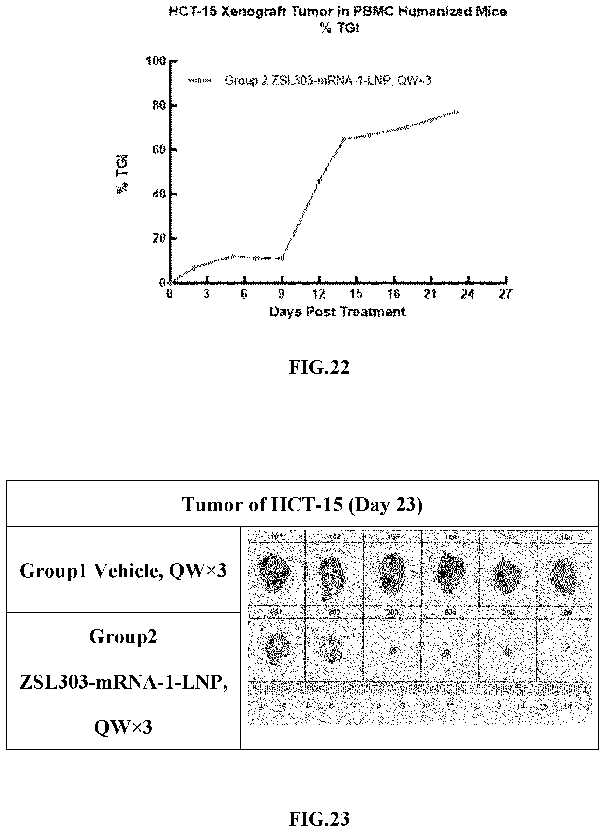

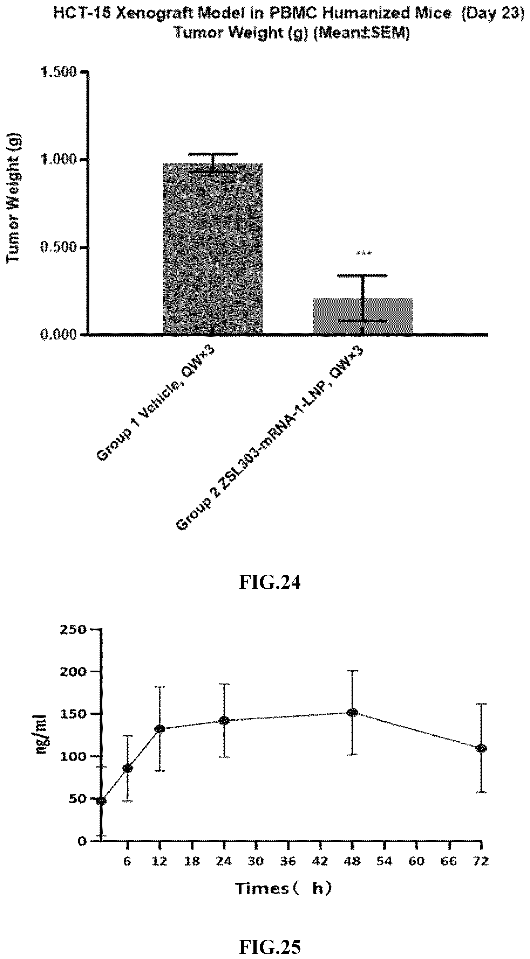

In this Embodiment, immunodeficient tumor-bearing mice with a reconstituted immune system with PBMC were used to detect the tumor inhibition effect of CD3×EpCAM-mRNA-LNP at animal level, and the specific results are shown in Figures 21-25. The tumor volume change curve of HCT-15 xenograft tumor in PBMC humanized mice after two groups of administrations is shown in Figure 21. The tumor inhibitory effect after three microneedle administrations is shown in Figure 22. The imaging results of mouse solid tumors are shown in Figure 23. The comparison of tumor weight 23 days after two groups of administrations is shown in Figure 24. The detected results of the concentration of bispecific antibody in mouse serum after three drug administration are shown in Figure 25.

The experimental results showed that, during the microneedle administration of LNP performed once a week for a total of three times, when LNP was administered before Day 9 (first dose), the tumor inhibitory effect was weak, but after the second dose, tumor growth was significantly inhibited, and the tumor no longer grew. After the third dose, the tumor inhibitory effect was significant and the TGI (Tumor Growth Inhibition) was as high as 80%. The tumor was photographed and weighed when the study endpoint was reached on Day 23, which was completely consistent with the data in Figure 21. Meanwhile, we began to collect mouse serum on Day 15 to detect the concentration of bispecific antibody in mouse serum (Figure 25). After two administrations until Day 15, the concentration of bispecific antibody in mouse serum was about 50 ng/mL, and the average concentration of antibody was 142 ng/mL from 12 h to 48 h. After 48 h, the concentration began to decrease slightly but was also maintained at about 100 ng/mL. This result indicated that the microneedle administration of LNP prepared by us can maintain the plasma concentration for a long time, and thus can manifest tumor inhibitory effects.

This embodiment preferably verifies the efficacy of the platform for mRNA drug delivery by microneedle administration of the composition for intradermal administration provided in the present invention. Our synthesized bispecific antibody mRNA not only confirms that it has good expression performance at the cellular level, but also shows a significant tumor inhibitory effect at animal level. Next, we will further verify the feasibility of our project and assess whether it has better efficacy than the bispecific antibody expressed and purified *in vitro.* If the above two points can be verified and confirmed, we will provide an effective solution for bispecific antibody drugs that are difficult to express and purify *in vitro* with low yield but have significant efficacy.

This specification also includes the subject matter of the following clauses:

1. A composition comprising lipid nanoparticles encapsulating an aqueous solution therein or comprising an aqueous solution therein, wherein the aqueous solution comprises a mRNA for encoding a substance for treating or preventing a disease and has a pH of 4.5-6.8.

2. The composition according to clause 1, wherein the aqueous phase solution comprises a buffer with a pH of 4.5-6.0.

3. The composition according to clause 1, wherein the pH of the buffer is 5.0-5.5; 5.5; 4.5; or 6.0.

4. The composition according to any of clauses 1-3, wherein the buffer comprises one or more of the following: water, Tris buffer, acetate buffer, phosphate buffer, citrate buffer, carbonate buffer, barbital buffer, TE buffer, and PBS buffer.

5. The composition according to clause 4, wherein the Tris buffer comprises tromethamine, Tris hydrochloride (Tris HCL), glacial acetic acid, sodium acetate trihydrate, and water; the acetate buffer comprises sodium acetate buffer, and the sodium acetate buffer comprises sodium acetate, glacial acetic acid, and water.

6. The composition according to any of clauses 1-3, wherein the lipid solution contains 8-20 mg/mL of lipids.

7. The composition according to clause 6, wherein the lipids comprise ionizable lipids, cholesterol, phospholipids, and PEGylated lipids.

8. The composition according to clause 7, wherein the ionizable lipids comprise one or more of the following: C12-200, MC3, DLinDMA, DLin-MC3-DMA, DLinkC2DMA, cKK-E12, ICE, HGT5000, HGT5001, OF-02, DODAC, DDAB DMRIE, DOSPA, DOGS, DODAP, DODMA, DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, SM-102, ALC-0315, HGT4003, and JK-102-CA.

9. The composition according to clause 7, wherein the phospholipids comprise one or more of the following: ceramide, cephalin, cerebroside, diacylglycerol, DPPG, DSPE, DSPC, DPPC, DOPE, DOPC, DPPE, DMPE, DOPG, POPE, POPC, SOPE, and sphingomyelin.

10. The composition according to clause 7, wherein the PEGylated lipids comprise one or more of the following: DMG-PEG1000, DMG-PEG1300, DMG-PEG1500, DMG-PEG1800, DMG-PEG2000, DMG-PEG2200, DMG-PEG2500, DMG- PEG2700, DMG-PEG3000, DMG-PEG3200, DMG-PEG3500, DMG-PEG3700, DMG-PEG4000, DMG-PEG4200, DMG-PEG4500, DMG-PEG4700, DMG-PEG5000, ALC-0159, M- DTDAM-2000, C8-PEG, DOGPEG, ceramide-PEG, and DSPE-PEG.

11. The composition according to clause 6, wherein the lipid solution contains 8-20 mg/mL of lipids.

12. The composition according to clause 11, wherein the mass ratio of said ionizable lipids: cholesterol: phospholipids:

PEGylated lipids is (9-10): (3-4): (2-3): 1.

13. The composition according toclause 1, wherein the active ingredients of substances for the treatment or prevention of diseases comprise protein, peptide, antibody or antibody fragment, antigen or antigen fragment.

14. The composition according to clause 13, wherein the active ingredients of substances for the treatment of diseases are mRNA vaccine or bispecific antibody.

15. An application of a composition in preparation of high mRNA-loaded composition for microneedle administration, wherein the composition comprises an aqueous phase solution and a lipid solution. The lipid solution encapsulates the aqueous phase solution to form lipid nanoparticles, wherein the aqueous phase solution comprises mRNA encoding substances for the treatment or prevention of diseases and a buffer with a pH of 4.5-6.8.

16. The composition according to clause 15, wherein the buffer has a pH of 4.5-6.0.

17. An application of buffer pH in preparation of high mRNA-loaded composition for microneedle administration, wherein the composition comprises an aqueous phase solution and a lipid solution. The lipid solution encapsulates the aqueous phase solution to form lipid nanoparticles, wherein the aqueous phase solution comprises antigen-encoding mRNA and a buffer with a pH of 4.5-6.8.

18. The composition according to clause 17, wherein the buffer has a pH of 4.5-6.0.

19. An application of lipid content in lipid solution in preparation of high mRNA-loaded composition for microneedle administration, wherein the composition comprises an aqueous phase solution and a lipid solution. The lipid solution encapsulates the aqueous phase solution to form lipid nanoparticles, wherein the aqueous phase solution comprises antigen-encoding mRNA and a buffer with a pH of 4.5-6.8.

20. An application of microneedle in an administration device for preparing mRNA composition encoded drug for intradermal administration, wherein the composition refers to the composition of any of claims 1-19.

21. An application of microneedle in preparation of administration device of mRNA composition with increased mRNA expression at the administration site and decreased mRNA expression in the liver.

22. An application of microneedle in preparation of administration device of mRNA composition with prolonged *in vivo* mRNA expression.

23. An application of microneedle in preparation of administration device of mRNA composition with increased antibody titer in response to low-dose mRNA.

24. A method for delivering a drug comprising: using a microneedle to deliver lipid nanoparticles, wherein said lipid nanoparticles comprise an aqueous solution therein, and said aqueous solution comprises a mRNA for encoding an active substance for treating or preventing a disease.

25. The method according to clause 24, wherein said administration is intradermal administration.

26. The method according to clause 25, wherein said aqueous phase solution has a pH of 4.5-6.8.

27. The method according to clause 26, wherein said aqueous phase solution has a pH of 4.5-6.0.

All patents and publications mentioned in the Specification of the present invention are open technologies in this field, which can be cited in the present invention. All patents and publications cited herein are listed in the References, just as each publication is cited individually. The present invention described herein can be realized in the absence of any element(s) or limitation(s), which are not specified here. For example, in each embodiment herein, the terms "containing", "consisting essentially of..." and "consisting of..." can be replaced with the remaining two terms of either. The "one" herein only means "a", and does not exclude to mean "two or above". The terms and expressions used herein are not limited by the way they are described and there is no intent to indicate that the terms and interpretations described in this Specification exclude any equivalent characteristics, but it is understood that any suitable changes or modifications may be made within the scope of the present invention and claim. It is understood that the embodiments described in the present invention are preferred embodiments and characteristics, and that any general technicians in the field may make some changes and modifications in accordance with the essence of the description in the present invention, and such changes and modifications are also considered to be within the scope of the present invention and the limitations of independent and dependent claims.

## Sequence Listing

SEQ ID NO: 1
ZSL303-mRNA-1:
TCTCAACACAACATATACAAAACAAACGAATCTCAAGCAATCAAGCATTCTACTTCT
ATTGCAGCAATTTAAATCATTTCTTTTAAAGCAAAAGCAATTTTCTGAAAATTTTCACCA
TTTACGAACGATAGCATGGGCTGGTCCTGCATCATCTTGTTCTTGGTGGCTACTGCCAC
TGGAGTACACAGCGAGATTGTCCTTACACAGTCACCAGGCACCCTTTCCTTGTCTCCTG
GGGAACGAGCCACCCTCAGTTGTCGGTCAAGCAAGAATCTGCTGCATTCCAATGGGAT
CACATACCTGTATTGGTACCAGCAGAAGCCTGGACAGGCACCCAGACTGCTCATCTAC
CAGATGTCCAATCTGGCCTCAGGCATTCCTGACAGGTTTTCCGGCAGCGGGTCTGGCA
CCGATTTCACCCTGACCATATCCAGGCTCGAACCAGAGGATTTTGCCGTGTATTACTGC
GCACAGAATCTGGAGATTCCCCGCACTTTTGGCCAAGGGACTAAACTGGAGATCAAGC
GGGGAGGAGGCGGCTCTGGAGGTGGCGGCAGTGGTGGAGGCGGGTCTGGCGGTGGC
GGATCTGGAGGTGGTGGGAGCCAGGTCCAGCTTGTTCAGTCAGGCGCAGAGGTGAAG
AAACCCGGAGCTAGCGTGAAAGTCTCCTGCAAAGCGTCAGGGTACACCTTCACCAAC
TATGGGATGAACTGGGTACGTCAAGCCCCAGGGCAAAGACTCGAATGGATGGGTTGGA
TCAACACGTACACAGGGGAACCGACTTATGGCGAGGACTTCAAGGGGAGAGTGACCA
TAACACTGGACACATCCGCTAGCACAGCGTATATGGAGCTGAGCAGTCTGAGGAGCGA
AGATACGGCTGTTTACTATTGTGCCCGCTTTGGTAACTACGTGGACTATTGGGGTCAGG
GAACTCTGGTGACGGTTTCTAGCAGTGGCGGCGGAGGATCCCAGGTGCAGCTGCAGC
AGTCTGGCGCTGAGCTGGCTAGACCTGGCGCCTCCGTGAAGATGTCCTGCAAGACCTC
CGGCTACACCTTCACCCGGTACACCATGCACTGGGTCAAGCAGAGGCCTGGACAGGG
CCTGGAATGGATCGGCTACATCAACCCCTCCCGGGGCTACACCAACTACAACCAGAAG
TTCAAGGACAAGGCCACCCTGACAACCGACAAGTCCTCCTCCACCGCCTACATGCAGC
TGTCCTCCCTGACCTCCGAGGACTCCGCCGTGTACTACTGCGCCCGGTACTACGACGA
CCACTACTCCCTGGACTACTGGGGCCAGGGCACCACACTGACAGTGTCTAGCGGAGGC
GGAGGATCTGGTGGTGGCGGATCTGGCGGCGGTGGAAGTGGCGGAGGTGGTAGCCAG
ATCGTGCTGACCCAGTCTCCCGCCATCATGTCTGCTAGCCCTGGCGAGAAAGTGACAA
TGACCTGCCGGGCCTCCTCCTCCGTGTCCTACATGAACTGGTATCAGCAGAAGTCCGG
CACCTCCCCCAAGCGGTGGATCTACGACACCTCCAAGGTGGCCTCTGGCGTGCCCTAC
AGATTCTCCGGCTCTGGCTCTGGCACCTCCTACAGCCTGACCATCTCCAGCATGGAAGC
CGAGGATGCCGCCACCTACTACTGCCAGCAGTGGTCCTCCAACCCCCTGACCTTTGGC
GCTGGCACCAAGCTGGAACTGAAGGGCGGCTCTCACCACCACCATCACCACTGATGA
GCTGCAGAATTCGTCGACGGATCCGATCTGGTACTGCATGCACGCAATGCTAGCTGCCC
CTTTCCCGTCCTGGGTACCCCGAGTCTCCCCCGACCTCGGGTCCCAGGTATGCTCCCAC
CTCCACCTGCCCCACTCACCACCTCTGCTAGTTCCAGACACCTCCCAAGCACGCAGCA

ATGCAGCTCAAAACGCTTAGCCTAGCCACACCCCCACGGGAAACAGCAGTGATTAACC
TTTAGCAATAAACGAAAGTTTAACTAAGCTATACTAACCCCAGGGGTTGGTCAATTTCGT
GCCAGCCACACCCTCGAGCTAGCAAAAAAAAAAAAAAAAAAAAAAAAAAAAAAAGCA
TATGACTAAAAAAAAAAAAAAAAAAAAAAAAAAAAAAAAAAAAAAAAAAAAAAAAAAA
AAAAAAAAAAAAAAAAAAAAAA

## Claims

1. A composition comprising lipid nanoparticles encapsulating an aqueous solution therein or comprising an aqueous solution therein, wherein the aqueous solution comprises a mRNA for encoding a substance for treating or preventing a disease and wherein the aqueous solution has a pH of 4.5-6.8.

2. The composition according to claim 1, wherein the aqueous solution comprises a buffer with a pH of 4.5-6.0.

3. The composition according to claim 2, wherein the pH of the buffer is 5.0-5.5; 5.5; 4.5; or 6.0.

4. The composition according to any of claims 2-3, wherein the buffer comprises one or more of the following: water, Tris buffer, acetate buffer, phosphate buffer, citrate buffer, carbonate buffer, barbital buffer, TE buffer, and PBS buffer.

5. The composition according to claim 4, wherein the Tris buffer comprises Tromethamine, Tris hydrochloride (Tris HCL), glacial acetic acid, sodium acetate trihydrate; the acetate buffer comprises sodium acetate buffer; or the sodium acetate buffer comprises sodium acetate, glacial acetic acid.

6. The composition according to any of claims 1-5, wherein the lipids comprise ionizable lipids, cholesterol, phospholipids, or PEGylated lipids.

7. The composition according to claim 6, wherein the ionizable lipids comprise one or more of the following: C12-200, MC3, DLinDMA, DLin-MC3-DMA, DLinkC2DMA, cKK-E12, ICE, HGT5000, HGT5001, OF-02, DODAC, DDABDMRIE, DOSPA, DOGS, DODAP, DODMA, DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, SM-102, ALC-0315, HGT4003, orJK-102-CA.

8. The composition according to claim 6, wherein the phospholipids comprise one or more of the following: ceramide, cephalin, cerebroside, diacylglycerol, DPPG, DSPE, DSPC, DPPC, DOPE, DOPC, DPPE, DMPE, DOPG, POPE, POPC, SOPE, or sphingomyelin.

9. The composition according to claim 6, wherein the PEGylated lipids comprise one or more of the following: DMG-PEG1000, DMG-PEG1300, DMG-PEG1500, DMG-PEG1800, DMG-PEG2000, DMG-PEG2200, DMG-PEG2500, DMG-PEG2700, DMG-PEG3000, DMG-PEG3200, DMG-PEG3500, DMG-PEG3700, DMG-PEG4000, DMG-PEG4200, DMG-PEG4500, DMG-PEG4700, DMG-PEG5000, ALC-0159, M-DTDAM-2000, C8-PEG, DOGPEG, ceramide-PEG, or DSPE-PEG.

10. The composition according to any of claims 1-9, wherein the mass ratio of said ionizable lipids: cholesterol: phospholipids: PEGylated lipids is: (9-10): (3-4): (2-3): 1.

11. The composition according to any of claims 1-10, wherein the substance comprises a protein, a peptide, an antibody or an antibody fragment, an antigen or an antigen fragment.

12. A composition for use in treating or preventing a disease, the composition comprising lipid nanoparticles, wherein said lipid nanoparticles include an aqueous solution therein, and wherein the aqueous solution comprises an mRNA encoding an active compound for treating or preventing the disease, and wherein the composition is for administration by a microneedle.

13. The composition according to claim 12, wherein the administration is an intradermal administration or a subcutaneous administration.

14. The composition according to any of claims 12-13, wherein said aqueous solution has a pH of 4.5-6.8.

15. The composition according to any of claims 12-14, wherein said aqueous solution comprises a buffer with a pH of 4.5-6.0, optionally wherein said buffer comprises one or more of the following: water, Tris buffer, acetate buffer, phosphate buffer, citrate buffer, carbonate buffer, barbital buffer, TE buffer, PBS buffer.

Epidermis
Dermis
Adipose tissue
Vein
Muscle

Intradermal injection

**FIG.1**

1x TE Buffer + Sample

2% Triton Buffer + Sample

**FIG.2**

FIG.3

**FIG.4**

Fluc-mRNA transfection in 293T cell

FIG.5

FIG.6A

**FIG.6B**

Luminescence

p/s/cm2/sr

| 6h | 12h | 24h | 48 | 96h | 120h |

G7: Fluc-mRNA, 10 µg, once, MN(G)

G8: Fluc-mRNA, 3.6 µg, once, MN

G9: mRNA, 10 µg, once, ID

**FIG.6C**

Injection site expression

G1: NT, NA, NA

G2: Empty LNP, NA, once, IV

G3: Fluc-mRNA, 10 μg, once, SC

G4: Fluc-mRNA, 10 μg, once, IV

G5: Fluc-mRNA, 10 μg, once, IM

G6: Fluc-mRNA, 10 μg, once, ID

G7: Fluc-mRNA, 10 μg, once, MN(G)

G8: Fluc-mRNA, 3.6 μg, once, MN

FIG.7

## Total expression

G1: NT, NA, NA

G2: Empty LNP, NA, once, IV

G3: Fluc-mRNA, 10 μg, once, SC

G4: Fluc-mRNA, 10 μg, once, IV

G5: Fluc-mRNA, 10 μg, once, IM

G6: Fluc-mRNA, 10 μg, once, ID

G7: Fluc-mRNA, 10 μg, once, MN(G)

G8: Fluc-mRNA, 3.6 μg, once, MN

G9: mRNA, 10 μg, once, ID

**FIG.8**

FIG.9

**Total expression**

G7: Fluc-mRNA, 10 μg, once, MN(G)
G8: Fluc-mRNA, 3.6 μg, once, MN

**FIG.9 contd**

## Injection site expression

## Liver expression

**FIG.10**

**FIG.10 contd**

FIG.11

**FIG.12**

| G1: Empty LNP, NA, once, MN | G2: Fluc-mRNA, 3.6 µg, once, MN | G3: Fluc-mRNA, 3.6 µg, once, SC | G4: Fluc-mRNA, 3.6 µg, once, IM |

PS: muscle 1 means close to the injection site
muscle 2 means far away from the injection site

Luminescence

2.003e+07
1.809e+07
1.614e+07
1.420e+07
1.225e+07
1.031e+07
8.363e+06
6.417e+06
4.474e+06
2.528e+06
5.843e+05

p/s/cm2/sr

FIG.13

Fluc Tissue distribution

G1: Empty LNP, NA, once, MN
G2: Fluc-mRNA, 3.6 µg, once, MN
G3: Fluc-mRNA, 3.6 µg, once, SC
G4: Fluc-mRNA, 3.6 µg, once, IM

FIG.14

**S protein Expression**

G1 : P B S , N A , M N

G2 : Empty LNP, NA, twice, MN

G3 : S protein-mRNA, 10 μg, twice, MN

G4 : S protein-mRNA-LNP, 10 μg, twice, IM

G5 : S protein-mRNA-LNP, 10 μg, twice, ID

G6 : S protein-mRNA-LNP, 10 μg, twice, MN

G7 : S protein-mRNA-LNP, 3.6 μg, twice, MN

G8 : S protein-mRNA-LNP, 1.2 μg, twice, MN

**FIG.15**

FIG.16

EP 4 292 584 A1

G1 : PBS, NA, MN

G2 : Empty LNP. NA. twice. MN

G3 : S protein-mRNA, 10 μg, twice, MN

G4 : S protein-mRNA-LNP, 10 μg, twice, IM

G5 : S protein-mRNA-LNP, 10 μg, twice, ID

G6 : S protein-mRNA-LNP, 10 μg, twice, MN

G7 : S protein-mRNA-LNP, 3.6 μg, twice, MN

G8 : S protein-mRNA-LNP, 1.2 μg, twice, MN

FIG.17

ZSL303-mRNA-1 Expression on 293T

FIG.18

Plasma Levels of CD3XEpCAM

FIG.19

FIG.20

HCT-15 Xenograft Model in PBMC Humanized Mice
Tumor Volume (mm$^3$) (Mean±SEM)

FIG.21

**FIG.22**

**FIG.23**

HCT-15 Xenograft Model in PBMC Humanized Mice (Day 23)
Tumor Weight (g) (Mean±SEM)

**FIG.24**

**FIG.25**

<table>
<tr><td>Europäisches Patentamt<br>European Patent Office<br>Office européen des brevets</td><td style="text-align:center">**EUROPEAN SEARCH REPORT**</td><td>**Application Number**<br><br>EP 23 17 9038</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/232276 A1 (TRANSLATE BIO INC [US]) 19 November 2020 (2020-11-19) * the whole document * * paragraph [0004] - paragraph [0034] * * examples * * claims * | 1-15 | INV. A61K9/127 C12N15/88 A61K31/7105 A61K9/51 C12N15/11 A61K9/00 |
| X | WO 2022/099003 A1 (SANOFI SA [FR]) 12 May 2022 (2022-05-12) * the whole document * * paragraph [0077] * * paragraph [0186] - paragraph [0187] * * examples * * claims * | 1-15 | |
| X | WO 2021/239880 A1 (CUREVAC AG [DE]) 2 December 2021 (2021-12-02) * the whole document * * page 151, line 34 - line 45 * * page 155, line 1 - line 3 * * page 171, line 38 * * examples * * claims * | 12,13 | |
| X | ROCES CARLA B. ET AL: "Manufacturing Considerations for the Development of Lipid Nanoparticles Using Microfluidics", PHARMACEUTICS, vol. 12, no. 11, 15 November 2020 (2020-11-15), pages 1-19, XP055972635, DOI: 10.3390/pharmaceutics12111095 * the whole document * * section 2.2. on page 3 * * section 3.3. on pages 11-12 * * figures 8,9 * | 1-15 | TECHNICAL FIELDS SEARCHED (IPC) A61K C12N |

-/--

The present search report has been drawn up for all claims

| Place of search | Date of completion of the search | Examiner |
|---|---|---|
| The Hague | 26 October 2023 | Marchand, Petra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 2**

Europäisches
Patentamt

European
Patent Office

Office européen
des brevets

# EUROPEAN SEARCH REPORT

**Application Number**

EP 23 17 9038

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GOLOMBEK SONIA ET AL: "Intradermal Delivery of Synthetic mRNA Using Hollow Microneedles for Efficient and Rapid Production of Exogenous Proteins in Skin", MOLECULAR THERAPY-NUCLEIC ACIDS, vol. 11, 1 June 2018 (2018-06-01), pages 382-392, XP93094868, US ISSN: 2162-2531, DOI: 10.1016/j.omtn.2018.03.005 * the whole document * * abstract * * figure 3 * ----- | 12,13 | |
| X | WO 2021/237174 A1 (VAXESS TECH INC [US]) 25 November 2021 (2021-11-25) * page 1, line 17 - line 20 * * page 2, line 5 - page 5, line 15 * * examples * * claims * ----- | 12,13 | |
| X,P | WO 2022/192594 A2 (SORRENTO THERAPEUTICS INC [US]) 15 September 2022 (2022-09-15) * the whole document * * paragraph [0001] * * paragraph [0689] - paragraph [0690] * * paragraph [0832] - paragraph [0836] * * examples * * claims * ----- | 1-15 | **TECHNICAL FIELDS SEARCHED** (IPC) |
| X,P | WO 2023/038892 A1 (MASSACHUSETTS INST TECHNOLOGY [US]) 16 March 2023 (2023-03-16) * the whole document * * page 1, line 9 - page 2, line 5 * * examples * * claims * ----- | 12,13 | |

The present search report has been drawn up for all claims

| Place of search | Date of completion of the search | Examiner |
|---|---|---|
| The Hague | 26 October 2023 | Marchand, Petra |

EPO FORM 1503 03.82 (P04C01)

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

## ANNEX TO THE EUROPEAN SEARCH REPORT
## ON EUROPEAN PATENT APPLICATION NO.

EP 23 17 9038

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2023

| Patent document cited in search report | | Publication date | Patent family member(s) | | Publication date |
|---|---|---|---|---|---|
| WO 2020232276 | A1 | 19-11-2020 | AU | 2020274758 A1 | 23-12-2021 |
| | | | BR | 112021022909 A2 | 25-01-2022 |
| | | | CA | 3140423 A1 | 19-11-2020 |
| | | | CN | 114126588 A | 01-03-2022 |
| | | | EP | 3968952 A1 | 23-03-2022 |
| | | | IL | 288020 A | 01-01-2022 |
| | | | JP | 2022532213 A | 13-07-2022 |
| | | | KR | 20220024022 A | 03-03-2022 |
| | | | SG | 11202112574R A | 30-12-2021 |
| | | | US | 2022218612 A1 | 14-07-2022 |
| | | | WO | 2020232276 A1 | 19-11-2020 |
| WO 2022099003 | A1 | 12-05-2022 | TW | 202233232 A | 01-09-2022 |
| | | | US | 2022142923 A1 | 12-05-2022 |
| | | | US | 2022347100 A1 | 03-11-2022 |
| | | | US | 2022378701 A1 | 01-12-2022 |
| | | | WO | 2022099003 A1 | 12-05-2022 |
| WO 2021239880 | A1 | 02-12-2021 | AU | 2021279312 A1 | 15-12-2022 |
| | | | CA | 3170740 A1 | 02-12-2021 |
| | | | CN | 116322758 A | 23-06-2023 |
| | | | EP | 3993828 A1 | 11-05-2022 |
| | | | IL | 298084 A | 01-01-2023 |
| | | | JP | 2023530229 A | 14-07-2023 |
| | | | KR | 20230053008 A | 20-04-2023 |
| | | | US | 2022211838 A1 | 07-07-2022 |
| | | | US | 2022211841 A1 | 07-07-2022 |
| | | | US | 2022249654 A1 | 11-08-2022 |
| | | | WO | 2021239880 A1 | 02-12-2021 |
| WO 2021237174 | A1 | 25-11-2021 | AU | 2021276000 A1 | 22-12-2022 |
| | | | CA | 3183930 A1 | 25-11-2021 |
| | | | CN | 116406285 A | 07-07-2023 |
| | | | EP | 4153139 A1 | 29-03-2023 |
| | | | JP | 2023527175 A | 27-06-2023 |
| | | | KR | 20230013274 A | 26-01-2023 |
| | | | US | 2023270842 A1 | 31-08-2023 |
| | | | WO | 2021237174 A1 | 25-11-2021 |
| WO 2022192594 | A2 | 15-09-2022 | NONE | | |
| WO 2023038892 | A1 | 16-03-2023 | US | 2023072606 A1 | 09-03-2023 |
| | | | WO | 2023038892 A1 | 16-03-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022106617762
- CN 2022109949172
- CN 2022108970458
- CN 2023105920502
- CN 2020074825 W
- CN 2020106696 W
- US 20160032356 A

- US 20160040154 A
- US 20150376220 A
- US 1819954 W
- US 1819978 W
- WO 201880001680 A
- WO 2011068810 A

**Non-patent literature cited in the description**

- Dictionary of Biochemistry and Molecular Biology. Wiley-Interscience, 1989
- Dictionary of Microbiology and Molecular Biology. Wiley-Interscience, 2007
- Chambers Dictionary of Science and Technology. Chambers, 2007
- Glossary of Genetics. Springer-Verlag, 1991
- The HarperCollins Dictionary of Biology. HarperCollins, 1991
- **CAUDY A A et al.** *Genes & Deve,* vol. 116, 2491-96
- **NEILSEN P E.** *CurrOpin Struct Biol,* vol. 9, 353-57
- **RAZ N KET.** *BiochemBiophys Res Commun.,* vol. 297, 1075-84
- Molecular Cloning. 2001
- **PURCHIO ; G. C. FAREED.** Methods for molecular cloningin eukaryotic cells. *Methods in Enzymology,* 2003
- **ASHIQUL HAQUE et al.** Chemically modified hCFTR mRNAs recuperate lung function in a mouse model of cystic fibrosis. *Scientific Reports,* 2018, vol. 8, 16776

- **KORE et al.** Recent Developments in 5'-Terminal Cap Analogs: Synthesis and Biological Ramifications. *Mini-Reviews in Organic Chemistry,* 2008, vol. 5, 179-192
- **RODGERS et al.** Regulated alpha-globin mRNA decay is a cytoplasmic event proceeding through 3'-to-5' exosome-dependent decapping. *RNA,* 2002, vol. 8, 1526-1537
- **WANG et al.** An mRNA stability complex functions with poly(A)-binding protein to stabilize mRNA in vitro. *Molecular and Cellular biology,* July 1999, vol. 19 (7), 4552-4560
- **LEDDA et al.** Effect of the 3'-UTR length on the translational regulation of 5'-terminal oligopyrimidine mRNAs. *Gene,* 2005, vol. 344, 213-220
- **JANOVICK GUTZKY et al.** Housekeeping gene expression in bovine liver is affected by physiological state, feed intake, and dietary treatment. *J. Day Sci.,* 2007, vol. 90, 2246-2252
- **LANGER.** *Science,* 1990, vol. 249, 1527-1533
- **TREATETAL.** Liposomes in the Therapy of Infectious Disease and Cancer. Liss, 1989, 353-365
- Remington: The Science and Practice of Pharmacy. Lippincott, Williams&Wilkins, 2006
- *Hum. Gene Ther.,* September 2008, vol. 19 (9), 887-95